# EUROPEAN PATENT SPECIFICATION

(11) **EP 2 375 907 B1**
(45) Date of publication and mention of the grant of the patent: **27.02.2019**
(21) Application number: 09828342.7
(22) Date of filing: 23.11.2009
(51) Int. Cl.: A01N 63/04, A01N 65/00, A61K 48/00

(54) **TREATMENT OF DISEASES, DISORDERS OR CONDITIONS OF THE LUNG USING PLACENTAL CELLS**
BEHANDLUNG VON KRANKHEITEN, STÖRUNGEN ODER LEIDEN DER LUNGE MITTELS PLAZENTAZELLEN
TRAITEMENT DE MALADIES, TROUBLES OU ÉTATS DU POUMON UTILISANT DES CELLULES PLACENTAIRES

(30) Priority: 21.11.2008 US 117004 P
(43) Date of publication of application: 19.10.2011
(73) Proprietor: Celularity, Inc., Warren, NJ 07059 (US)
(72) Inventor: HARIRI, Robert, J., Warren,NJ 07059 (US); FALECK, Herbert, West Orange,NJ 07052 (US); ZEITLIN, Andrew, Basking Ridge,NJ 07920 (US)
(74) Representative: Jones Day
(86) International application number: PCT/US2009/065509
(87) International publication number: WO 2010/060031

(56) References cited:
- WO-A1-2008/144820
- WO-A2-2008/151846
- US-A1- 2005 131 028
- US-A1- 2007 190 034
- US-A1- 2008 131 410
- US-A1- 2008 152 629
- US-A1- 2008 226 595
- CARGNONI A ET AL: "Transplantation of Allogeneic and Xenogeneic Placenta-Derived Cells Reduces Bleomycin-Induced Lung Fibrosis", CELL TRANSPLANTATION, vol. 18, no. 4, 6 April 2009 (2009-04-06), pages 405-422, XP55138427, ISSN: 0963-6897, DOI: 10.3727/096368909788809857
- MOODLEY Y ET AL: "Human Amnion Epithelial Cell Transplantation Abrogates Lung Fibrosis and Augments Repair", AMERICAN JOURNAL OF RESPIRATORY AND CRITICAL CARE MEDICINE, vol. 182, no. 5, 3 June 2010 (2010-06-03), pages 643-651, XP55138430, ISSN: 1073-449X, DOI: 10.1164/rccm.201001-0014OC
- BAILO M ET AL: "Engraftment potential of human amnion and chorion cells derived from term placenta.", TRANSPLANTATION, vol. 78, no. 10, 27 November 2004 (2004-11-27), pages 1439-1448, ISSN: 0041-1337

## Description

The present application claims benefit of U.S. Provisional Application No. 61/117,004, filed November 21, 2008.

### 1. FIELD

Provided herein are placental cells for use in methods of treating individuals having a disease, disorder or condition of the lungs. In certain embodiments, the disease, disorder or condition is caused by, or relates to, an unwanted or harmful immune response.

### 2. BACKGROUND

Lung disease is the number three killer in America, responsible for one in six deaths. Lung disease and other breathing problems constitute one of the leading causes of death in babies younger than one year old. Today, more than 35 million Americans are living with chronic lung disease such as asthma, and chronic obstructive pulmonary disease (COPD) otherwise known as emphysema and chronic bronchitis. (American Lung Association website, www.lungusa.org/site/c.dvLUK900E/b.33316/, downloaded November 21, 2008). There are not enough adequate treatments for lung diseases, and new therapies are urgently needed.

### 3. SUMMARY

The present invention provides placental stem cells for use in a method for treatment of a disease, disorder or condition of the lung, wherein the method comprises the administration of the placental stem cell to an individual in a therapeutically effective amount, wherein the therapeutically effective amount is an amount sufficient to cause a detectable improvement in one or more symptoms of said disease, disorder or condition, wherein the placental stem cells are CD10⁺, CD34⁻, CD200⁺, and CD105⁺, as detectable by flow cytometry, and wherein the method comprises detecting said improvement by one or more of peak flow meter, detection of CO₂ levels in the blood, radiography, CT scanning, magnetic resonance imaging, bronchoscopy, or broncheolar lavage.

In certain embodiments of the placental stem cells for use in the invention, said disease, disorder or condition is (a) associated with or caused by an immune response; (b) an interstitial lung disease; (c) an obstructive lung disease; (d) an acute lung injury; or (e) a lung injury caused by a neoplastic or paraneoplastic disease, pneumonia, or cystic fibrosis.

In a certain embodiment, said disease, disorder or condition associated with or caused by an immune response is an autoimmune disease, or a graft-versus-host disease. In yet another embodiment, said autoimmune disease is rheumatoid arthritis, scleroderma, inflammatory bowel disease, or systemic lupus erythematosus. In another embodiment, said interstitial lung disease is interstitial pulmonary fibrosis. In another embodiment, said obstructive lung disease is asthma, bronchitis, acute respiratory distress syndrome or chronic obstructive pulmonary disease. In another embodiment, said acute lung injury is caused by a chemical burn, smoke inhalation, or exposure to a toxic substance. In another embodiment, said disease, disorder or condition is a lung injury caused by neopolastic or paraneoplastic disease, pneumonia, or cystic fibrosis, and wherein said administration results in a forced expiratory volume in 1 second (FEV₁) to forced vital capacity (FVC) ratio of above 0.7.

In certain embodiment, said placental stem cells are CD90⁺ and CD45⁻, as detectable by flow cytometry. Preferably, said placental stem cells are CD44⁺, as detectable by flow cytometry. Preferably. said placental stem cells are CD80⁻ and CD86⁻, as detectable by flow cytometry. In another embodiment, said placental stem cells are one or more of CD29⁺, CD38⁻, CD44⁺, CD54⁺, SH3⁺ or SH4⁺, as detectable by flow cytometry. In yet another embodiment, said placental stem cells are at least one of CD200⁺, CD44⁺, CD45 , CD90⁺, CD117⁻, CD133⁻, KDR , CD80⁻, CD86⁻, HLA-ABC⁺, HLA-DR⁻, or PDL⁺.

Within the boundaries of the present invention, reference to placenta stem cells for use in the invention in the following description shall mean placental stem cells that are CD10⁺, CD34⁻, CD200⁺, and CD105⁺, for use in treating a disease, disorder or condition of the lung as defined above. The cells may optionally be positive or negative for additional cell markers.

Provided herein are placental stem cells or umbilical cord for use in methods and compositions for treating, managing, or ameliorating diseases, disorders and/or conditions of the lung comprising administering placental cells or umbilical cord cells to an individual in need thereof. In certain embodiments, the disease, disorder of condition is associated with or caused by an immune response, *e.g*., associated with, resulting in or caused by inflammation. The placental cells or umbilical cord cells are tissue culture plastic adherent, non-trophoblast multipotent cells referred to herein as placental stem cells, which are described in detail in Section 4.2, below.

In one embodiment, provided herein are placental stem cells for use in a method of treating an individual having, suspected of having, or at risk of developing a disease, disorder or condition of the lung comprising administering to the individual placental stem cells, or medium conditioned by placental stem cells, so that detectable improvement in one or more symptoms of, or a reduction in the progression of one or more symptoms of, said disease, disorder or condition occurs. In a specific embodiment, said disease, disorder or condition is caused by an immune response, e.g., inflammation. In another specific embodiment, said disease, disorder or condition results from a cause in addition to an immune response, e.g., inflammation. In a specific embodiment, said disease, disorder or condition results from a cause other than an immune response, e.g., inflammation.

In a specific embodiment, said lung disease, disorder, or condition is an acute lung injury. In more specific embodiments, said acute lung injury is one or more of physical trauma, a chemical injury, *e.g.,* a chemical burn, smoke inhalation, or exposure to a toxic substance. In another specific embodiment, said lung disease, disorder, or condition is an injury caused by a neoplastic or paraneoplastic disease.

In certain embodiments, the disease, disorder or condition is one or more of a fibrotic disease of the lung, acute respiratory distress syndrome (ARDS), chronic obstructive pulmonary disease (COPD), emphysema, asthma, a viral or bacterial infection of the lung, pneumonia (including chemically-induced pneumonia), or cystic fibrosis. In a specific embodiment, the fibrotic disease of the lung is interstitial lung disease (diffuse parenchymal lung disease). In more specific embodiments, the interstitial lung disease is silicosis, asbestosis, berylliosis, systemic sclerosis, polymyositis, or dermatomyositis. In other more specific embodiments, the interstitial lung disease is caused by an antibiotic, a chemotherapeutic drug, an antiarrhythmic drug, or an infection.

In certain embodiments, the disease, disorder or condition of the lung is associated with or caused by a harmful, deleterious, inappropriate or unwanted immune response, e.g., inflammation, wherein said disease, disorder or condition affects, or manifests symptoms in, the lungs. In specific embodiments, said disease, disorder or condition is one or more of lupus, *e.g.,* lupus erythematosus, scleroderma, or a rheumatological disease (*e.g.,* rheumatoid arthritis).

In another specific embodiment, said disease, disorder or condition is rheumatoid lung disease (RLD), e.g., rheumatoid lung disease associated with rheumatoid arthritis. In another specific embodiment, the administration is sufficient to cause a detectable improvement in one or more symptoms of RLD, or sufficient to detectably reduce or slow the progression of one or more symptoms of RLD, *e.g.,* in a lung of the individual. In a more specific embodiment, said symptom of RLD is a condition adjunct to RLD. In a more specific embodiment, said condition adjunct to RLD is an infection, e.g., a viral infection of the lungs, or fibrosis of the lungs (*e*.*g*., as a consequence of methotrexate therapy).

In another specific embodiment of the method of treatment, the placental cells, *e.g.,* placental stem cells, have been genetically engineered to express a fusion protein comprising IL-1Ra and DHFR.

In another specific embodiment, the disease, disorder or condition is lupus erythematosus, e.g., systemic lupus erythematosus (SLE). In a more specific embodiment, said symptom of lupus erythematosus is one or more of lung and/or pleural inflammation, pleurisy, pleuritis, pleural effusion, lupus pneumonitis, or chronic diffuse interstitial lung disease.

In another specific embodiment of any of the above methods, the method comprises administration of a second therapeutic agent to the individual having the disease, disorder or condition. In a more specific embodiment, said second therapeutic agent is an anti-inflammatory agent, an immunomodulatory agent, and immunosuppressive agent, a pain medication, or an antibiotic. In a more specific embodiment, the second therapeutic agent is an immunomodulatory agent. In another more specific embodiment, the second agent is an anti-CD3 antibody (*e*.*g*., OKT3, muronomab), an anti-IL-2 receptor antibody (*e*.*g*., basiliximab (SIMULECT®) and daclizumab (ZENAPAX®)), an anti T cell receptor antibody (e.g., Muromonab-CD3), azathioprine, a calcineurin inhibitor, a corticosteroid, cyclosporine, methotrexate, mercaptopurine, mycophenolate mofetil, tacrolimus, or sirolimus. In another more specific embodiment, the second therapeutic agent comprises a stem cell of another type, *e*.*g*., a bone marrow-derived mesenchymal stem cell, bone marrow, or a hematopoietic stem cell.

In certain embodiments, the placental cells are placental stem cells that are fibroblastoid, adherent to tissue culture plastic, have the capacity to differentiate into cells displaying one or more characteristics of an osteogenic cell, chondrogenic cell or neurogenic cell, can replicate between about 10-40 times in culture, and/or display characteristic cellular markers, as described herein.

In a specific embodiment, the placental stem cells, are CD10⁺, CD34⁻, CD105⁺, and CD200⁺. In yet another embodiment, the placental cells are CD10⁺, CD34⁻, CD105⁺,CD200⁺, and at least one of: CD44⁺, CD45⁻, CD90⁺, CD117⁻, CD133⁻, KDR⁻, CD80⁻, CD86⁻, HLA-ABC⁺, HLA-DR⁻, or PDL⁺. In another specific embodiment, said placental stem cells additionaly express HLA-G, or express CD73, or express OCT-4 (also known as Octamer-4; octamer binding protein 4; POU5F1), or express CD73, and HLA-G, or express CD73 and facilitate the formation of one or more embryoid-like bodies in a population of placental cells comprising said stem cell when said population is cultured under conditions that allow for the formation of an embryoid-like body, or express OCT-4 and facilitate the formation of one or more embryoid-like bodies in a population of placental cells comprising said stem cell when said population is cultured under conditions that allow for the formation of an embryoid-like body. In a more specific embodiment, the placental cells suppress the activity of an immune cell, *e.g.,* suppress proliferation of T cells, *e*.*g*., CD4⁺ T cells or CD8⁺ T cells.

Placental stem cells, or medium conditioned by placental stem cells, can be administered in a single dose, or in multiple doses. Where administered in multiple doses, the doses can be part of a therapeutic regimen designed to relieve one or more acute symptoms of disease, disorder or condition, wherein the disease, disorder or condition is caused by, or is associated with, an inappropriate or undesirable immune response, or can be part of a long-term therapeutic regimen designed to prevent, or lessen the severity, of a chronic course of such a disease, disorder or condition. When a second therapeutic agent is administered, administration of the two agents can be concurrent, or sequential.

### 3.1 DEFINITIONS

As used herein, the term "SH2" refers to an antibody that binds an epitope on the marker CD105. Thus, cells that are referred to as SH2⁺ are CD105⁺.

As used herein, the terms "SH3" and SH4" refer to antibodies that bind epitopes present on the marker CD73. Thus, cells that are referred to as SH3⁺ and/or SH4⁺ are CD73⁺.

As used herein, the term "isolated cell," *e.g.,* an isolated stem cell, means a cell that is substantially separated from other cells of the tissue, e.g., placenta or umbilical cord, from which the cell, *e.g.,* stem cell, is derived. A cell is "isolated" if at least 50%, 60%, 70%, 80%, 90%, 95%, or at least 99% of the cells with which the cell is naturally associated are removed from the cell, *e.g.,* during collection and/or culture of the cell.

As used herein, the term "isolated population of cells" means a population of cells that is substantially separated from other cells of the tissue, e.g., placenta, from which the population of cells is derived. A population of, *e.g.,* stem cells is "isolated" if at least 50%, 60%, 70%, 80%, 90%, 95%, or at least 99% of the cells with which the population of stem cells are naturally associated are removed from the population of stem cells, e.g., during collection and/or culture of the population of stem cells.

As used herein, the term "placental stem cell" refers to a stem cell or progenitor cell that is derived from a mammalian placenta or umbilical cord, regardless of morphology, cell surface markers, or the number of passages after a primary culture, which adheres to a tissue culture substrate (e.g., tissue culture plastic or a fibronectin-coated tissue culture plate). The term "derived," in this context, includes primary isolates of placental stem cells or placental stem cells that have been expanded in culture. The term "placental stem cell" as used herein does not, however, refer to a trophoblast, a cytotrophoblast, embryonic germ cell, or embryonic stem cell, as those cells are understood by persons of skill in the art. In one embodiment, a placental stem cell is a multipotent cell, derived from a mammalian placenta, that adheres to a tissue culture substrate, e.g., to tissue culture plastic.

As used herein, the term "placental cells" includes cells derived from placenta and cells derived from umbilical cord.

As used herein, the term "umbilical cord stem cell" refers to a stem cell or progenitor cell that is derived from a mammalian umbilical cord, regardless of morphology, cell surface markers, or the number of passages after a primary culture, which adheres to a tissue culture substrate (e.g., tissue culture plastic or a fibronectin-coated tissue culture plate). In one embodiment, an umbilical cord stem cell is a multipotent cell, derived from a mammalian umbilical cord, that adheres to a tissue culture substrate, e.g., to tissue culture plastic.

A cell is considered a "stem cell" if the cell retains at least one attribute of a stem cell, *e.g.,* a marker or gene expression profile associated with one or more types of stem cells; the ability to replicate at least 10-40 times in culture; multipotency, e.g., the ability to differentiate, either *in vitro*, *in vivo* or both, into at least a subset of the cell types in the body, for example, of one or more of the three germ layers, endoderm, mesoderm, or ectoderm; the lack of adult (i.e., differentiated) cell characteristics, or the like. The terms "placental stem cell" and "placenta-derived stem cell" may be used interchangeably. The placental stem cells disclosed herein are, in certain embodiments, multipotent *in vitro* (that is, the cells differentiate *in vitro* under differentiating conditions), multipotent *in vivo* (that is, the cells differentiate *in vivo*), or both.

As used herein, a cell, *e.g.,* stem cell is "positive" for a particular marker when that marker is detectable. For example, a placental stem cell is positive for, *e.g.,* CD73 because CD73 is detectable on placental stem cells in an amount detectably greater than background (in comparison to, *e.g.,* an isotype control). A cell is also positive for a marker when that marker can be used to distinguish the cell from at least one other cell type, or can be used to select or isolate the cell when present or expressed by the cell. Markers expressed on the cell surface can, for example, be detected using cell sorting technology such as flow cytometry. Markers can also, for example, be detected using nucleic acid microarray or RT-PCR technology.

As used herein, "immunomodulation" and "immunomodulatory" mean causing, or having the capacity to cause, a detectable change in an immune response.

As used herein, "immunosuppression" and "immunosuppressive" mean causing, or having the capacity to cause, a detectable reduction in an immune response, and the ability to cause a detectable suppression of an immune response.

### 4. DETAILED DESCRIPTION

The present invention provides placental stem cells for use in a method for treatment of a disease, disorder or condition of the lung, wherein the method comprises the administration of the placental stem cell to an individual in a therapeutically effective amount, wherein the therapeutically effective amount is an amount sufficient to cause a detectable improvement in one or more symptoms of said disease, disorder or condition, wherein the placental stem cells are CD10⁺, CD34⁻, CD200⁺, and CD105⁺, as detectable by flow cytometry, and wherein the method comprises detecting said improvement by one or more of peak flow meter, detection of CO₂ levels in the blood, radiography, CT scanning, magnetic resonance imaging, bronchoscopy, or broncheolar lavage.

In certain embodiments of the placental stem cells for use in the invention, said disease, disorder or condition is (a) associated with or caused by an immune response; (b) an interstitial lung disease; (c) an obstructive lung disease; (d) an acute lung injury; or (e) a lung injury caused by a neoplastic or paraneoplastic disease, pneumonia, or cystic fibrosis.

In a certain embodiment, said disease, disorder or condition associated with or caused by an immune response is an autoimmune disease, or a graft-versus-host disease. In yet another embodiment, said autoimmune disease is rheumatoid arthritis, scleroderma, inflammatory bowel disease, or systemic lupus erythematosus. In another embodiment, said interstitial lung disease is interstitial pulmonary fibrosis. In another embodiment, said obstructive lung disease is asthma, bronchitis, acute respiratory distress syndrome or chronic obstructive pulmonary disease. In another embodiment, said acute lung injury is caused by a chemical burn, smoke inhalation, or exposure to a toxic substance. In another embodiment, said disease, disorder or condition is a lung injury caused by neopolastic or paraneoplastic disease, pneumonia, or cystic fibrosis, and wherein said administration results in a forced expiratory volume in 1 second (FEV₁) to forced vital capacity (FVC) ratio of above 0.7.

In certain embodiment, said placental stem cells are CD90⁺ and CD45⁻, as detectable by flow cytometry. Preferably, said placental stem cells are CD44⁺, as detectable by flow cytometry. Preferably, said placental stem cells are CD80⁻ and CD86⁻, as detectable by flow cytometry. In another embodiment, said placental stem cells are one or more of CD29⁺, CD38⁻, CD44⁺, CD54⁺, SH3⁺ or SH4⁺, as detectable by flow cytometry. In yet another embodiment, said placental stem cells are at least one of CD200⁺, CD44⁺, CD45⁻, CD90⁺, CD117⁻, CD133⁻, KDR , CD80⁻, CD86⁻, HLA-ABC⁺, HLA-DR⁻, or PDL⁺.

Provided herein are placental stem cells for use in methods for the treatment of an individual having, suspected of having, or at risk of developing a disease, disorder or condition of, or affecting, the lungs, comprising administering to the individual one or more doses of placental stem cells, medium conditioned by placental stem cells, umbilical cord cells, *e.g.,* umbilical cord stem cells, and/or medium conditioned by umbilical cord cells. In certain embodiments, the disease, disorder or condition is associated with, arises from, or relates to an inappropriate, unwanted, harmful or deleterious immune response, e.g., an autoimmune disease. Placental stem cells for use in methods for the treatment of such individuals, and for the administration of such stem cells, alone or in combination with other therapies, are discussed in detail below.

### 4.1 PLACENTAL STEM CELLS FOR USE IN METHODS OF TREATMENT OF LUNG DISEASES, DISORDERS OR CONDITIONS

In one embodiment, provided herein are placental stem cells for use in a method of treating an individual having, suspected of having, or at risk of developing a disease, disorder or condition of, or affecting, the lung comprising administering to the individual a therapeutically-effective amount of placental stem cells, or medium conditioned by placental stem cells, wherein said therapeutically-effective amount causes a detectable improvement in one or more symptoms of, or a reduction in the progression of one or more symptoms of, said disease, disorder or condition occurs. Also described herein is the use of certain placental cells, e.g., placental stem cells, in the manufacture of a medicament for treating, managing, ameliorating or preventing diseases, disorders and/or conditions of the lung. Cells useful in the treatment methods disclosed herein are further described in Section 4.2, below.

In certain embodiments, the disease, disorder or condition of, or affecting, the lung is a disease, disorder or condition of, or affecting, the lung not caused by, or related to, an autoimmune disease. In specific embodiments, the disease, disorder or condition of, or affecting, the lung is not related to, or caused by, graft-versus-host disease, systemic lupus erythematosus, inflammatory bowel disease, or rheumatoid arthritis (e.g., is not a rheumatoid lung disease). In certain other embodiments, the disease, disorder or condition of, or affecting, the lung is not caused by or related to inflammation (e.g., is not caused by inflammation of the lung, or is not caused by an inflammatory response in a non-lung tissue, etc.).

In a specific embodiment of any of the embodiments herein, the placental stem cells are multipotent placental stem cells. Placental stem cells, used in the methods described herein can be derived or obtained from a single placenta, or from multiple placentas. The placental stem cells can also be derived from a single species, *e.g.,* the species of the intended recipient, or can be derived from multiple species. "Derived," as used herein, means isolated from placenta or umbilical cord, or expanded from cells isolated from placenta or umbilical cord.

### 4.1.1 Treatment of Interstitial Lung Diseases or Disorders

In certain embodiments, the disease, disorder or condition of the lung treatable using placental stem cells, is an interstitial lung disease (also known as diffuse placental stem cells for use in parenchymal lung disease). Thus, provided herein placental stem cells for use in a method of treating an individual having an interstitial lung disease, comprising administering a therapeutically effective amount of placental stem cells, to said individual, e.g., to the affected lung of said individual. In a specific embodiment, the method of treatment comprises assessing said individual for improvement in one or more parameters of lung function after said administering (e.g., from 7 days to 30 days afterwards), wherein said parameters of lung function are forced expiratory volume in one second (FEV₁); forced volume vital capacity (FVC); FEV₁/ FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement of one or more of said parameters of lung function (1) to 80% or more of expected; or (2) by at least 2%, 3%, 4%, 5%, 6%, 7%, 8%, 9%, 50%. In a more specific embodiment, the method comprises identifying any of said parameters that, prior to administration, are less than 80% of expected values for an individual of the same height and weight, and assessing said parameters after said administering, wherein said administering results in improvement of one or more of said parameters of lung function (1) to 80% or more of expected; or (2) by at least 2%, 3%, 4%, 5%, 6%, 7%, 8%, 9%, 50%.

In certain embodiments, the interstitial lung disease is not an obstructive airway disease or obstructive lung disease. Such diseases may involve inflammation of the interstitium, the tissue and space around the air sacs of the lungs. In certain embodiments, the interstitial lung disease is not caused by inflammation. Symptoms of interstitial lung disease include, without limitation, shortness of breath (particularly with exertion); fatigue; weakness; loss of appetite; loss of weight; dry, nonproductive cough (little or no phlegm production); discomfort in the chest; labored breathing; or evidence of hemorrhage in one or both of the lungs.

Provided herein are placental stem cells for use in a method of treating an individual having an interstitial lung disease or disorder *(e.g.,* a diffuse parenchymal lung disease), comprising administering to said individual a therapeutically-effective amount of placental stem cells;. In a specific embodiment, the therapeutically-effective amount of placenta stem cells is an amount that, permanently or transiently, results in a detectable improvement in, or lessening of the worsening of, one or more symptoms of said interstitial lung disease. In a specific embodiment, said symptom is a below-normal capacity of a lung of the individual to diffuse carbon monoxide (e.g., low diffusion capacity of carbon monoxide (DLCO) compared to normal). In certain more specific embodiments, said one or more symptoms of interstitial lung disease comprises shortness of breath (particularly with exertion); fatigue; weakness; loss of appetite; loss of weight; dry, nonproductive cough (little or no phlegm production); discomfort in the chest; labored breathing; or evidence of hemorrhage in one or both of the lungs.

In certain specific embodiments, said administration of placental stem cells; results in a detectable improvement in one or more measures of lung function in said individual, e.g., as evidenced by spirometry, peak flow monitoring, forced expiratory volume, or the like. In a more specific embodiment, said administration results in an increase of at least 5%, 10%, 15% or 20% carbon monoxide diffusion, as compared to carbon monoxide diffusion prior to administration, in a lung of said individual. In certain other specific embodiments, said administration of placental stem cells; results in a detectable improvement in one or more of a chest X-ray, CT scan, MRI, bronchoscopy or similar scan (e.g., visible improvement in the appearance of the lung). In another specific embodiment, said administration of placental stem cells, results in a detectable improvement in the level of carbon dioxide detectable in the blood *(e.g.,* movement of CO₂ levels to within a normal range).

In a specific embodiment, the interstitial lung disease is not caused by lupus erythematosus. In a more specific embodiment, said interstitial lung disease is not chronic diffuse interstitial lung disease.

In certain embodiments, the interstitial lung disease is an interstitial lung disease with fibrosis *(e.g.,* a fibrotic lung disease), such as interstitial pulmonary fibrosis. In certain embodiments, the interstitial lung disease, e.g., fibrotic lung disease, is idiopathic pulmonary pneumonia, idiopathic pulmonary fibrosis (cryptogenic fibrosing alveolitis), pneumoconiosis, asbestosis, baritosis, bauxite fibrosis, berylliosis, Caplan's syndrome, chalicosis, coal worker pneumoconiosis, pulmonary sarcoidosis, siderosis, silicosis, byssinosis, hypersensitivity pneumonitis, bagassosis, bird fancier's lung, or farmer's lung.

### 4.1.2 Treatment of Obstructive Lung Diseases and Disorders

In certain embodiments, the disease, disorder or condition of the lung treatable using placental stem cells, is an obstructive lung disease or disorder. Thus, provided here are placental stem cells for use in a method of treating an individual having an obstructive lung disease or disorder, comprising administering a therapeutically effective amount of placental stem cells to said individual, e.g., to the affected lung of said individual. In a specific embodiment, the method of treatment comprises assessing said individual for improvement in one or more parameters of lung function after said administering (e.g., from 7 days to 30 days afterwards), wherein said parameters of lung function are forced expiratory volume in one second (FEV₁); forced volume vital capacity (FVC); FEV₁/FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement one of said parameters of lung function (1) to 80% or more of expected; or (2) by at least 2%, 3%, 4%, 5%, 6%, 7%, 8%, 9%, 50%. In another more specific embodiment, the method comprises identifying any of said parameters that, prior to administration, are less than 80% of expected values for an individual of the same height and weight, and assessing said parameters after said administering, wherein said administering results in improvement in one or more of said parameters of lung function (1) to 80% or more of expected; or (2) by at least 2%, 3%, 4%, 5%, 6%, 7%, 8%, 9%, 50%.

In specific embodiments, the obstructive lung disease is acute respiratory distress syndrome (ARDS), asthma, bronchiectasis, bronchiolectasis, bronchiolitis, bronchitis, chronic obstructive pulmonary disease (COPD), or emphysema. In another specific embodiment, the obstructive lung disease or disorder is not caused by an autoimmune disease. In a more specific embodiment, said obstructive lung disease or disorder is not caused by inflammatory bowel disease or graft-versus-host disease.

Thus, provided herein are placental stem cells for use in method of treating an individual having an obstructive lung disease or disorder, comprising administering to said individual a therapeutically-effective amount of placental stem cells;. In a specific embodiment, the therapeutically-effective amount of placental stem cells is an amount that, permanently or transiently, results in a detectable improvement in, or lessening of worsening of, one or more symptoms of said obstructive lung disease or disorder.

In a specific embodiment, said obstructive lung disease or disorder is chronic obstructive pulmonary disease, *e.g.,* as diagnosed by a forced expiratory air volume in 1 second (FEV₁) to forced vital capacity (FVC) ratio of less than 0.7. In a more specific embodiment, said therapeutically effective amount of placental stem cells is an amount that results in a detectable rise in the FEV₁/FEC ratio above 0.7 after administration, e.g., a rise of 0.02, 0.03, 0.04, 0.05, 0.06, 0.07, 0.08, 0.09, 0.10, or more or more.

In another specific embodiment, said obstructive lung disease or disorder is asthma. In more specific embodiments, said therapeutically effective amount of placental stem cells is an amount that results in a detectable improvement in one or more symptoms of asthma, e.g., airway obstruction, as determined by spirometry or a peak flow meter.

Treatment of an obstructive lung disease, disorder or condition can comprise administration of a second therapeutic compound. In specific embodiments, e.g., wherein the disease, disorder or condition of the lung is asthma, the second therapeutic composition or second therapy can comprise one or more of an inhaled therapeutic compound or therapeutic compound taken orally, *e.g*., without limitation, inhaled corticosteroids (*e*.*g*., hydrocortisone, prednisone, prednisolone, methylprednisolone, dexamethasone, betamethasone, triamcinolone, beclometasone, fludrocortisone acetate, deoxycortisone acetate, aldosterone, or the like), or inhaled beta agonists (e.g., salbutamol, levosalbutamol, terbutaline, pirbuterol, procaterol, metaproterenol, fenoterol, bitolterol, salmeterol, formoterol, bambuterol, clenbuterol, indacaterol, or the like), leukotriene inhibitors (*e*.*g*., zileuton, MK-866, montelukast, zafirlukast, or the like).

In other specific embodiments, wherein the disease, disorder or condition of the lung is, *e.g.,* COPD, the second therapeutic composition or second therapy can comprise one or more of an inhaled beta agonist (see the list of beta agonists recited in the discussion of treatment of asthma, above); or anticholinergics (e.g., ipratropium bromide (Atrovent), oxitropium bromide (Oxivent), tiotropium (Spiriva), or the like).

### 4.1.3 Treatment of Lung Diseases, Disorders or Conditions Caused by an Immune Response

In certain embodiments, provided herein are placental stem cells for use in a method of treating a lung of an individual having a disease, disorder or condition of the lung associated with or caused by a harmful, deleterious, inappropriate or unwanted immune response, comprising administering a therapeutically effective amount of placental stem cells; to said individual, e.g, to an affected lung of said individual. In certain embodiments, the lung disease, disorder, or condition is, for example, an allergic or autoimmune disease affecting the lungs. In a specific embodiment, said disease, disorder or condition is a lung disorder or disease associated with, or caused by, lupus, e.g., systemic lupus erythematosus, scleroderma, graft-versus-host disease, or rheumatoid arthritis (e.g., rheumatoid lung disease).

In a specific embodiment, the method of treatment comprises identifying, in an individual having an inappropriate immune response, e.g., an autoimmune disease, a symptom of said immune response in the lung; administering placental stem cells; to said individual, e.g., to the affected lung of said individual; and assessing said individual for improvement in one or more parameters of lung function after said administering (e.g., from 7 days to 30 days afterwards), wherein said parameters of lung function are forced expiratory volume in one second (FEV₁); forced volume vital capacity (FVC); FEV₁/ FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement one of said parameters of lung function (1) to 80% or more of expected; or (2) by at least 2%, 3%, 4%, 5%, 6%, 7%, 8%, 9%, or 10%, or more. In a more specific embodiment, the method comprises identifying any of said parameters that, prior to administration, are less than 80% of expected values for an individual of the same height and weight, and assessing said parameters after said administering, wherein said administering results in the improvement of one or more of said parameters of lung function (1) to 80% or more of expected; or (2) by at least 2%, 3%, 4%, 5%, 6%, 7%, 8%, 9%, or 10%, or more.

In certain embodiments, the placental stem cells for use in the invention suppress an immune response when contacted with a plurality of immune cells *(e.g.,* CD4⁺ T cells, CD8⁺ T cells, or natural killer (NK) cells) *in vivo*, *e*.*g*., in an individual afflicted with said disease, disorder or condition affecting the lungs. In various specific embodiments, said contact is sufficient to suppress an immune function associated with, or causative of, said lung disease, disorder or condition by at least 50%, 60%, 70%, 80%, 90% or 95%, compared to immune function in the affected individual in the absence of the placental stem cells.

The contacting of placental cells, e.g., placental stem cells with immune cells can occur *in vivo* in the context of, or as an adjunct to, for example, grafting or transplanting of one or more types of tissues to a recipient individual. Such tissues may be, for example, lung tissue. In this regard, the placental stem cells can be used to suppress one or more immune responses of one or more immune cells contained within the recipient individual, within the transplanted lung tissue, or both. The contacting can occur before, during and/or after the grafting or transplanting. For example, placental stem cells can be administered at the time of the transplant or graft. The placental cells can also, or alternatively, be administered prior to the transplanting or grafting, e.g., about 1, 2, 3, 4, 5, 6 or 7 days prior to the transplanting or grafting. Placental cells, e.g., placental stem cells, can also, or alternatively, be administered to a transplant or graft recipient after the transplantation or grafting, for example, about 1, 2, 3, 4, 5, 6 or 7 days after the transplanting or grafting. Preferably, the placental cells are contacted with the immune cells before any detectable sign or symptom of an immune response, either by the recipient individual or the transplanted tissue or graft, *e.g.,* a detectable sign or symptom of graft-versus-host disease or detectable inflammation, is detectable.

Treatment of an individual having a lung disorder, disease, or condition associated with, worsened by, or caused by an unwanted or harmful immune response can additionally comprise administration to the individual of one or more immunosuppressive agents, particularly in the *in vivo* context. In one embodiment, the plurality of placental stem cells, are contacted with the plurality of immune cells *in vivo* in an individual, and a composition comprising an immunosuppressive agent is administered to the individual having the disease, disorder or condition of the lungs. Immunosuppressive agents are well-known in the art and include, *e*.*g*., anti-T cell receptor antibodies (monoclonal or polyclonal, or antibody fragments or derivatives thereof), anti-IL-2 receptor antibodies (e.g., Basiliximab (SIMULECT®) or daclizumab (ZENAPAX)®), anti T cell receptor antibodies (e.g., Muromonab-CD3), azathioprine, corticosteroids, cyclosporine, tacrolimus, mycophenolate mofetil, sirolimus, calcineurin inhibitors, and the like. In a specific embodiment, the immumosuppressive agent is a neutralizing antibody to macrophage inflammatory protein (MIP)-1α or MIP-1β. Preferably, the anti-MIP-la or MIP-1β antibody is administered in an amount sufficient to cause a detectable reduction in the amount of MIP-1α and/or MIP-1β in said individual, e.g., at the time of transplanting.

In certain embodiments, an individual having a disease, disorder or condition of the lungs is treated by administration of placental stem cells and, optionally, one or more therapeutic agents, at any time during the progression of the disease. For example, the individual can be treated immediately after diagnosis, or within 1, 2, 3, 4, 5, 6 days of diagnosis, or within 1, 2, 3, 4, 5, 6, 7, 8, 9, 10, 15, 20, 25, 30, 35, 40, 45, 50 or more weeks, or 1, 2, 3, 4, 5, 6, 7, 8, 9, 10 or more years after diagnosis. The individual can be treated once, or multiple times during the clinical course of the disease. The individual can be treated, as appropriate, during an acute attack, during remission, or during a chronic degenerative phase.

In a specific embodiment, treatment of a disease, disorder or condition of the lung related to or caused by an inappropriate, deleterious or harmful immune response comprises administration of a population of a second type of cell, *e*.*g*., stem cells, in addition to the placental stem cells. In a specific embodiment, said stem cells are mesenchymal stem cells, *e*.*g*., bone marrow-derived mesenchymal stem cells. In other embodiments, the second type of cells are multipotent stem cells, pluripotent stem cells, progenitor cells, hematopoietic stem cells, *e.g*., CD34⁺ hematopoietic stem cells (*e.g.,* contained within unprocessed bone marrow or cord blood, or cells isolated from bone marrow or cord blood), adult stem cells, embryonic stem cells, or embryonic germ cells. The second type of cell, e.g., mesenchymal stem cell, can be administered with the placental stem cells in any ratio, *e*.*g*., a ratio of about 100:1, 75:1, 50:1, 25:1, 20:1, 15:1, 10:1, 5:1, 1:1, 1:5, 1:10, 1:15, 1:20, 1:25, 1:50, 1:75 or 1:100. Mesenchymal stem cells can be obtained commercially or from an original source, e.g., bone marrow, bone marrow aspirate, adipose tissue, and the like.

### 4.1.3.1 Lung Disorders Associated with Graft-Versus Host Disease

In certain embodiments, provided herein are placental stem cells for use in a method of treating an individual, e.g., a transplant recipient or individual who will receive, or has received, a transplant, comprising administering to the individual, e.g., an affected lung of the individual, a therapeutically-effective amount of placental stem cells. In certain embodiments, the transplant is a lung transplant or a graft of a part of a lung. In certain other embodiments, the transplant is a bone marrow transplant. In specific embodiments, the transplant recipient has, is experiencing a symptom of, or is at risk for developing, graft-versus-host disease (GVHD).

In one embodiment, said method of treatment comprises assessing an individual for one or more parameters of lung function prior to a transplant, e.g., an organ transplant or bone marrow transplant, and, if said one or more parameters worsen after said transplant, administering a therapeutically effective amount of placental stem cells wherein said parameters of lung function are forced expiratory volume in one second (FEV₁); forced volume vital capacity (FVC); FEV₁/FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement one of said parameters of lung function (1) to 80% or more of expected values for an individual of the same height and weight; or (2) by at least 2%, 3%, 4%, 5%, 6%, 7%, 8%, 9%, or 10%, or more.

In preferred embodiments, said method comprises administering to the individual a therapeutically effective amount of placental stem cells;, culture medium conditioned by placental stem cells, wherein the therapeutically effective amount is an amount that results in a detectable improvement in one or more symptoms, or delay of onset of one or more symptoms, of the disease, disorder or condition of the lung associated with or caused by or associated with transplant or GVHD. In certain embodiments, said administration results in at least stabilization of one or more symptoms of GVHD; that is, said one or more symptoms do not significantly improve, but do not significantly worsen, either. In more specific embodiments, said one or more symptoms of GVHD comprise obstructive lung disease (including any of wheezing dyspnea and/or chronic coughing).

In certain embodiments, said method of treatment comprises an assessment of effectiveness of the administration of the placental stem cells. For example, in one embodiment, the method of treating a lung disease or disorder caused by, or associated with, a transplant or GVHD comprises (1) administering a therapeutically effective amount of placental stem cells, culture medium conditioned by placental stem cells; and (2) assessing the individual for detectable improvement in one or more symptoms, or delay of onset of one or more symptoms, of the disease, disorder or condition of the lung associated with or caused by GVHD. In certain specific embodiments, said method of treating comprises a second (or further) administration of placental stem cells to said individual, optionally followed by a second (or further) assessment of the individual for detectable improvement in one or more symptoms, or delay of onset of one or more symptoms, of the disease, disorder or condition of the lung associated with or caused by GVHD.

The invention is not limited by the nature of the donor or recipient. Transplantation can cross species lines. In preferred embodiments, the donor and recipient are the same species, *e.g.,* are both human. The transplant recipient can be fully- or partially-allogeneic to the donor. The transplantation can be autologous. Transplant recipients or donors can be less than five years of age, from 1 to 10 years of age, from 5 to 15 years of age, from 10 to 20 years of age, from 15 to 25 years of age, from 20 to 30 years of age, from 25 to 35 years of age, from 30 to 40 years of age, from 35 to 45 years of age, from 40 to 50 years of age, from 45 to 55 years of age, from 50 to 60 years of age, from 55 to 65 years of age, from 60 to 70 years of age, or 70 years of age or older.

The placental stem cells can be used to treat one or more pulmonary manifestations of GVHD in individuals exhibiting symptoms indicative of a grading or staging of the disease as shown in Tables 1 and 2, below. GVHD is generally graded by severity of symptoms. For example, in one embodiment, symptoms of GVHD are staged, and GVHD is graded from 0 (no GVHD) - IV (life-threatening GVHD) according to skin, liver, and/or intestinal symptoms, as shown in Tables 1 and 2.

**Table 1. Staging of Acute Graft-Versus-Host Disease**

| **Stage** | **Skin** | **Liver (Bilirubin Level, mg/dL)** | **Intestine** |
|---|---|---|---|
| + | Maculopapular rash on <25% of body surface | 2-3 | Diarrhea 500-1000 mL/d or persistent nausea |
| ++ | Maculopapular rash on 25-50% of body surface | 3-6 | Diarrhea 1000-1500 mL/d |
| +++ | Generalized erythroderma | 6-15 | Diarrhea >1500 mL/d |
| ++++ | Desquamation and bullae | >15 | Pain with or without ileus |

**Table 2. Grading of Acute GVHD**

| **Overall Grade** | **Stage** | | | |
|---|---|---|---|---|
| | **Skin** | **Liver** | **Gut** | **Functional Impairment** |
| 0 (None) | 0 | 0 | 0 | 0 |
| I (Mild) | + to ++ | 0 | 0 | 0 |
| II (Moderate) | + to +++ | + | + | + |
| III (Severe) | ++ to +++ | ++ to +++ | ++ to +++ | ++ |
| IV (Life threatening) | ++ to ++++ | ++ to ++++ | ++ to ++++ | +++ |

In specific embodiments, the placental stem cells; are administered to the individual within 14, 13, 12, 11, 10, 9, 8, 7, 6, 5, 4, 3, 2 or 1 day prior to transplantation. In another specific embodiment, the placental stem cells are administered concurrently with transplantation. In another specific embodiment, the placental stem cells are administered within 1, 2, 3, 4, 5, 6, 7, 8, 9, 10, 11, 12, 13 or 14 days of transplantation. Administration of placental stem cells; can be performed multiple times, e.g., multiple times before, with or after transplantation, or any combination thereof. In another embodiment, placental stem cells, are administered at any time post-transplantation when graft-versus-host disease of Grade II or worse is manifested in the individual (transplant recipient).

In another embodiment of the method, the individual, e.g., transplant recipient or an individual who will receive a transplant, is administered a therapeutically effective amount of placental stem cells; and additionally at least one other therapeutic agent. In a specific embodiment, the therapeutic agent is athymocyte globulin, mycophenolate mofetil, sirolimus, Campath-1H, keratinocyte growth factor (KGF), suberoylanilide hydroxamic acid (SAHA), cortisone, hydrocortisone, predisone, or methylprednisone. In another specific embodiment, the therapeutic agent is an immunosuppressive agent or immunomodulatory agent. Immunosuppressive agents and immunomodulatory agents that can be used as second agents to treat GVHD, *e.g.,* to treat a manifestation of GVHD in the lungs, include, but are not limited to, methotrexate, leflunomide, cyclophosphamide, cyclosporine A, macrolide antibiotics (*e*.*g*., FK506 (tacrolimus)), methylprednisolone (MP), corticosteroids, steroids, mycophenolate mofetil, rapamycin (sirolimus), mizoribine, deoxyspergualin, brequinar, malononitriloamindes (*e*.*g*., leflunamide), T cell receptor modulators, and cytokine receptor modulators, peptide mimetics, and antibodies (*e*.*g*., human, humanized, chimeric, monoclonal, polyclonal, Fvs, ScFvs, Fab or F(ab)₂ fragments or epitope binding fragments), nucleic acid molecules (e.g., antisense nucleic acid molecules and triple helices), small molecules, organic compounds, and inorganic compounds. In particular, immunomodulatory agents include, but are not limited to, methotrexate, leflunomide, cyclophosphamide, cytoxan, Immuran, cyclosporine A, minocycline, azathioprine, antibiotics *(e.g.,* FK506 (tacrolimus)), methylprednisolone (MP), corticosteroids, steroids, mycophenolate mofetil, rapamycin (sirolimus), mizoribine, deoxyspergualin, brequinar, malononitriloamindes (e.g., leflunamide), T cell receptor modulators, and cytokine receptor modulators. Examples of T cell receptor modulators include, but are not limited to, anti-T cell receptor antibodies (*e*.*g*., anti-CD4 antibodies (*e.g.,* cM-T412 (Boehringer), IDEC-CE9.Is (IDEC and SKB), mAB 4162W94, ORTHOCLONE® and OKTcdr4a (Janssen-Cilag)), anti-CD3 antibodies (e.g., NUVION® (Product Design Labs), OKT3 (Johnson & Johnson), or Rituxan (IDEC)), anti-CD5 antibodies (e.g., an anti-CD5 ricin-linked immunoconjugate), anti-CD7 antibodies (e.g., CHH-380 (Novartis)), anti-CD8 antibodies, anti-CD40 ligand monoclonal antibodies (*e*.*g*., IDEC-131 (IDEC)), anti-CD52 antibodies *(e.g.,* CAMPATH® 1H (Ilex)), anti-CD2 antibodies, anti-CDla antibodies (e.g., Xanelim (Genentech)), and anti-B7 antibodies (*e*.*g*., IDEC-114) (IDEC))), CTLA4-immunoglobulin, thalidomide, or one of the compounds in Section 5.6.6, above. In a specific embodiment, a T cell receptor modulator is a CD2 antagonist. In other embodiments, a T cell receptor modulator is not a CD2 antagonist. In another specific embodiment, the agent is antibody MEDI-501 (T10B9). In another specific embodiment, a T cell receptor modulator is a CD2 binding molecule, preferably MEDI-507. In other embodiments, a T cell receptor modulator is not a CD2 binding molecule. Any combination of the above therapeutic agents, suitable for treatment of GVHD or a symptom of GVHD, can be administered to the individual. Such therapeutic agents can be administered in any combination with the placental stem cells, culture medium conditioned by placental cells, umbilical cord cells, e.g., umbilical cord stem cells, and/or culture medium conditioned by umbilical cord cells, at the same time or as a separate course of treatment.

### 4.1.3.2 Lung Disorders Associated with Rheumatoid Arthritis

In another embodiment, provided herein are placental stem cells for use in a method of treating an individual that has, or is experiencing a symptom of, or is at risk for developing, a disease, disorder or condition of the lung associated with, or caused by, rheumatoid arthritis (RA), comprising administering to the individual a therapeutically effective amount of placental stem cells, or culture medium conditioned by placental stem cells, wherein said therapeutically effective amount is an amount that results in a detectable improvement in one or more symptoms, or delay of onset of one or more symptoms, of the disease, disorder or condition of the lung associated with or caused by RA. In certain embodiments, said administration results in at least stabilization of one or more symptoms of RA that manifest in a lung of the individual; that is, said one or more symptoms do not significantly improve, but do not significantly worsen, either.

In certain embodiments, said method of treatment comprises identifying, in an individual having RA, a symptom of said RA in the lung; administering a therapeutically effective amount of placental stem cells; to said individual, e.g., to the affected lung of said individual; and assessing said individual for improvement in one or more parameters of lung function after said administering (e.g., from 7 days to 30 days afterwards), wherein said parameters of lung function are forced expiratory volume in one second (FEV₁); forced volume vital capacity (FVC); FEV₁/FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement one of said parameters of lung function (1) to 80% or more of expected; or (2) by at least 2%, 3%, 4%, 5%, 6%, 7%, 8%, 9%, 50%. In a more specific embodiment, the method comprises identifying any of said parameters that, prior to administration, are less than 80% of expected values for an individual of the same height and weight, and assessing said parameters after said administering, wherein said administering results in the improvement one of said parameters of lung function (1) to 80% or more of expected; or (2) by at least 2%, 3%, 4%, 5%, 6%, 7%, 8%, 9%, 50%.

In a specific embodiment, the administration is sufficient to cause a detectable improvement in one or more symptoms of RA, or condition adjunct to RA, or sufficient to detectably reduce the onset of one or more symptoms of RA or condition adjunct to RA, in a lung in the individual, *e.g.,* painful breathing, shortness of breath *(e.g.,* due to pleural effusion), development or presence of lung nodules (rheumatoid nodules), scarring of the lungs (pulmonary fibrosis or bronchiolitis obliterans), viral infection of the lung, fibrosis of the lungs *(e.g.,* as a consequence of methotrexate therapy).

In certain embodiments, said method of treatment comprises an assessment of effectiveness of the administration of the placental stem cells. For example, in one embodiment, the method of treating a lung disease or disorder caused by, or associated with, RA comprises (1) administering a therapeutically effective amount of placental stem cells and/or culture medium conditioned by placental stem cells; and (2) assessing the individual for detectable improvement in one or more symptoms, or delay of onset of one or more symptoms, of the disease, disorder or condition of the lung associated with or caused by RA. In certain specific embodiments, said method of treating comprises a second (or further) administration of placental stem cells; to said individual, optionally followed by a second (or further) assessment of the individual for detectable improvement in one or more symptoms, or delay of onset of one or more symptoms, of the disease, disorder or condition of the lung associated with or caused by RA.

In a specific embodiment, the individual having a disease, disorder or condition of the lung associated with RA is administered therapeutically effective amount of placental stem cells; or culture medium conditioned by placental stem cells, and additionally at least one other therapeutic agent, *e.g.,* an analgesic or an anti-inflammatory agent. In more specific embodiments, the at least one other therapeutic agent is a disease-modifying antirheumatic drug (DMARD), *e.g.,* a xenobiotic (*e.g.,* azathioprine, cyclosporine A, D-pennicillamine, gold salts, hydroxychloroquine, leflunomide, methotrexate, minocycline or sulfasalazine) or a biological agent (*e*.*g*., tumor necrosis factor alpha (TNF-α) blockers, such as etanercept (ENBREL®), infliximab (REMICADE®), adalimumab (HUMIRA®); interleukin-1 blockers; anti-B cell (CD20) antibody (e.g., rituximab or RITUXAN®); or blockers of T cell activation (*e*.*g*., abatacept or ORENCIA®). In another more specific embodiment, the analgesic or anti-inflammatory agent is a glucocorticoid, a non-steroidal anti-inflammatory drug, acetaminophen, ibuprofen, aspirin, an opiate, or lidocaine (topical). Such therapeutic agents can be administered in any combination with the placental stem cells; at the same time or as a separate course of treatment.

In a specific embodiment, a plurality of the placental stem cells, administered to an individual having RA, are genetically engineered to express a polypeptide therapeutic for RA. In a more specific embodiment, the polypeptide therapeutic for RA is IL-1Ra (interleukin-1 receptor antagonist). In another more specific embodiment, the polypeptide therapeutic for RA is a fusion protein comprising IL-IRa and DHFR (dihydrofolate reductase). In a more specific embodiment, the placental stem cells are transformed with a nucleic acid encoding IL-1Ra-DHFR fusion protein, wherein expression of the fusion protein is enhanced by an antifolate, e.g., methotrexate. In an even more specific embodiment, the nucleic acid encodes IL-1Ra-DHFR-IRES-Luc, where IRES is an internal ribosomal entry site, and Luc is luciferase. In another specific embodiment, said nucleic acid comprises a nucleotide sequence that enables control of expression of the IL-IRa or IL-1Ra-DHFR fusion polypeptide.

### 4.1.3.3 Lung Disorders Associated with Lupus Erythematosus

In another embodiment, provided herein are placental stem cells for use in a method of treating an individual that has, or is experiencing a symptom of, a disease, disorder or condition of the lung associated with, or caused by, lupus erythematosus (LE), comprising administering to the individual a therapeutically effective amount of placental stem cells, or culture medium conditioned by placental stem cells, wherein said therapeutically effective amount is an amount that results in a detectable improvement in one or more symptoms, or delay of onset of one or more symptoms, of the disease, disorder or condition of the lung associated with or caused by LE. In certain embodiments, said administration results in at least stabilization of one or more symptoms of LE that manifest in a lung of the individual; that is, said one or more symptoms do not significantly improve, but do not significantly worsen, either. In specific embodiments, said symptoms comprise pleuritis (with or without effusion), lupus pneumonitis or chronic diffuse interstitial lung disease.

In certain embodiments, said method of treatment comprises identifying, in an individual having LE, a symptom of said LE in the lung; administering placental stem cells to said individual, e.g., to the affected lung of said individual; and assessing said individual for improvement in one or more parameters of lung function after said administering (e.g., from 7 days to 30 days afterwards), wherein said parameters of lung function are forced expiratory volume in one second (FEV₁); forced volume vital capacity (FVC); FEV₁/ FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement of one or more of said parameters of lung function (1) to 80% or more of expected; or (2) by at least 2%, 3%, 4%, 5%, 6%, 7%, 8%, 9%, 50%. In a more specific embodiment, said method comprises identifying any of said parameters that, prior to administration, are less than 80% of expected values for an individual of the same height and weight, and assessing said parameters after said administering, wherein said administering results in the improvement of one or more of said parameters of lung function (1) to 80% or more of expected; or (2) by at least 2%, 3%, 4%, 5%, 6%, 7%, 8%, 9%, 50%.

In certain embodiments, said method of treatment comprises an assessment of effectiveness of the administration of the placental stem cells. For example, in one embodiment, said method of treating a lung disease or disorder caused by, or associated with, LE comprises (1) administering a therapeutically effective amount of placental stem cells, or culture medium conditioned by placental stem cells; and (2) assessing the individual for detectable improvement in one or more symptoms, or delay of onset of one or more symptoms, of the disease, disorder or condition of the lung associated with or caused by LE. In certain specific embodiments, said method of treating comprises a second (or further) administration of placental stem cells; to said individual, optionally followed by a second (or further) assessment of the individual for detectable improvement in one or more symptoms, or delay of onset of one or more symptoms, of the disease, disorder or condition of the lung associated with or caused by LE.

### 4.1.3.4 Lung Disorders Associated with Scleroderma

In another embodiment, provided herein are placental stem cells for use in a method of treating an individual that has, or is experiencing a symptom of, a disease, disorder or condition of the lung associated with, or caused by, scleroderma, comprising administering to the individual a therapeutically effective amount of placental stem cells, or culture medium conditioned by placental stem cells, wherein said therapeutically effective amount is an amount that results in a detectable improvement in one or more symptoms, or delay of onset of one or more symptoms, of the disease, disorder or condition of the lung associated with or caused by scleroderma. In certain embodiments, said administration results in at least stabilization of one or more symptoms of scleroderma that manifest in a lung of the individual; that is, said one or more symptoms do not significantly improve, but do not significantly worsen, either. In specific embodiments, said symptoms comprise dyspnea (shortness of breath), interstitial lung disease (also referred to as fibrosing alveolitis or pulmonary fibrosis) or pulmonary vascular disease (alone or including pulmonary hypertension).

In certain embodiments, said method of treatment comprises identifying, in an individual having scleroderma, a symptom of said scleroderma in the lung; administering a therapeutically effective amount of placental stem cells, to said individual, e.g., to the affected lung of said individual; and assessing said individual for improvement in one or more parameters of lung function after said administering (e.g., from 7 days to 30 days afterwards), wherein said parameters of lung function are forced expiratory volume in one second (FEV₁); forced volume vital capacity (FVC); FEV₁/FVC; peak expiratory flow (PEF); forced expiratory flow 25%-50% or 25%-75% (average flow of air exiting the lung during the middle portion of the expiration); forced expiratory time (FET); total lung capacity (TLC); diffusing capacity, carbon monoxide (DLCO); or maximum voluntary ventilation. In a more specific embodiment, said administering results in improvement one of said parameters of lung function (1) to 80% or more of expected; or (2) by at least 2%, 3%, 4%, 5%, 6%, 7%, 8%, 9%, 50%. In a more specific embodiment, the method comprises identifying any of said parameters that, prior to administration, are less than 80% of expected values for an individual of the same height and weight, and assessing said parameters after said administering, wherein said administering results in the improvement one of said parameters of lung function (1) to 80% or more of expected; or (2) by at least 2%, 3%, 4%, 5%, 6%, 7%, 8%, 9%, 50%.

In certain embodiments, said method of treatment comprises an assessment of effectiveness of the administration of the placental stem cells. For example, in one embodiment, the method of treating a lung disease or disorder caused by, or associated with, scleroderma comprises (1) administering a therapeutically effective amount of placental cells or umbilical cord cells, i.e., placental stem cells, or culture medium conditioned by placental stem cells; and (2) assessing the individual for detectable improvement in one or more symptoms, or delay of onset of one or more symptoms, of the disease, disorder or condition of the lung associated with or caused by scleroderma. In certain specific embodiments, said method of treating comprises a second (or further) administration of placental stem cells to said individual, optionally followed by a second (or further) assessment of the individual for detectable improvement in one or more symptoms, or delay of onset of one or more symptoms, of the disease, disorder or condition of the lung associated with or caused by scleroderma.

In a specific embodiment, said individual is assessed for diffusing capacity for carbon monoxide (DLCO), and improvement in the individual comprises a significant improvement in DLCO after administration of placental stem cells, *(e.g.,* an increase of at least 3, 4, 5 or more percentage points) compared with DLCO prior to administration. In another specific embodiment, said individual is assessed for forced vital capacity (FVC), and improvement in the individual comprises an increase in FVC of at least 10% at least 15% or at least 20% after administration of the placental stem cells; compared with FVC prior to administration, e.g. for a period of at least 1, 2, 3, or 4 weeks, or 1, 2, 3, 4, 5, 6, 7, 8, 9, 10, 11 or 12 months after administration.

In another specific embodiment, the individual is assessed for lung scarring or pulmonary hypertension using, e.g., high resolution CT scan of the lungs, bronchoalveolar lavage, and/or surgical lung biopsy.

In a specific embodiment, the individual having a disease, disorder or condition of the lung associated with RA is administered placental stem cells, or culture medium conditioned by placental stem cells, and additionally at least one other therapeutic agent. In more specific embodiments, the therapeutic agent comprises, without limitation, an inhibitor of collagen synthesis and/or collagen crosslinking (e.g., penicillamine), a steroid (*e*.*g*., prednisone), cyclophosphamide (*e*.*g*., CYTOXAN®), a combination of cyclophosphamide and a steroid, a combination of cyclophosphamide and azathioprine, an antifibrotic (*e*.*g*., interferon-gamma (IFN-γ)), an antiendothelin (*e.g*., Bosentan (TRACLEER®)), a prostacyclin analog (*e*.*g*., epoprostenol (FLOLAN®), treprostinil (REMODULIN®)), and/or an endothelin receptor (*e*.*g*., endothelin receptor B) antagonist (*e*.*g*., sitaxentan, ambrisentan (LETAIRIS™)).

### 4.1.3.5 Determining Immunosuppressive Potential of Placental Cells

In certain optional embodiments, the capacity of a particular population of placental stem cells; for immunosuppression is determined prior to use, *e.g*., prior to administration to an individual having a disease, disorder or condition of the lung associated with or caused by an inappropriate or unwanted immune response. For example, the MLR can be used to determine the immunosuppressive capacity of a particular population of placental stem cells, *e*.*g*., a particular dose of placental stem cells. Procedures for performing the MLR and regression assays are well-known in the art. *See*, *e*.*g*. Schwarz, "The Mixed Lymphocyte Reaction: An In Vitro Test for Tolerance," J. Exp. Med. 127(5):879-890 (1968); Lacerda et al., "Human Epstein-Barr Virus (EBV)-Specific Cytotoxic T Lymphocytes Home Preferentially to and Induce Selective Regressions of Autologous EBV-Induced B Lymphoproliferations in Xenografted C.B-17 Scid/Scid Mice," J. Exp. Med. 183:1215-1228 (1996). In a preferred embodiment, an MLR is performed in which a plurality of placental stem cells are contacted with a plurality of immune cells (e.g., lymphocytes, for example, CD3⁺, CD4⁺ and/or CD8⁺ T lymphocytes). For example, a plurality of placental cells can be tested in an MLR comprising combining CD4⁺ or CD8⁺ T cells, dendritic cells (DC) and placental cells in a ratio of about 10:1:2, wherein the T cells are stained with a dye such as, *e.g.,* CFSE that partitions into daughter cells, and wherein the T cells are allowed to proliferate for about 6 days. The plurality of placental cells is immunosuppressive if the T cell proliferation at 6 days in the presence of placental cells is detectably reduced compared to T cell proliferation in the presence of DC and absence of placental cells. In such an MLR, placental cells are either thawed or harvested from culture. About 20,000 placental cells, e.g., placental stem cells, are resuspended in 100 µl of medium (RPMI 1640, 1 mM HEPES buffer, antibiotics, and 5% pooled human serum), and allowed to attach to the bottom of a well for 2 hours. CD4⁺ and/or CD8⁺ T cells are isolated from whole peripheral blood mononuclear cells using Miltenyi magnetic beads. The cells are CFSE stained, and a total of 100,000 T cells (CD4⁺ T cells alone, CD8⁺ T cells alone, or equal amounts of CD4⁺ and CD8⁺ T cells) are added per well. The volume in the well is brought to 200µl, and the MLR is allowed to proceed.

### 4.1.4 Treatment of Other Lung Diseases or Disorders

Also provided herein are placental stem cells for use in methods of treating lung diseases, disorders or conditions arising from other causes, comprising administering to an individual having said disease, disorder or condition, e.g., to an affected lung of said individual, a therapeutically effective amount of placental stem cells. For example, in certain embodiments, provided herein is a method of treating an individual having a disease, disorder or condition affecting the lungs, wherein said disease, disorder, or condition of the lung is an acute lung injury. In specific embodiments, said acute lung injury is one or more of physical trauma, injury due to drug or chemotherapeutic toxicity (e.g., toxicity due to treatment with bleomycin, cyclophosphamide, nitrofurantoin, methotrexate, combination 5-fluorouracil and oxaliplatinum therapy or the like), a radiation-induced injury, a chemical injury, *e.g.,* a chemical burn, smoke inhalation, exposure to a toxic substance, or chemically-induced pneumonia.

In certain other embodiments, said lung disease, disorder, or condition is an injury caused by a neoplastic or paraneoplastic disease.

In still other embodiments, the disease, disorder or condition affecting the lung is a viral or bacterial infection of the lung (e.g., pneumonia), an infectious lung disease, or cystic fibrosis.

In specific embodiments, the disease, disorder or condition of the lung is an environmental lung disease, a granulomatous disease, an obstructive disease, a vascular disease, a neoplasm, or a pleural disorder.

### 4.1.5 Second Therapeutic Compositions and Second Therapies

In any of the above aspects of the invention, the method can comprise the administration of a second therapeutic composition or second therapy. Second therapeutic compositions or second therapies can consist of, or comprise, the specific second therapeutic compositions or second therapies listed above for the lung-related diseases, disorders or conditions discussed in Sections 4.1.2-4.1.4, above. However, the recitation of specific second therapeutic compounds or second therapies in the methods of treating specific diseases, above, are not intended to be exclusive. For example, any of the diseases, disorders or conditions discussed herein can be treated with any of the anti-inflammatory compounds or immunosuppressive compounds described herein.

In embodiments in which placental stem cells, are administered with a second therapeutic agent, *e.g.,* a second type of cell, the placental stem cells and second therapeutic agent can be administered at the same time or different times, *e.g.,* the administrations can take place within 1, 2, 3, 4, 5, 6, 7, 8, 9 10, 20, 30, 40, or 50 minutes of each other, or 1, 2, 3, 4, 5, 6, 7, 8, 9, 10, 12, 14, 16, 18, 20, or 22 hours of each other, or within 1, 2, 3, 4, 5, 6, 7 8, 9 or 10 days of each other.

Said second therapy may comprise an immunomodulatory compound, wherein the immunomodulatory compound is a compound having the structure 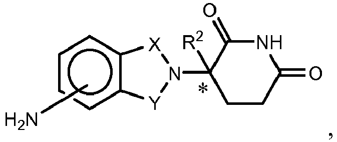 wherein one of X and Y is C=O, the other of X and Y is C=O or CH₂, and R² is hydrogen or lower alkyl, or a pharmaceutically acceptable salt, hydrate, solvate, clathrate, enantiomer, diastereomer, racemate, or mixture of stereoisomers thereof. In another more specific embodiment, said immunomodulatory compound is a compound having the structure 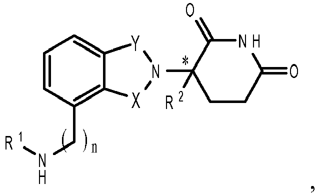 wherein one of X and Y is C=O and the other is CH₂ or C=O;
R¹ is H, (C₁-C₈ )alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴, (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center;
or a pharmaceutically acceptable salt, hydrate, solvate, clathrate, enantiomer, diastereomer, racemate, or mixture of stereoisomers thereof. Said immunomodulatory compound may be a compound having the structure 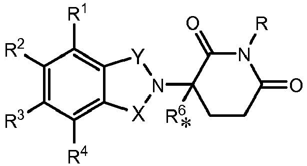 wherein:
one of X and Y is C=O and the other is CH₂ or C=O;
R is H or CH₂OCOR';
   (i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X₁CH₂CH₂- in which X₁ is -O-, -S-, or -NH-;
R¹⁰ is hydrogen, alkyl of to 8 carbon atoms, or phenyl; and
* represents a chiral-carbon center;
or a pharmaceutically acceptable salt, hydrate, solvate, clathrate, enantiomer, diastereomer, racemate, or mixture of stereoisomers thereof.

The immunomodulatory compound may be 3-(4-amino-1-oxo-1,3-dihydroisoindol-2-yl)-piperidine-2,6-dione (lanalidomide); 3-(4'aminoisolindoline-1'-one)-1-piperidine-2,6-dione; 4-(Amino)-2-(2,6-dioxo(3-piperidyl))-isoindoline-1 ,3-dione (pomalidomide); or α-(3-aminophthalimido) glutarimide (lenalidomide).

Placental stem cells can be administered to an individual suffering a disease, disorder or condition of the lung in the form of a pharmaceutical composition, e.g., a pharmaceutical composition suitable for intravenous, intramuscular or intraperitoneal injection. Placental stem cells can be administered in a single dose, or in multiple doses. Where placental stem cells are administered in multiple doses, the doses can be part of a therapeutic regimen designed to relieve one or more acute symptoms of a lung disease, condition, or disorder, or can be part of a long-term therapeutic regimen designed to prevent, or lessen the severity, of a chronic course of the disease. In embodiments in which placental stem cells are administered with a second therapeutic agent, or with a second type of cell, the placental stem cells and second therapeutic agent and/or second type of stem cell can be administered at the same time or different times, *e.g.,* the administrations can take place within 1, 2, 3, 4, 5, 6, 7, 8, 9 10, 20, 30, 40, or 50 minutes of each other, or 1, 2, 3, 4, 5, 6, 7, 8, 9, 10, 12, 14, 16, 18, 20, or 22 hours of each other, or within 1, 2, 3, 4, 5, 6, 7 8, 9 or 10 days of each other.

### 4.1.6 Administration of Placental Cells

Administration of the placental stem cells; can be by any medically acceptable route. In specific embodiments, placental stem cells; are administered intravenously, intra-arterially, parenterally, subcutaneously, intramuscularly, intraperitoneally, or the like. The placental stem cells; can be administered systemically, or directly to an affected area of the lung. In certain embodiments, the placental stem cells; are administered to the individual by inhalation, *e*.*g*., in a spray, aerosol, by external or mechanical ventilation, or by direct application (*e.g.,* injection or topical application) to a part of the lung, *e.g.,* trachea, bronchus, bronchiole, lobule, alveolus, or the like.

For *in vivo* administration, placental stem cells; can be formulated as a pharmaceutical composition, as described below.

In one embodiment, the individual is administered a dose of about 200 million placental cells. Dosage, however, can vary according to the individual's physical characteristics, *e.g.,* weight, and can range from 1 million to 10 billion placental cells, *e.g.,* placental stem cells, per dose, preferably between 10 million and 1 billion per dose, or between 100 million and 50 million placental stem cells, per dose.

Administration during a course of treatment of an individual having a disease, disorder or condition of the lung can comprise a single administration of placental stem cells, or multiple administrations of placental stem cells. If more than one administration of placental stem cells is used in the course of treatment, the individual can be given the same approximate number of placental stem cells, per administration, or can be given different numbers of placental stem cells. For example, in one embodiment, an individual suffering from a disease, disorder or condition of the lung is administered an initial, relatively large dose of placental stem cells, while subsequent administrations comprise administration of a relatively smaller number of placental stem cells, e.g., about 5%, 10%, 15%, 20%, 25%, 30%, 35%, 40%, 45%, 50%, 55%, 60%, 65%, 70%, 75%, 80%, 85%, 90% or 95% of the number of cells in the initial dose.

### 4.2 PLACENTAL CELLS AND PLACENTAL CELL POPULATIONS

The placental stem cells for use in methods of treatment of diseases, disorders or conditions of the lung, provided herein comprise administration of placental stem cells, to an individual having the disease, disorder or condition. Placental stem cells, useful in the methods of treatment described herein, and methods of obtaining and culturing such cells, are described, e.g., in U.S. Patent Nos. 7,045,148; 7,255,879; 7,311,904; and 7,311,905; and in U.S. Patent Application Publication Nos. 2007/0275362 and 2008/0226595.

The placental stem cells, can be either fetal or maternal in origin (that is, can have the genotype of either the mother or fetus). Populations of placental stem cells, or populations of cells comprising placental cells, can comprise placental cells that are solely fetal or maternal in origin, or can comprise a mixed population of placental cells of both fetal and maternal origin. The placental stem cells, and populations of cells comprising the placental cells, can be identified and selected by the morphological, marker, and culture characteristics discussed below.

### 4.2.1 Physical and Morphological Characteristics

The placental stem cells useful in the methods of treating lung diseases, disorders or conditions disclosed herein, when cultured in primary cultures or in cell culture, adhere to the tissue culture substrate, *e*.*g*., tissue culture container surface (*e.g*., tissue culture plastic). The cells in culture assume a generally fibroblastoid, stellate appearance, with a number of cyotplasmic processes extending from the central cell body. The cells are, however, morphologically differentiable from fibroblasts cultured under the same conditions. For example, the cells generally exhibit a greater number of such processes than do fibroblasts grown under the same conditions. Morphologically, the cells are also distinguishable from hematopoietic stem cells, which generally assume a more rounded, or cobblestone, morphology in culture.

### 4.2.2 Cell Surface, Molecular and Genetic Markers

The isolated placental stem cells, and populations of isolated placental stem cells, useful in the methods of treatment disclosed herein, are tissue culture plastic-adherent human placental cells or umbilical cord cells that have characteristics of multipotent cells or stem cells, and express a plurality of markers that can be used to identify and/or isolate the cells, or populations of cells that comprise the stem cells. The isolated placental stem cells, and cell populations comprising placental stem cells (that is, two or more isolated cells), described herein, include cells and cell-containing cell populations obtained directly from the placenta, or any part thereof (e.g., amnion, chorion, placental cotyledons, and the like) or umbilical cord. Isolated placental stem cell populations also include populations of (that is, two or more) isolated placental stem cells in culture, and a population in a container, *e*.*g*., a bag.

The isolated placental stem cells described herein are not bone marrow-derived mesenchymal cells, adipose-derived mesenchymal stem cells, or mesenchymal cells obtained from umbilical cord blood, placental blood, or peripheral blood. As used herein, the term "placental stem cells" encompass any of the stem cells or multipotent cells described in this section. The isolated placental stem cells are also not trophoblasts. Trophoblasts in culture typically form multinucleated cells called syncytiotrophoblasts; placental stem cells, in contrast, do not form multinucleated cells in culture, but instead expand as a population of uninuclear cells, similar to the expansion of fibroblasts.

In certain embodiments, the placental stem cells are CD34⁻, CD10⁺, CD105⁺, CD200⁺ isolated stem cells. In certain other embodiments, said placental stem cells are isolated multipotent cells. In one embodiment, the placental stem cells are CD34⁻, CD10⁺ CD200⁺, and CD105⁺ as detected by flow cytometry. In another specific embodiment, the isolated CD34⁻, CD10⁺, CD105⁺, CD200⁺ placental stem have the potential to differentiate into cells of a neural phenotype, cells of an osteogenic phenotype, and/or cells of a chondrogenic phenotype. In a more specific embodiment, the isolated CD34⁻, CD10⁺, CD105⁺, CD200⁺ cells are additionally CD90⁺ or CD45⁻, as detected by flow cytometry. In another more specific embodiment, the isolated CD34⁻, CD10⁺, CD105⁺, CD200⁺ cells are additionally CD90⁺ and CD45⁻, as detected by flow cytometry, *i*.*e*., the cells are CD34⁻, CD10⁺, CD45⁻, CD90⁺, CD105⁺ and CD200⁺. In a more specific embodiment, said CD34⁻, CD10⁺, CD45⁻, CD90⁺, CD105⁺, CD200⁺ cells are additionally CD80⁻ and CD86⁻.

In a specific embodiment, any of the CD34⁻, CD10⁺, CD105⁺, CD200⁺ placental stem cells described above are additionally one or more of CD29⁺, CD38⁻, CD44⁺, CD54⁺, SH3⁺ or SH4⁺. In another more specific embodiment, the cells are additionally CD44⁺. In another specific embodiment of any of the isolated CD34⁻, CD10⁺, CD105⁺ CD200⁺ placental stem cells above, the cells are additionally one or more of CD117⁻, CD133⁻, KDR (VEGFR2⁻), HLA-A,B,C⁺, HLA-DP,DQ,DR⁻, or Programmed Death-1 Ligand (PDL1)⁺, or any combination thereof.

In another embodiment, the CD34⁻, CD10⁺, CD105⁺, CD200⁺ placental stem cells are additionally one or more of CD13⁺, CD29⁺, CD33⁺, CD38⁻, CD44⁺, CD45⁻, CD54⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁺, or Programmed Death-1 Ligand (PDL1)⁺, or any combination thereof. In another embodiment, the CD34⁻, CD10⁺, CD105⁺, CD200⁺ placental stem cells are additionally CD13⁺, CD29⁺, CD33⁺, CD38⁻, CD44⁺, CD45⁻, CD54/ICAM⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻(VEGFR2⁻), HLA-A,B,C⁺, HLA-DP,DQ,DR⁻, HLA-G⁺, and Programmed Death-1 Ligand (PDL1)⁺.

In certain embodiments, the cells are additonnally one or more of SSEA3⁻, SSEA4⁻ or ABC-p⁺. The isolated cells can also express HLA-ABC (MHC-1). These markers can be used, in any combination, to identify the isolated cells, *e.g.,* isolated stem cells or isolated multipotent cells and to distinguish the isolated cells from other cell types. Lack of expression of CD34, CD38 and/or CD45, for example, identifies the isolated cells as non-hematopoietic stem cells.

Also provided herein are populations of placental stem cells, that are useful in the methods of treatment disclosed herein. Some populations of cells comprising placental stem cells, wherein the populations of cells are useful in the methods of treatment disclosed herein, comprise, e.g., at least 10%, 15%, 20%, 25%, 30%, 35%, 40%, 45%, 50%, 55%, 60%, 65%, 70%, 75%, 80%, 85%, 90%, 95% or 98% placental stem cells, that are CD10⁺,CD105⁺, CD200⁺ and CD34⁻ cells; that is, at least 10%, 15%, 20%, 25%, 30%, 35%, 40%, 45%, 50%, 55%, 60%, 65%, 70%, 75%, 80%, 85%, 90%, 95% or 98% of cells in said population are placental stem cells, that are CD10⁺, CD105⁺ CD200⁺ and CD34⁻ cells. In a more specific embodiment, the isolated CD34⁻, CD10⁺, CD105⁺, CD200⁺ cells are additionally CD90⁺ or CD45⁻, as detected by flow cytometry. In another more specific embodiment, the isolated CD34⁻, CD10⁺, CD105⁺, CD200⁺ cells are additionally CD90⁺ and CD45⁻, as detected by flow cytometry. In a more specific embodiment, any of the isolated CD34⁻, CD10⁺, CD105⁺, CD200⁺ cells described above are additionally one or more of CD29⁺, CD38⁻, CD44⁺, CD54⁺, SH3⁺ or SH4⁺. In another more specific embodiment, the isolated CD34⁻, CD10⁺, CD105⁺, CD200⁺ cells, are additionally CD44⁺. In a specific embodiment of any of the populations of cells comprising isolated CD34⁻, CD10⁺, CD105⁺, CD200⁺ cells above, the isolated cells are additionally one or more of CD13⁺, CD29⁺, CD33⁺, CD38⁻, CD44⁺, CD45⁻, CD54⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁺, or Programmed Death-1 Ligand (PDL1)⁺, or any combination thereof. In a more specific embodiment, the CD34⁻, CD10⁺, CD105⁺, CD200⁺ cells are additionally CD13⁺, CD29⁺, CD33⁺, CD38⁻, CD44⁺, CD45⁻, CD54/ICAM⁺, CD62E⁻, CD62L⁻, CD62P⁻, SH3⁺ (CD73⁺), SH4⁺ (CD73⁺), CD80⁻, CD86⁻, CD90⁺, SH2⁺ (CD105⁺), CD106/VCAM⁺, CD117⁻, CD144/VE-cadherin^{low}, CD184/CXCR4⁻, CD200⁺, CD133⁻, OCT-4⁺, SSEA3⁻, SSEA4⁻, ABC-p⁺, KDR⁻ (VEGFR2⁻), HLA-A,B,C⁺, HLA-DP,DQ,DR⁻, HLA-G⁺, and Programmed Death-1 Ligand (PDL1)⁺.

In certain embodiments, placental stem cells for use in the methods of treatment described herein are isolated cells that are CD10⁺, CD34⁻, CD200⁺, CD105⁺, and one or more, or all, of CD29⁺, CD38⁻, CD44⁺, CD45⁻, CD54⁺, CD90⁺, SH2⁺, SH3⁺, SH4⁺, SSEA3⁻, SSEA4⁻, OCT-4⁺, and ABC-p⁺, wherein said isolated cells are obtained by physical and/or enzymatic disruption of placental tissue or umbilical cord tissue. In a specific embodiment, said isolated cells are additionally OCT-4⁺ and ABC-p⁺. In another specific embodiment, said isolated cells are also OCT-4⁺, wherein said isolated cells have at least one of the following characteristics: CD29⁺, CD44⁺, CD45⁻, CD54⁺, CD90⁺, SH3⁺, SH4⁺, SSEA3⁻, and SSEA4⁻. In another specific embodiment, said isolated cells are OCT-4^{+,} CD29⁺, CD44⁺, CD45⁻, CD54⁺, CD90⁺, SH3⁺, SH4⁺, SSEA3⁻, and SSEA4⁻. In another embodiment, said isolated cells are OCT-4⁺, SSEA3⁻, and SSEA4⁻. In a more specific embodiment, said isolated cells are OCT-4⁺ and either SH2⁺ or SH3⁺. In a more specific embodiment, said isolated cells are OCT-4⁺, SH2⁺, and SH3⁺. In another more specific embodiment, said isolated cells are OCT-4⁺, SSEA3⁻, and SSEA4⁻, and are either SH2⁺ or SH3⁺. In another more specific embodiment, said isolated cells are OCT-4⁺ and either SH2⁺ or SH3⁺, and at least one of CD29⁺, CD44⁺, CD45⁻, CD54⁺, CD90⁺, SSEA3⁻, or SSEA4⁻. In another more specific embodiment, said isolated cells are OCT-4⁺, CD29⁺, CD44⁺, CD45⁻, CD54⁺, CD90⁺, SSEA3⁻, and SSEA4⁻, and either SH2⁺ or SH3⁺.

In another embodiment, the CD10⁺, CD34⁻, CD200⁺, CD105⁺ placental stem cells for use in the methods of treatment disclosed herein are optionnally SH2⁺, SH3⁺, SH4⁺ and OCT-4⁺ cells. In a more specific embodiment, said isolated cells are optionally CD29⁺, CD44⁺, CD54⁺, CD90⁺, CD45⁻, SSEA3⁻, or SSEA4⁻. In another embodiment, said isolated cells are optionally SH2⁺, SH3⁺, SH4⁺, SSEA3⁻ and SSEA4⁻. In a more specific embodiment, said isolated cells are optionally SH2⁺, SH3⁺, SH4⁺, SSEA3⁻ and SSEA4⁻, CD29⁺, CD44⁺, CD54⁺, CD90⁺, OCT-4⁺, or CD45⁻.

In another embodiment, the CD10⁺, CD34⁻, CD200⁺, CD105⁺ placental stem cells for use in the methods disclosed herein are optionally CD29^{+,} CD44^{+,} CD45⁻, CD54⁺, CD90⁺, SH2⁺, SH3⁺, and SH4⁺; are additionally one or more of OCT-4⁺, SSEA3⁻ or SSEA4⁻.

In certain embodiments, the CD10⁺, CD34⁻, CD200⁺, CD105⁺ placental stem cells useful in the methods of treatment disclosed herein are optionally HLA-G⁺. In another specific embodiment, said cells are additionally CD73⁺. In another specific embodiment, said cells are additionally CD38⁻ or CD45⁻. In another specific embodiment, said cells are additionally CD38⁻ and CD45⁻. In another specific embodiment, said cells are CD38⁻, CD45⁻, CD73⁺. In another specific embodiment, said isolated cells facilitate the formation of embryoid-like bodies in a population of placental cells comprising the cells, under conditions that allow the formation of embryoid-like bodies. In another specific embodiment, the placental stem cells are isolated away from placental cells or umbilical cord cells that are not stem or multipotent cells. In another specific embodiment, the placental stem cells are isolated away from placental stem cells that do not display the markers described above.

A cell population useful in the methods of treatment described herein is an isolated population of cells comprising, *e.g*., that is enriched for, CD200⁺, HLA-G⁺ placental stem cells. Said population is a population of placental cells. Said population is a population of umbilical cord cells. For example, at least about 10%, at least about 20%, at least about 30%, at least about 40%, at least about 50%, or at least about 60% of cells in said cell population are isolated CD200⁺, HLA-G⁺ cells. In some examples, at least about 70% of cells in said cell population are isolated CD200⁺, HLA-G ⁺ cells. In other examples, at least about 80%, 90%, 95%, or 99% of said cells are isolated CD200⁺, HLA-G⁺ cells. In another example of the cell populations, said isolated CD200⁺, HLA-G⁺ cells are also CD73⁺ and CD105⁻. In another example, said isolated CD200⁺, HLA-G⁺ cells are also CD34⁻, CD38⁻ or CD45⁻. In another example, said isolated CD200⁺, HLA-G⁺ cells are also CD34⁻, CD38⁻, CD45⁻, CD73⁺ and CD105⁺. In another example, said cell population produces one or more embryoid-like bodies when cultured under conditions that allow the formation of embryoid-like bodies. In another example, the CD200⁺, HLA-G⁺ cells produces one or more embryoid-like bodies when cultured under conditions that allow the formation of embryoid-like bodies. In another example, said cell population is isolated away from cells that are not stem cells. In another example, said isolated CD200⁺, HLA-G⁺ cells are isolated away from placental cells or umbilical cord cells that do not display these markers.

In another embodiment, placental stem cells for use in the methods of treatment described herein are isolated CD10⁺, CD34⁻, CD105⁺, and CD200⁺ cells. In another specific embodiment, the cells are also HLA-G⁺. In another specific embodiment, said isolated cells are also CD38⁻ or CD45⁻. In another specific embodiment, said cells are also CD38⁻ and CD45⁻. In a more specific embodiment, said cells are also CD38⁻, CD45⁻, and HLA-G⁺. In another specific embodiment, said isolated CD10⁺, CD34⁻, CD105⁺, and CD200⁺ cells facilitate the formation of one or more embryoid-like bodies in a population of placental cells or umbilical cord cells comprising the placental stem cells, when the population is cultured under conditions that allow the formation of embryoid-like bodies. In another specific embodiment, said isolated placental cells are isolated away from placental cells or umbilical cord cells that do not display these markers.

In another embodiment, a cell population useful in the methods of treatment described herein is an isolated population of cells comprising, *e.g*., that is enriched for, isolated CD10⁺, CD34⁻, CD105⁺, CD200⁺ placental stem cells. In a specific embodiment, said population is a population of placental cells. In another specific embodiment, said population is a population of umbilical cord cells. In various embodiments, at least about 10%, at least about 20%, at least about 30%, at least about 40%, at least about 50%, or at least about 60% of cells in said cell population are said CD10⁺, CD34⁻, CD105⁺, CD200⁺ cells. In another embodiment, at least about 70% of said cells in said cell population are isolated CD10⁺, CD34⁻, CD105⁺, CD200⁺ cells. In another embodiment, at least about 80%, 90%, 95% or 99% of cells in said cell population are isolated CD10⁺, CD34⁻, CD105⁺, CD200⁺ cells. In a specific embodiment of said cell population, the isolated cells are HLA-G⁺. In another specific embodiment, the isolated cells are additionally CD34⁻, CD38⁻ or CD45⁻. In another specific embodiment, the isolated cells are additionally CD34⁻, CD38⁻ and CD45⁻. In a more specific embodiment, the isolated cells are additionally CD34⁻, CD38⁻, CD45⁻, and HLA-G⁺. In another specific embodiment, said cell population produces one or more embryoid-like bodies when cultured under conditions that allow the formation of embryoid-like bodies. In another specific embodiment, said cell population is isolated away from cells that are not stem cells. In another specific embodiment, said isolated CD10⁺, CD34⁻, CD105⁺, CD200⁺ cells are isolated away from placental cells or umbilical cord cells that do not display these markers.

In certain other embodiments, the CD10⁺, CD34⁻, CD200⁺, CD105⁺ placental stem cells are isolated placental or umbilical cord cells that are additionally one or more of CD29⁺, CD38⁻, CD44⁺, CD45⁻, CD54⁺, CD90⁺, SH2⁺, SH3⁺, SH4⁺, SSEA3-, SSEA4⁻, OCT-4⁺, HLA-G⁺ or ABC-p⁺. In a specific embodiment, said cells are CD29⁺, CD38⁻, CD44⁺, CD45⁻, CD54⁺, CD90⁺, SH2⁺, SH3⁺, SH4⁺, SSEA3-, SSEA4⁻, and OCT-4⁺. In another specific embodiment, said cells are CD29⁺, CD38⁻, CD45⁻, CD54⁺, SH2⁺, SH3⁺, and SH4⁺. In another specific embodiment, said cells are CD29⁺, CD38⁻, CD45⁻, CD54⁺, SH2⁺, SH3⁺, SH4⁺ and OCT-4⁺. In another specific embodiment, said cells are CD29⁺, CD38⁻, CD44⁺, CD45⁻, CD54⁺, CD90⁺, HLA-G⁺, SH2⁺, SH3⁺, SH4⁺. In another specific embodiment, said cells are OCT-4⁺ and ABC-p⁺. In another specific embodiment, said cells are SH2⁺, SH3⁺, SH4⁺ and OCT-4⁺. In another embodiment, said cells are OCT-4⁺, CD34⁻, SSEA3⁻, and SSEA4⁻. In a specific embodiment, said cells are OCT-4⁺, CD34⁻, SSEA3⁻, and SSEA4⁻ and additionally CD29⁺, CD44⁺, CD45⁻, CD54⁺, CD90⁺, SH2⁺, SH3⁺, and SH4⁺. In another embodiment, said cells are OCT-4⁺ and CD34⁻, and either SH3⁺ or SH4⁺. In another embodiment, said cells additionally either CD10⁺, CD29⁺, CD44⁺, CD54⁺, CD90⁺, or OCT-4⁺.

In another embodiment, the CD10⁺, CD34⁻, CD200⁺, CD105⁺ placental stem cells for use in the methods of treatment described herein are also OCT-4⁺ cells. In a specific embodiment, said cells are also CD73⁺. In another specific embodiment, said cells are also HLA-G⁺. In another specific embodiment, said isolated OCT-4⁺ cells are also CD38⁻ or CD45⁻. In another specific embodiment, said isolated OCT-4⁺ cells are CD38⁻ and CD45⁻. In a more specific embodiment, said isolated OCT-4⁺ cells are CD38⁻, CD45⁻, CD73⁺, and HLA-G⁺. In another specific embodiment, said isolated OCT-4⁺ cells facilitate the production of one or more embryoid-like bodies by a population of placental cells or umbilical cord cells that comprises the placental stem cells, when the population is cultured under conditions that allow the formation of embryoid-like bodies. In another specific embodiment, said isolated OCT-4⁺ cells are isolated away from placental cells that are not stem cells or multipotent cells. In another specific embodiment, said isolated OCT-4⁺ cells are isolated away from placental cells that do not display these markers.

A cell population useful in the methods of treatment described herein is an isolated population of cells comprising, *e.g*., that is enriched for, CD200⁺, OCT-4⁺ placental stem cells. In some examples, at least about 10%, at least about 20%, at least about 30%, at least about 40%, at least about 50%, or at least about 60% of cells in said cell population are the isolated CD200⁺, OCT-4⁺ cells. In another example, at least about 70% of said cells are the isolated CD200⁺, OCT-4⁺ cells. In another example, at least about 80%, 90%, 95%, or 99% of cells in said cell population are said isolated CD200⁺, OCT-4⁺ cells. In one example of the isolated populations, said isolated CD200⁺, OCT-4⁺ cells are additionally CD73⁺ and CD105⁺. In another example, said isolated CD200⁺, OCT-4⁺ cells are additionally HLA-G⁺. In another example, said isolated CD200⁺, OCT-4⁺ cells are additionally CD34⁻, CD38⁻ and CD45⁻. In a more specific example, said isolated CD200 , OCT-4 cells are additionally CD34⁻, CD38⁻, CD45⁻, CD73⁺, CD105⁺ and HLA-G⁺. In another specific example, the cell population produces one or more embryoid-like bodies when cultured under conditions that allow the formation of embryoid-like bodies.

In another embodiment, the CD10⁺, CD64⁻, CD200⁺, CD105⁺ placental stem cells for use in the methods of treatment described herein are also CD73⁺ and HLA-G⁺ cells. In another specific embodiment, said placental stem cells are addittionally CD38⁻ or CD45⁻. In another specific embodiment, said placental stem cells are additionally CD38⁻ and CD45⁻. In another specific embodiment, said placental stem are additionally OCT-4⁺. In a more specific embodiment, said placental stem cells are additionally CD38⁻, CD45⁻, OCT-4⁺. In another specific embodiment, said placental stem cells facilitate the formation of embryoid-like bodies in a population of placental cells comprising said cells, when the population is cultured under conditions that allow the formation of embryoid-like bodies. In another specific embodiment, said placental stem cells are isolated away from placental cells that are not the isolated CD73⁺, CD105⁺, HLA-G⁺ placental cells. In another specific embodiment, said placental stem cells are isolated away from placental cells that do not display these markers.

A cell population useful in the methods of treatment described herein is an isolated population of cells comprising, *e.g*., that is enriched for, isolated CD73⁺, CD105⁺ and HLA-G⁺ placental stem cells. In some examples, at least about 10%, at least about 20%, at least about 30%, at least about 40%, at least about 50%, or at least about 60% of cells in said population of cells are CD73⁺, CD105⁺, HLA-G⁺ cells. In another example, at least about 70% of cells in said population of cells are isolated CD73 , CD105⁺, HLA-G⁺ cells. In another example, at least about 80%, 90%, 95% or 99% of cells in said population of cells are isolated CD73⁺, CD105⁺, HLA-G⁺ cells. In one example of the above populations, said isolated CD73⁺, CD105⁺, HLA-G⁺ cells are additionally CD34⁻, CD38⁻ or CD45⁻. In another example, said isolated CD73⁺, CD105⁺, HLA-G⁺ cells are additionally CD34⁻, CD38⁻ and CD45⁻. In another example said isolated CD73⁺, CD105⁺, HLA-G⁺ cells are additionally OCT-4⁺. In another example, said isolated CD73⁺, CD105⁺, HLA-G⁺ cells are additionally CD200⁺. In another example, said isolated CD73⁺, CD105⁺, HLA-G⁺ cells are additionally CD34⁻, CD38⁻, CD45⁻, OCT-4⁺ and CD200⁺.

In another embodiment the CD10⁺, CD34⁻, CD200⁺, CD105⁺ placental stem cells for use in the methods of treatment described herein are optionally CD73⁺ and facilitate the formation of one or more embryoid-like bodies in a population of isolated cells comprising said CD73⁺, CD105⁺ cells when said population is cultured under conditions that allow formation of embryoid-like bodies. In another specific embodiment, said cells are additionally CD34⁻, CD38⁻ or CD45⁻. In another specific embodiment, said cells are additionally CD34⁻, CD38⁻ and CD45⁻. In another specific embodiment, said cells are additionally OCT-4⁺. In a more specific embodiment, said cells are additionally OCT-4⁺, CD34⁻, CD38⁻ and CD45⁻. In another specific embodiment, said cells are isolated away from placental cells or umbilical cord cells that do not display these characteristics.

A cell population useful in the methods of treatment described herein is a population of cells comprising, e.g., that is enriched for, isolated placental stem cells that are CD73⁺, CD105⁺ and facilitate the formation of one or more embryoid-like bodies in a population of isolated placental cells or umbilical cord cells comprising said cells when said population is cultured under conditions that allow formation of embryoid-like bodies. For example, at least about 10%, at least about 20%, at least about 30%, at least about 40%, at least about 50%, or at least about 60% of cells in said population of cells are said isolated CD73⁺, CD105⁺ cells. In another example, at least about 70% of cells in said population of cells are said isolated CD73⁺, CD105⁺ cells. In another example, at least about 80%, 90%, 95% or 99% of cells in said population of cells are said isolated CD73⁺, CD105⁺ placental cells. In another example of the above populations, said isolated CD73⁺, CD105⁺ cells are additionally CD34⁻, CD38⁻ or CD45⁻. In another example, said isolated CD73⁺, CD105⁺ cells are additionally CD34⁻, CD38⁻ and CD45⁻. In another example, said isolated CD73⁺, CD105⁺ cells are additionally OCT-4⁺. In another example, said isolated CD73⁺, CD105⁺ cells are additionally CD200⁺. In another example, said isolated CD73⁺, CD105⁺ cells are additionally CD34⁻, CD38⁻, CD45⁻, OCT-4⁺ and CD200⁺. In another example, said cell population is isolated away from placental cells that do not display these markers.

In another embodiment, the CD10⁺, CD34⁻, CD200⁺, CD105⁺ placental stem cells for use in the methods of treatment described herein are additionally OCT-4⁺ that facilitate formation of one or more embryoid-like bodies in a population of isolated placental cells comprising said cells when cultured under conditions that allow formation of embryoid-like bodies. In a specific embodiment, stem said isolated OCT-4⁺ placental stem cells are additionally CD73⁺. In another specific embodiment, said isolated OCT-4⁺ placental stem cells are additionally CD38⁻, or CD45⁻. In a more specific embodiment, said isolated OCT-4⁺ placental stem cells are additionally CD73⁺, CD200⁺, CD38⁻, and CD45⁻. In another specific embodiment, said isolated OCT-4⁺ placental stem cells, are isolated away from placental cells that are not OCT-4⁺ placental cells. In another specific embodiment, said isolated OCT-4⁺ placental stem cells are isolated away from placental cells that do not display these characteristics.

A cell population useful in the methods of treatment described herein is a population of cells comprising, *e.g*., that is enriched for, placental stem cells that are OCT-4⁺ and facilitate the formation of one or more embryoid-like bodies in a population of isolated placental cells or umbilical cord cells comprising said cells when said population is cultured under conditions that allow formation of embryoid-like bodies. In some example, at least about 10%, at least about 20%, at least about 30%, at least about 40%, at least about 50%, or at least about 60% of cells in said population of cells are said isolated OCT-4⁺ cells. In another example, at least about 70% of cells in said population of cells are said isolated OCT-4⁺ cells. In another example, at least about 80%, 90%, 95% or 99% of cells in said population of cells are said isolated OCT-4⁺ cells. In a specific example of the above populations, said isolated OCT-4⁺ cells are additionally CD34⁻, CD38⁻ or CD45⁻. In another example, said isolated OCT-4⁺ cells are additionally CD34⁻, CD38⁻ and CD45⁻. In another example, said isolated OCT-4⁺ cells are additionally CD73⁺ and CD105⁺. In another example, said isolated OCT-4⁺ cells are additionally CD200⁺. In another example, said isolated OCT-4⁺ cells are additionally CD73⁺, CD105⁺, CD200⁺, CD34⁻, CD38⁻, and CD45⁻. In another example, said cell population is isolated away from placental cells or umbilical cord cells that do not display these markers.

In another embodiment, the CD10⁺, CD34⁻, CD200⁺, CD105⁺ placental stem cells for use in the methods of treatment described herein are additionally HLA-A,B,C⁺, CD45⁻, CD133⁻. In another example, a cell population useful for the methods of treatment described herein is an isolated population of cells comprising placental stem cells, wherein at least about 70%, at least about 80%, at least about 90%, at least about 95% or at least about 99% of cells in said isolated population of cells are isolated HLA-A,B,C⁺, CD45⁻, CD133⁻ and CD34⁻ placental stem cells. In another example, said isolated cell or population of isolated cells is isolated away from cells that are not HLA-A,B,C⁺, CD45⁻, CD133⁻ and CD34⁻ cells. In another example, said isolated placental cells are non-maternal in origin. In another example, said isolated population of cells are substantially free of maternal components; *e.g*., at least about 40%, 45%, 5-0%, 55%, 60%, 65%, 70%, 75%, 90%, 85%, 90%, 95%, 98% or 99% of said cells in said isolated population of cells are non-maternal in origin.

In another embodiment, the CD10⁺, CD34⁻, CD200⁺, CD105⁺ placental stem cells for use in the methods of treatment described herein are additionally CD13⁺, CD33⁺, CD45⁻, CD117⁻ and CD133⁻. In one example, a cell population useful for the methods of treatment described herein is a population of cells comprising placental stem cells, wherein at least about 70%, at least about 80%, at least about 90%, at least about 95% or at least about 99% of cells in said population of cells are isolated CD10⁺, CD13⁺, CD33⁺, CD45⁻, CD117⁻ and CD133⁻ placental stem cells. In one example, said isolated cells or isolated population of cells is isolated away from cells that are not said cells. In another example, said isolated CD10⁺, CD13⁺, CD33⁺, CD45⁻, CD117⁻ and CD133⁻ cells are non-maternal in origin, i.e., have the fetal genotype. In another example, at least about 40%, 45%, 50%, 55%, 60%, 65%, 70%, 75%, 90%, 85%, 90%, 95%, 98% or 99% of said cells in said isolated population of cells are non-maternal in origin. In another example, said isolated cells or isolated population of placental cells are isolated away from cells that do not display these characteristics.

In another embodiment CD10⁺, CD34⁻, CD200⁺, CD105⁺ placental stem cells for use in the methods of treatment described herein are additionally CD33⁻, CD44⁺, CD45⁻, and CD117⁻ cells. In one example, a cell population useful for the methods of treatment disclosed herein is a population of cells comprising, *e.g*., enriched for, isolated placental cells, wherein at least about 70%, at least about 80%, at least about 90%, at least about 95% or at least about 99% of cells in said population of cells are isolated CD10⁻, CD33⁻, CD44⁺, CD45⁻, and CD117⁻ placental stem cells. In one example, said isolated cell or isolated population of cells is isolated away from cells that are not said cells. In another example, said isolated cells are non-maternal in origin. In another example, at least about 40%, 45%, 50%, 55%, 60%, 65%, 70%, 75%, 90%, 85%, 90%, 95%, 98% or 99% of said cells in said cell population are non-maternal in origin. In example, said isolated cell or isolated population of placental cells is isolated away from cells that do not display these markers.

In another embodiment, the CD10⁺, CD34⁻, CD200⁺, CD105⁺ placental stem cells for use in the methods of treatment described herein are additionally CD13⁻, CD33⁻, CD45⁻, and CD117⁻ cells. In one example, a cell population useful for the methods of treatment disclosed herein is an isolated population of cells comprising, *e.g*., enriched for, isolated CD10⁻, CD13⁺, CD33⁻, CD45⁻, and CD117⁻ placental stem cells, wherein at least about 70%, at least about 80%, at least about 90%, at least about 95% or at least about 99% of cells in said population are said CD10⁻, CD13⁻, CD33⁻, CD45⁻, and CD117⁻ cells. In a specific embodiment, said isolated cells or isolated population of cells are isolated away from cells that are not said cells. In another specific embodiment, said isolated cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated cells or isolated population of cells is isolated away from cells that do not display these characteristics.

In another embodiment, the CD10⁺, CD34⁻, CD105⁺, CD200⁺ placental stem cells for use in the methods of treatment described herein are additionally HLA A,B,C⁺, CD45⁻, CD34⁻, CD133⁻ CD13⁺, CD38⁺, CD44⁺, CD90⁺, and/or HLA-G⁺, and/or negative for CD117. In another embodiment, a cell population useful for the methods of treatment disclosed herein is an isolated population of cells comprising, *e.g*., enriched for, isolated placental stem cells, wherein at least about 20%, 25%, 30%, 35%, 40%, 45%, 50%, 55%, 60%, 65%, 70%, 75%, 80%, 85%, 90%, 95%, 98% or about 99% of the cells in said population are placental stem cells that are HLA A,B,C⁻, CD45⁻, CD34⁻, CD133⁻, and that are additionally positive for CD10, CD13, CD38, CD44, CD90, CD105, CD200 and/or HLA-G, and/or negative for CD117. In a specific embodiment, said isolated cells or isolated population of cells are isolated away from cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated cells or isolated population of cells are isolated away from placental cells that do not display these markers.

In another embodiment, placental stem cells for use in the methods of treatment described herein are isolated cells that are CD105⁺, CD34⁻, CD200⁺ and CD10⁺, as determined by antibody binding, and CD117⁻, as determined by both antibody binding and RT-PCR. In another embodiment, the placental stem cells useful in the methods of treatment disclosed herein are isolated cells, *e.g.,* stem cells or multipotent cells, that are CD105⁺, CD34⁻, CD10⁺, CD29⁻, CD54⁺, CD200⁺, HLA-G⁺, HLA class I⁺ and β-2-microglobulin⁺.

In another embodiment, the CD10⁺, CD34⁻, CD200⁺, CD105⁺ placental stem cells useful in the methods of treatment described herein are isolated placental cells or umbilical cord cells, *e.g*., placental stem cells, placental multipotent cells, umbilical cord stem cells or umbilical cord multipotent cells, that are additionally one or more of CD29⁺, CD44⁺, CD45⁻, CD54/ICAM⁺, CD62E⁻, CD62L⁻, CD62P⁻, CD80⁻, CD86⁻, CD103⁻, CD104⁻, CD106/VCAM⁺, CD144/VE-cadherin^{low}, CD184/CXCR4⁻, β2-microglobulin^{low}, MHC-I^{low}, MHC-II⁻, HLA-G^{low}, and/or PDL1^{low}. In a specific embodiment, said isolated cells are at least CD29⁺ and CD54⁺. In another specific embodiment, said isolated cells are at least CD44⁺ and CD106⁺. In another specific embodiment, said isolated cells are at least CD29⁺.

In another embodiment, a cell population useful in the methods of treatment described herein comprises isolated placental stem cells, and at least 50%, 60%, 70%, 80%, 90%, 95%, 98% or 99% of the cells in said cell population are isolated placental stem cells that are CD10⁺, CD34⁻, CD200⁺, CD105⁺, and additionally one or more of CD29⁺, CD44⁺, CD45⁻, CD54/ICAM⁺, CD62-E⁻, CD62-L⁻, CD62-P⁻, CD80⁻, CD86⁻, CD103⁻, CD104⁻, CD106/VCAM⁺, CD144/VE-cadherin^{low}, CD184/CXCR4⁻, β-microglobulin^{low}, HLA-I^{low}, HLA-II⁻, HLA-G^{low}, and/or PDL1^{low} In a more specific embodiment, at least 50%, 60%, 70%, 80%, 90%, 95%, 98% or 99% of placental stem cells in said cell population are CD10⁺, CD34⁻, CD200⁺, CD105⁺, and additionally CD29⁺, CD44⁺, CD45⁻, CD54/ICAM⁺, CD62-E⁻, CD62-L⁻, CD62-P⁻, CD80⁻, CD86⁻, CD103⁻, CD104⁻, CD106/VCAM⁺, CD144/VE-cadherin^{low}, CD184/CXCR4⁻, β2-microglobulin^{low}, MHC-I^{low}, MHC-II⁻, HLA-G^{low}, and PDL1^{low}.

In another embodiment, the CD10⁺, CD34⁻, CD200⁺, and CD105⁺ placental stem cells useful in the methods of treatment described herein are isolated placental cells or umbilical cord cells (*e.g*., stem cells or multipotent cells) that are additionally one or more, or all, of CD29⁺, CD38⁻, CD44⁺, CD45⁻, CD54⁺, CD90⁺, SH2⁺, SH3⁺, SH4⁺, SSEA3⁻, SSEA4⁻, OCT-4⁺, and ABC-p⁺, where ABC-p is a placenta-specific ABC transporter protein (also known as breast cancer resistance protein (BCRP) and as mitoxantrone resistance protein (MXR)), wherein said placental stem cells are obtained by perfusion of a mammalian, *e.g*., human, placenta that has been drained of cord blood and perfused to remove residual blood.

In another embodiment, the CD10⁺, CD34⁻, CD200⁺, and CD105⁺ placental stem cells useful in the methods of treatment described herein are isolated placental cells or umbilical cord cells wherein the expression of at least one cellular marker in said cells is at least two-fold higher than for a mesenchymal stem cell (*e.g*., a bone marrow-derived mesenchymal stem cell) or dermal fibroblast. In another specific embodiment, said isolated cells are non-maternal in origin (*i.e*., have the fetal genotype). In another specific embodiment, said pare contained within a cell population, wherein at least about 40%, 45%, 50%, 55%, 60%, 65%, 70%, 75%, 90%, 85%, 90%, 95%, 98% or 99% of cells in said cell population are placental stem cells that are non-maternal in origin.

Gene profiling confirms that isolated placental stem cells, and isolated populations of placental stem cells, are distinguishable from other cells, *e.g.,* dermal fibroblasts or mesenchymal stem cells, *e.g.,* bone marrow-derived mesenchymal stem cells, on the basis of expression of certain genes. The isolated placental stem cells described herein can be distinguished from, *e.g.,* dermal fibroblasts or bone marrow-derived mesenchymal stem cells on the basis of the expression of one or more genes, the expression of which is significantly higher (*e.g*., statistically significantly higher, or twofold higher) in the placental stem cells in comparison to the dermal fibroblasts or bone marrow-derived mesenchymal stem cells. In particular, placental stem cells can be distinguished from mesenchymal stem cells on the basis of the expression of one or more genes, the expression of which is significantly higher (that is, at least twofold higher) in the placental stem cells than in an equivalent number of dermal fibroblasts or bone marrow-derived mesenchymal stem cells, wherein the one or more genes are ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, HLA-G, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. *See, e.g.,* U.S. Patent Application Publication No. 2007/0275362. In a more specific embodiment, said placental stem cells express said one or more genes (differentially compared to dermal fibroblasts or bone marrow-derived mesenchymal stem cells) when cultured for from about 3 to about 35 population doublings in a medium comprising DMEM-LG (*e.g*., from Gibco); 2% fetal calf serum (*e.g*., from Hyclone Labs.); 1x insulin-transferrin-selenium (ITS); 1x linoleic acid-bovine serum albumin (LA-BSA); 10⁻⁹ M dexamethasone (*e.g*., from Sigma); 10⁻⁴ M ascorbic acid 2-phosphate (*e.g*., from Sigma); epidermal growth factor 10 ng/mL (*e.g*., from R&D Systems); and platelet-derived growth factor (PDGF-BB) 10 ng/mL (*e.g*., from R&D Systems). In a specific embodiment, the placental stem cell-specific gene is CD200.

Specific sequences for these genes can be found in GenBank at accession nos. NM_001615 (ACTG2), BC065545 (ADARB1), (NM_181847 (AMIGO2), AY358590 (ARTS-1), BC074884 (B4GALT6), BC008396 (BCHE), BC020196 (C11orf9), BC031103 (CD200), NM_001845 (COL4A1), NM_001846 (COL4A2), BC052289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (ILIA), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BC023312 (ST3GAL6), BC001201 (ST6GALNAC5), BC126160 or BC065328 (SLC12A8), BC025697 (TCF21), BC096235 (TGFB2), BC005046 (VTN), and BC005001 (ZC3H12A) as of March 2008.

In a more specific embodiment, said placental stem cells express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, HLA-G, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

Expression of the above-referenced genes can be assessed by standard techniques. For example, probes based on the sequence of the gene(s) can be individually selected and constructed by conventional techniques. Expression of the genes can be assessed, *e.g*., on a microarray comprising probes to one or more of the genes, *e.g*., an Affymetrix GENECHIP® Human Genome U133A 2.0 array, or an Affymetrix GENECHIP® Human Genome U133 Plus 2.0 (Santa Clara, California). Expression of these genes can be assessed even if the sequence for a particular GenBank accession number is amended because probes specific for the amended sequence can readily be generated using well-known standard techniques.

The level of expression of these genes can be used to confirm the identity of a population of isolated placental stem cells, to identify an isolated population of cells as comprising at least a plurality of placental stem cells, or the like. Isolated populations of cells comprising placental stem cells, the identity of which is confirmed, can be clonal, *e.g.,* isolated populations of placental stem cells expanded from a single isolated placental stem cell, or a mixed population of stem cells comprising placental stem cells, *e.g.,* a population of cells comprising placental stem cells that are expanded from multiple isolated placental stem cells, or a population of cells comprising isolated placental stem cells, as described herein, and at least one other type of cell.

The level of expression of these genes can be used to select populations of isolated placental cells. For example, a population of cells, *e.g*., clonally-expanded cells, may be selected if the expression of one or more of the genes listed above is significantly higher in a sample from the population of cells than in an equivalent population of mesenchymal stem cells. Such selecting can be of a population from a plurality of isolated placental cell populations, from a plurality of cell populations, the identity of which is not known, *etc.*

Isolated placental cells, *e.g*., placental stem cells can be selected on the basis of the level of expression of one or more such genes as compared to the level of expression of said one or more genes in, *e.g.,* a mesenchymal stem cell control, for example, the level of expression of said one or more genes in an equivalent number of bone marrow-derived mesenchymal stem cells. In one embodiment, the level of expression of said one or more genes in a sample comprising an equivalent number of mesenchymal stem cells is used as a control. In another embodiment, the control, for isolated placental cells tested under certain conditions, is a numeric value representing the level of expression of said one or more genes in mesenchymal stem cells under said conditions.

The isolated placental stem cells described herein display the above characteristics (*e.g*., combinations of cell surface markers and/or gene expression profiles) in primary culture, or during proliferation in medium comprising, *e.g*., DMEM-LG (Gibco), 2% fetal calf serum (FCS) (Hyclone Laboratories), 1x insulin-transferrin-selenium (ITS), 1x lenolenic-acid-bovine-serum-albumin (LA-BSA), 10⁻⁹ M dexamethasone (Sigma), 10⁻⁴M ascorbic acid 2-phosphate (Sigma), epidermal growth factor (EGF)10ng/ml (R&D Systems), platelet derived-growth factor (PDGF-BB) 10ng/ml (R&D Systems), and 100U penicillin/1000U streptomycin.

In another specific embodiment of said isolated placental cells, *e.g*., placental stem cells, or populations of cells comprising the isolated placental cells, said cells or population have been expanded, for example, passaged at least, about, or no more than, 1, 2, 3, 4, 5, 6, 7, 8, 9, 10, 11, 12, 13, 14, 15, 16, 17, 18, 19, or 20 times, or proliferated for at least, about, or no more than, 1, 2, 3, 4, 5, 6, 7, 8, 9, 10, 12, 14, 16, 18, 20, 22, 24, 26, 28, 30, 32, 34, 36, 38 or 40 population doublings. In another specific embodiment of the isolated placental cells, *e.g.,* placental stem cells, or populations of cells comprising isolated placental cells, that are disclosed herein, said isolated placental cells are fetal in origin (that is, have the fetal genotype).

In certain embodiments of isolated placental cells, *e.g*., placental stem cells, said isolated placental cells do not differentiate during culturing in growth medium, *i.e.,* medium formulated to promote proliferation, *e.g*., during proliferation in growth medium. In another specific embodiment, said isolated placental cells, *e.g*., placental stem cells, do not require a feeder layer in order to proliferate. In another specific embodiment, said isolated placental cells do not differentiate in culture in the absence of a feeder layer, solely because of the lack of a feeder cell layer.

In another embodiment, placental stem cells useful in the methods of treatment disclosed herein are isolated placental cells or umbilical cord cells, wherein a plurality of the cells are positive for aldehyde dehydrogenase (ALDH), as assessed by an aldehyde dehydrogenase activity assay. Such assays are known in the art (*see, e.g.,* Bostian and Betts, Biochem. J., 173, 787, (1978)). In a specific embodiment, said ALDH assay uses ALDEFLUOR® (Aldagen, Inc., Ashland, Oregon) as a marker of aldehyde dehydrogenase activity. In a specific embodiment, said plurality is between about 3% and about 25% of cells in said population of cells. In another embodiment, said placental stem cells show at least three-fold, or at least five-fold, higher ALDH activity than a population of bone marrow-derived mesenchymal stem cells having about the same number of cells and cultured under the same conditions.

In certain embodiments of any of the populations of cells comprising placental stem cells, as described herein, the placental stem cells in said populations of cells are substantially free of cells having a maternal genotype; *e.g.,* at least 40%, 45%, 50%, 55%, 60%, 65%, 70%, 75%, 80%, 85%, 90%, 95%, 98% or 99% of the, *e.g*., placental stem cells in said populations have a fetal genotype. In certain other embodiments of any of the populations of cells comprising the placental stem cells described herein, the populations of cells comprising said placental stem cells are substantially free of cells having a maternal genotype; *e.g.,* at least 40%, 45%, 50%, 55%, 60%, 65%, 70%, 75%, 80%, 85%, 90%, 95%, 98% or 99% of the cells (*e.g*., , *e.g.,* placental stem cells) in said population have a fetal genotype.

In a specific embodiment of any of the above isolated , *e.g*., placental stem cells or isolated cell populations comprising the, *e.g.,* placental stem cells, the karyotype of the , *e.g.,* placental stem cells, or at least about 95% or about 99% of the cells in said population, is normal. In another specific embodiment of any of the above placental stem cells or cell populations, the placental stem cells, or , *e.g.,* placental stem cells in the population of cells, are non-maternal in origin.

Isolated placental stem cells, or isolated populations of cells comprising placental stem cells, *e.g*., consisting essentially of placental stem cells, bearing any of the above combinations of markers, can be combined in any ratio. Any two or more of the above isolated cell populations can be combined to form an isolated cell population. For example, an isolated population of cells can comprise a first population of cells defined by one of the marker combinations described above, and a second population of cells defined by another of the marker combinations described above, wherein said first and second populations are combined in a ratio, *e.g.,* of about 1:99, 2:98, 3:97, 4:96, 5:95, 10:90, 20:80, 30:70, 40:60, 50:50, 60:40, 70:30, 80:20, 90:10, 95:5, 96:4, 97:3, 98:2, or about 99:1. In like fashion, any three, four, five or more of the cells or isolated cell populations can be combined.

Isolated placental stem cells useful in the methods of treatment disclosed herein can be obtained, *e.g*., by disruption of placental tissue or umbilical cord tissue, with or without enzymatic digestion (*see* Section 5.5.3), or by perfusion (*see* Section 5.5.4). For example, populations of isolated placental stem cells can be produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises isolated placental cells; and isolating a plurality of said isolated placental cells from said population of cells. In a specific embodiment, the perfusion solution is passed through both the umbilical vein and umbilical arteries and collected after it exudes from the placenta. In another specific embodiment, the perfusion solution is passed through the umbilical vein and collected from the umbilical arteries, or passed through the umbilical arteries and collected from the umbilical vein

In various embodiments, the isolated placental stem cells, contained within a population of cells obtained from perfusion of a placenta, are at least 50%, 60%, 70%, 80%, 90%, 95%, 99% or at least 99.5% of said population of placental cells. In another specific embodiment, the isolated placental stem cells collected by perfusion comprise fetal and maternal cells. In another specific embodiment, the isolated placental cells collected by perfusion are at least 50%, 60%, 70%, 80%, 90%, 95%, 99% or at least 99.5% fetal cells.

Disclosed herein is also a composition comprising a population of the isolated placental cells, *e.g*., placental stem cells, as described herein, collected by perfusion, wherein said composition comprises at least a portion of the perfusion solution used to collect the isolated placental cells.

In certain embodiments, isolated populations of the placental stem cells described herein can be produced by digesting placental tissue or umbilical cord tissue with a tissue-disrupting enzyme to obtain a population of cells comprising the placental stem cells, and isolating, or substantially isolating, a plurality of the placental stem cells from the remainder of said placental or umbilical cord cells. The whole, or any part of, the placenta or umbilical cord can be digested to obtain the placental stem cells described herein. In specific embodiments, for example, said tissue can be a whole placenta, an amniotic membrane, chorion, a combination of amnion and chorion, umbilical cord, or a combination of any of the foregoing. In other specific embodiment, the tissue-disrupting enzyme is trypsin, collagenase, dispase or the like. In various embodiments, the isolated placental stem cells, contained within a population of cells obtained from digesting a placenta, are at least 50%, 60%, 70%, 80%, 90%, 95%, 99% or at least 99.5% of said population of placental cells.

The isolated populations of placental stem cells, or isolated populations of cells comprising placental stem cells, described above, can comprise about, at least, or no more than, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more of the isolated placental cells. Populations of isolated placental cells useful in the methods of treatment described herein comprise at least 50%, 55%, 60%, 65%, 70%, 75%, 80%, 85%, 90%, 95%, 98%, or 99% viable isolated placental cells, as determined by, *e.g.,* trypan blue exclusion

### 4.2.3 Growth in Culture

The growth of the placental cells, *e.g*., placental stem cells, described herein, as for any mammalian cell, depends in part upon the particular medium selected for growth. Under optimum conditions, placental stem cells typically double in number in 1-5 days. During culture, the placental stem cells provided herein adhere to a substrate in culture, *e.g*. the surface of a tissue culture container (*e.g*., tissue culture dish plastic, fibronectin-coated plastic, and the like) and form a monolayer.

Populations of isolated placental cells that comprise the placental cells, *e.g*., placental stem cells, when cultured under appropriate conditions, may form embryoid-like bodies, that is, three-dimensional clusters of cells grow atop the adherent placental stem cells layer. Cells within the embryoid-like bodies express markers associated with very early stem cells, *e.g.,* OCT-4, Nanog, SSEA3 and SSEA4. Cells within the embryoid-like bodies are typically not adherent to the culture substrate, as are the placental stem cells described herein, but remain attached to the adherent cells during culture. Embryoid-like body cells are dependent upon the placental stem cells for viability, as embryoid-like bodies do not form in the absence of the placental cells, *e.g*., placental stem cells. The adherent placental stem cells thus facilitate the growth of one or more embryoid-like bodies in a population of placental cells that comprise the placental stem cells. Without wishing to be bound by theory, the cells of the embryoid-like bodies are thought to grow on the adherent placental stem cells much as embryonic stem cells grow on a feeder layer of cells. Mesenchymal stem cells, *e.g.,* bone marrow-derived mesenchymal stem cells, do not develop embryoid-like bodies in culture.

### 4.3 METHODS OF OBTAINING PLACENTAL STEM CELLS

### 4.3.1 Stem Cell Collection Composition

Placental stem cells can be collected and isolated according to the methods described herein. Generally, stem cells are obtained from a mammalian placenta or umbilical cord using a physiologically-acceptable solution, *e.g*., a stem cell collection composition. A stem cell collection composition is described in detail in U.S. Patent Application Publication No. 2007/0190042.

The stem cell collection composition can comprise any physiologically-acceptable solution suitable for the collection and/or culture of stem cells, for example, a saline solution (*e.g*., phosphate-buffered saline, Kreb's solution, modified Kreb's solution, Eagle's solution, 0.9% NaCl. *etc*.), a culture medium (*e.g.,* DMEM, HDMEM, *etc*.), and the like.

The stem cell collection composition can comprise one or more components that tend to preserve placental stem cells, that is, prevent placental stem cells from dying, or delay the death of the placental stem cells, reduce the number of placental stem cells in a population of cells that die, or the like, from the time of collection to the time of culturing. Such components can be, *e.g.,* an apoptosis inhibitor (*e.g*., a caspase inhibitor or JNK inhibitor); a vasodilator (*e.g*., magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc*.); a necrosis inhibitor (*e.g*., 2-(1H-Indol-3-yl)-3-pentylamino-maleimide, pyrrolidine dithiocarbamate, or clonazepam); a TNF-α inhibitor; and/or an oxygen-carrying perfluorocarbon (*e.g.,* perfluorooctyl bromide, perfluorodecyl bromide, *etc*.).

The stem cell collection composition can comprise one or more tissue-degrading enzymes, *e.g*., a metalloprotease, a serine protease, a neutral protease, an RNase, or a DNase, or the like. Such enzymes include, but are not limited to, collagenases (*e.g*., collagenase I, II, III or IV, a collagenase from *Clostridium histolyticum, etc*.); dispase, thermolysin, elastase, trypsin, LIBERASE™, hyaluronidase, and the like.

The stem cell collection composition can comprise a bacteriocidally or bacteriostatically effective amount of an antibiotic. In certain non-limiting embodiments, the antibiotic is a macrolide (*e.g.,* tobramycin), a cephalosporin (*e.g.,* cephalexin, cephradine, cefuroxime, cefprozil, cefaclor, cefixime or cefadroxil), a clarithromycin, an erythromycin, a penicillin (*e.g.,* penicillin V) or a quinolone (*e.g.,* ofloxacin, ciprofloxacin or norfloxacin), a tetracycline, a streptomycin, *etc.* In a particular embodiment, the antibiotic is active against Gram(+) and/or Gram(-) bacteria, *e.g., Pseudomonas aeruginosa, Staphylococcus aureus,* and the like.

The stem cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (*e.g*., a synthetic or naturally occurring colloid, a polysaccharide such as dextran or a polyethylene glycol present at about 25 g/l to about 100 g/l, or about 40 g/l to about 60 g/l); an antioxidant (*e.g*., butylated hydroxyanisole, butylated hydroxytoluene, glutathione, vitamin C or vitamin E present at about 25 µM to about 100 µM); a reducing agent (*e.g*., N-acetylcysteine present at about 0.1 mM to about 5 mM); an agent that prevents calcium entry into cells (*e.g*., verapamil present at about 2 µM to about 25 µM); nitroglycerin (*e.g*., about 0.05 g/L to about 0.2 g/L); an anticoagulant present in an amount sufficient to help prevent clotting of residual blood (*e.g.,* heparin or hirudin present at a concentration of about 1000 units/l to about 100,000 units/l); or an amiloride containing compound (*e.g*., amiloride, ethyl isopropyl amiloride, hexamethylene amiloride, dimethyl amiloride or isobutyl amiloride present at about 1.0 µM to about 5 µM).

### 4.3.2 Collection and Handling of Placenta and Umbilical Cord

Generally, a human placenta, with umbilical cord, is recovered shortly after its expulsion after birth. In a preferred embodiment, the placenta is recovered from a patient after informed consent and after a complete medical history of the patient is taken and is associated with the placenta. Preferably, the medical history continues after delivery. Such a medical history can be used to coordinate subsequent use of the placenta/umbilical cord or the stem cells harvested therefrom. For example, placental cells, *e.g*., placental stem cells, can be used, in light of the medical history, for personalized medicine for the infant associated with the placenta and umbilical cord, or for parents, siblings or other relatives of the infant.

Prior to recovery of placental stem cells, the umbilical cord blood and placental blood are removed. In certain embodiments, after delivery, the cord blood and placental blood is recovered. The placenta can be subjected to a conventional cord blood recovery process. Typically a needle or cannula is used, with the aid of gravity, to exsanguinate the placenta (*see, e.g.,* Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e.g*., LifeBank Inc., Cedar Knolls, N.J., ViaCord, Cord Blood Registry and Cryocell. In certain embodiments, the placenta is gravity drained without further manipulation so as to minimize tissue disruption during cord blood recovery.

Typically, a placenta is transported from the delivery or birthing room to another location, *e.g.,* a laboratory, for recovery of cord blood and collection of stem cells by, *e.g.,* perfusion or tissue dissociation. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in pending United States Patent Application No. 2006/0060494, filed September 19, 2005, or in U.S. Patent No. 7,147,626. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In certain other embodiments, the umbilical cord is clamped distally, *e.g.,* at or near the end of the umbilical cord away from the placenta. In other embodiments, the umbilical cord is clamped after cord blood recovery but prior to further processing of the placenta.

The placenta and umbilical cord, prior to stem cell collection, can be stored under sterile conditions and at either room temperature or at a temperature of 5 to 25°C (centigrade). The placenta and umbilical cord may be stored for a period of longer than forty eight hours, and preferably for a period of four to twenty-four hours prior to perfusing the placenta to remove any residual cord blood. The placenta and umbilical cord are preferably stored in an anticoagulant solution at a temperature of 5 to 25°C (centigrade). Suitable anticoagulant solutions are well known in the art. For example, a solution of heparin or warfarin sodium can be used. In a preferred embodiment, the anticoagulant solution comprises a solution of heparin (*e.g*., 1% w/w in 1:1000 solution). The exsanguinated placenta and umbilical cord are preferably stored for no more than 36 hours before cells, *e.g.,* placental stem cells, are collected.

The mammalian placenta or a part thereof, or umbilical cord, once collected and prepared generally as above, can be treated in any art-known manner, *e.g*., can be perfused or disrupted, *e.g*., digested with one or more tissue-disrupting enzymes, to obtain stem cells.

### 4.3.3 Physical Disruption and Enzymatic Digestion of Tissue

In one embodiment, stem cells are collected from a mammalian placenta or umbilical cord by physical disruption, *e.g.,* enzymatic digestion, *e.g.,* using the stem cell collection composition described in Section 4.3.1, above. For example, the placenta, or a portion thereof, or umbilical cord may be, *e.g.,* crushed, sheared, minced, diced, chopped, macerated or the like, while in contact with, *e.g.,* a buffer, medium or a stem cell collection composition, and the tissue subsequently digested with one or more enzymes. The placenta, or a portion thereof, or umbilical cord may also be physically disrupted and digested with one or more enzymes, and the resulting material then immersed in, or mixed into, a buffer, medium or a stem cell collection composition. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, *e.g*., trypan blue exclusion.

The placenta or umbilical cord can be dissected into components prior to physical disruption and/or enzymatic digestion and stem cell recovery. For example, placental stem cells can be obtained from the amniotic membrane, chorion, placental cotyledons, or any combination thereof, or from a portion of an umbilical cord. In one embodiment, placental stem cells are obtained from placental tissue comprising amnion and chorion, amnion-chorion, or umbilical cord. Typically, placental stem cells can be obtained by disruption of a small block of placental tissue or umbilical cord tissue, *e.g.,* a block of placental tissue or umbilical cord tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume.

A preferred stem cell collection composition comprises one or more tissue-disruptive enzyme(s). Enzymatic digestion preferably uses a combination of enzymes, *e.g*., a combination of trypsin, collagenase, and/or dispase, optionally including hyaluronidase, or a combination of LIBERASE (Boehringer Mannheim Corp., Indianapolis, Ind.) and hyaluronidase. Other enzymes that can be used to disrupt placenta tissue include papain, deoxyribonucleases, serine proteases, such as trypsin, chymotrypsin, or elastase. Serine proteases may be inhibited by alpha 2 microglobulin in serum and therefore the medium used for digestion is usually serum-free. EDTA and DNase are commonly used in enzyme digestion procedures to increase the efficiency of cell recovery. The digestate is preferably diluted so as to avoid trapping stem cells within the viscous digest.

Any combination of tissue digestion enzymes can be used. Typical concentrations for tissue digestion enzymes include, *e.g.,* 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to liberate placental stem cells. For example, in one embodiment, a placenta, or part thereof, or umbilical cord, is digested first with an appropriate amount of collagenase I at 2 mg/ml for 30 minutes, followed by digestion with trypsin, 0.25%, for 10 minutes, at 37°C. Serine proteases are preferably used consecutively following use of other enzymes.

In another embodiment, the tissue can further be disrupted by the addition of a chelator, *e.g*., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

It will be appreciated that where an entire placenta, or portion of a placenta comprising both fetal and maternal cells (for example, where the portion of the placenta comprises the chorion or cotyledons), the placental stem cells collected will comprise a mix of placental stem cells derived from both fetal and maternal sources. Where a portion of the placenta that comprises no, or a negligible number of, maternal cells (for example, amnion), the placental stem cells collected will comprise almost exclusively fetal placental stem cells.

### 4.3.4 Placental Perfusion

Placental stem cells can also be obtained by perfusion of a mammalian placenta. Methods of perfusing mammalian placentae to obtain stem cells are disclosed, *e.g.,* in Hariri, U.S. Patent Nos. 7,045,148 and 7,468,276, and in U.S. Patent Application Publication No. 2007/0190042.

Placental stem cells can be collected by perfusion, *e.g*., through the placental vasculature, using, *e.g.,* a stem cell collection composition as a perfusion solution. In one embodiment, a mammalian placenta is perfused by passage of perfusion solution through either or both of the umbilical artery and umbilical vein. The flow of perfusion solution through the placenta may be accomplished using, *e.g*., gravity flow into the placenta. Preferably, the perfusion solution is forced through the placenta using a pump, *e.g.,* a peristaltic pump. The umbilical vein can be, *e.g.,* cannulated with a cannula, *e.g.,* a TEFLON® or plastic cannula, that is connected to a sterile connection apparatus, such as sterile tubing. The sterile connection apparatus is connected to a perfusion manifold. In certain embodiments, the umbilical cord vessels are cannulated proximal to the placenta, *e.g*., within about 2-3 centimeters of the placenta; in other embodiments, the umbilical cord vessels are cannulated distal to the placenta, *e.g*., within about 2-3 centimeters of the end of the umbilical cord furthest from the placenta.

In preparation for perfusion, the placenta is preferably oriented (*e.g*., suspended) in such a manner that the umbilical artery and umbilical vein are located at the highest point of the placenta. The placenta can be perfused by passage of a perfusion fluid, *e.g.,* the stem cell collection composition provided herein, through the placental vasculature, or through the placental vasculature and surrounding tissue. In one embodiment, the umbilical artery and the umbilical vein are connected simultaneously to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. In another embodiment, the perfusion solution is passed through the umbilical veins and collected from the umbilical artery, or is passed through the umbilical artery and collected from the umbilical veins.

In one embodiment, the proximal umbilical cord is clamped during perfusion, and more preferably, is clamped within 4-5 cm (centimeter) of the cord's insertion into the placental disc.

The first collection of perfusion fluid from a mammalian placenta during the exsanguination process is generally colored with residual red blood cells of the cord blood and/or placental blood. The perfusion fluid becomes more colorless as perfusion proceeds and the residual cord blood cells are washed out of the placenta. Generally from 30 to 100 ml (milliliter) of perfusion fluid is adequate to initially exsanguinate the placenta, but more or less perfusion fluid may be used depending on the observed results.

The volume of perfusion liquid used to collect placental stem cells, may vary depending upon the number of stem cells to be collected, the size of the placenta, the number of collections to be made from a single placenta, *etc.* In various embodiments, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with the stem cell collection composition, or a standard perfusion solution (*e.g*., a normal saline solution such as phosphate buffered saline ("PBS")) with or without an anticoagulant (*e.g*., heparin, warfarin sodium, coumarin, bishydroxycoumarin), and/or with or without an antimicrobial agent (*e.g.,* β-mercaptoethanol (0.1 mM); antibiotics such as streptomycin (*e.g.,* at 40-100 µg/ml), penicillin (*e.g*., at 40U/ml), amphotericin B (*e.g.,* at 0.5 µg/ml). In one embodiment, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), *e.g*., 1, 2, 3, 4, 5, 6, 7, 8, 9, 10, 11, 12, 13, 14, 15, 16, 17, 18, 19, 20, 21, 22, 23, 24 or more hours, and perfused a second time with, *e.g.,* 700-800 mL perfusion fluid. The placenta can be perfused 1, 2, 3, 4, 5 or more times, for example, once every 1, 2, 3, 4, 5 or 6 hours. In a preferred embodiment, perfusion of the placenta and collection of perfusion solution, *e.g*., stem cell collection composition, is repeated until the number of recovered nucleated cells falls below 100 cells/ml. The perfusates at different time points can be further processed individually to recover time-dependent populations of cells, *e.g.,* stem cells. Perfusates from different time points can also be pooled.

Without wishing to be bound by any theory, after exsanguination and a sufficient time of perfusion of the placenta, stem cells are believed to migrate into the exsanguinated and perfused microcirculation of the placenta and umbilical cord where they are collected, preferably by washing into a collecting vessel by perfusion. Perfusing the isolated placenta not only serves to remove residual cord blood but also provide the placenta with the appropriate nutrients, including oxygen. The placenta may be cultivated and perfused with a similar solution which was used to remove the residual cord blood cells, preferably, without the addition of anticoagulant agents.

Perfusion as described herein results in the collection of significantly more stem cells than the number obtainable from a mammalian placenta not perfused with said solution, and not otherwise treated to obtain stem cells (*e.g.,* by tissue disruption, *e.g.,* enzymatic digestion). In this context, "significantly more" means at least 10% more. Perfusion yields significantly more stem cells than, *e.g.,* the number of stem cells obtainable from culture medium in which a placenta, or portion thereof, has been cultured.

Stem cells can be isolated from placenta by perfusion with a solution comprising one or more proteases or other tissue-disruptive enzymes. In a specific embodiment, a placenta or portion thereof (*e.g*., amniotic membrane, amnion and chorion, placental lobule or cotyledon, or combination of any of the foregoing) is brought to 25-37°C, and is incubated with one or more tissue-disruptive enzymes in 200 mL of a culture medium for 30 minutes. Cells from the perfusate are collected, brought to 4°C, and washed with a cold inhibitor mix comprising 5 mM EDTA, 2 mM dithiothreitol and 2 mM beta-mercaptoethanol. The stem cells are washed after several minutes with a cold (*e.g.,* 4°C) stem cell collection composition described elsewhere herein.

It will be appreciated that perfusion using the pan method, that is, whereby perfusate is collected after it has exuded from the maternal side of the placenta, results in a mix of fetal and maternal cells. As a result, the cells collected by this method comprise a mixed population of placental stem cells of both fetal and maternal origin. In contrast, perfusion solely through the placental vasculature, whereby perfusion fluid is passed through one or two placental vessels and is collected solely through the remaining vessel(s), results in the collection of a population of placental stem cells almost exclusively of fetal origin.

### 4.3.5 Isolation, Sorting, and Characterization of Placental Stem Cells

Stem cells from mammalian placenta or umbilical cord, whether obtained by perfusion or enyzmatic digestion, can initially be purified from (*i.e*., be isolated from) other cells by Ficoll gradient centrifugation. Such centrifugation can follow any standard protocol for centrifugation speed, *etc.* In one embodiment, for example, cells collected from the placenta or umbilical cord are recovered from perfusate by centrifugation at 5000 x g for 15 minutes at room temperature, which separates cells from, e.g., contaminating debris and platelets. In another embodiment, placental perfusate is concentrated to about 200 ml, gently layered over Ficoll, and centrifuged at about 1100 x g for 20 minutes at 22°C, and the low-density interface layer of cells is collected for further processing.

Cell pellets can be resuspended in fresh stem cell collection composition, or a medium suitable for stem cell maintenance, *e.g*., IMDM serum-free medium containing 2U/ml heparin and 2mM EDTA (GibcoBRL, NY). The total mononuclear cell fraction can be isolated, *e.g*., using Lymphoprep (Nycomed Pharma, Oslo, Norway) according to the manufacturer's recommended procedure.

As used herein, "isolating" placental stem cells, means to remove at least 20%, 30%, 40%, 50%, 60%, 70%, 80%, 90%, 95% or 99% of the cells with which the stem cells are normally associated in the intact mammalian placenta or umbilical cord. A stem cell from an organ is "isolated" when it is present in a population of cells that comprises fewer than 50% of the cells with which the stem cell is normally associated in the intact organ.

Placental stem cells obtained by perfusion or digestion can, for example, be further, or initially, isolated by differential trypsinization using, *e.g.,* a solution of 0.05% trypsin with 0.2% EDTA (Sigma, St. Louis MO). Differential trypsinization is possible because placental stem cells and umbilical cord stem cells typically detach from plastic surfaces within about five minutes, whereas other adherent populations typically require more than 20-30 minutes incubation. The detached cells can be harvested following trypsinization and trypsin neutralization, using, *e.g*., Trypsin Neutralizing Solution (TNS, Cambrex). In one embodiment of isolation of adherent cells, aliquots of, for example, about 5-10 x 10⁶ cells are placed in each of several T-75 flasks, preferably fibronectin-coated T75 flasks. In such an embodiment, the cells can be cultured with commercially available Mesenchymal Stem Cell Growth Medium (MSCGM) (Cambrex), and placed in a tissue culture incubator (37°C, 5% CO₂). After 10 to 15 days, non-adherent cells are removed from the flasks by washing with PBS. The PBS is then replaced by MSCGM. Flasks are preferably examined daily for the presence of various adherent cell types and in particular, for identification and expansion of clusters of fibroblastoid cells.

The number and type of cells collected from a mammalian placenta can be monitored, for example, by measuring changes in morphology and cell surface markers using standard cell detection techniques such as flow cytometry, cell sorting, immunocytochemistry (*e.g*., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using antibodies to CD34, one can determine, using the techniques above, whether a cell comprises a detectable amount of CD34; if so, the cell is CD34⁺. Likewise, if a cell produces enough OCT-4 RNA to be detectable by RT-PCR, or significantly more OCT-4 RNA than an adult cell, the cell is OCT-4⁺ Antibodies to cell surface markers (*e.g*., CD markers such as CD34) and the sequence of stem cell-specific genes, such as OCT-4, are well-known in the art.

Placental stem cells, *e.g.,* cells that have been isolated by Ficoll separation, differential adherence, or a combination of both, may be sorted using a fluorescence activated cell sorter (FACS). Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In one sorting scheme, stem cells from placenta or umbilical cord are sorted on the basis of expression of the markers CD34, CD38, CD44, CD45, CD73, CD105, and/or HLA-G; *i.e.,* the absence of CD34, CD38 and CD45, and the presence of CD44, CD73, CD105 and/or HLA-G. This can be accomplished in connection with procedures to select stem cells on the basis of their adherence properties in culture. For example, an adherence selection stem can be accomplished before or after sorting on the basis of marker expression. In one embodiment, for example, cells are sorted first on the basis of their expression of CD34; CD34⁻ cells are retained, and cells that are, *e.g*., CD200⁺HLA-G⁺ are separated from all other CD34⁻ cells. In another embodiment, cells from placenta are based on their expression of markers CD200 and/or HLA-G; for example, cells displaying either of these markers are isolated for further use. Cells that express, *e.g*., CD200 and/or HLA-G can, in a specific embodiment, be further sorted based on their expression of CD73 and/or CD105, or epitopes recognized by antibodies SH2, SH3 or SH4, or lack of expression of CD34, CD38 or CD45. For example, in one embodiment, placental cells and/or umbilical cord cells are sorted by expression, or lack thereof, of CD200, HLA-G, CD73, CD105, CD34, CD38 and CD45, and cells that are CD200⁺, HLA-G⁺, CD73⁺, CD105⁺, CD34⁻, CD38⁻ and CD45⁻ are isolated from other cells for further use.

In another embodiment, magnetic beads can be used to separate cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then be isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Placental stem cells can also be characterized and/or sorted based on cell morphology and growth characteristics. For example, placental stem cells can be characterized as having, and/or selected on the basis of, *e.g.,* a fibroblastoid appearance in culture. Placental stem cells can also be characterized as having, and/or be selected, on the basis of their ability to form embryoid-like bodies. In one embodiment, for example, placental cells or umbilical cord cells that are fibroblastoid in shape, express CD73 and CD105, and produce one or more embryoid-like bodies in culture are isolated from other placental cells or umbilical cord cells. In another embodiment, OCT-4⁺ placental cells that produce one or more embryoid-like bodies in culture are isolated from other placental cells.

Placental stem cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay, MTT cell proliferation assay (to assess proliferation). Longevity may be determined by methods well known in the art, such as by determining the maximum number of population doubling in an extended culture.

Placental stem cells can also be separated from other cells using other techniques known in the art, *e.g*., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 4.4 CULTURE OF PLACENTAL STEM CELLS

### 4.4.1 Culture Media

Isolated placental stem cells, or populations of placental stem cells, or placental tissue or umbilical cord tissue from which stem cells grow out, can be used to initiate, or seed, cell cultures. Cells are generally transferred to sterile tissue culture vessels either uncoated or coated with extracellular matrix or ligands such as laminin, collagen (e.g., native or denatured), gelatin, fibronectin, ornithine, vitronectin, and extracellular membrane protein (*e.g*., MATRIGEL (BD Discovery Labware, Bedford, Mass.)).

Placental stem cells can be cultured in any medium, and under any conditions, recognized in the art as acceptable for the culture of stem cells. Preferably, the culture medium comprises serum. Placental stem cells can be cultured in, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising 10% fetal bovine serum (FBS); DMEM-HG comprising 15% FBS; IMDM (Iscove's modified Dulbecco's medium) comprising 10% FBS, 10% horse serum, and hydrocortisone; M199 comprising 10% FBS, EGF, and heparin; α-MEM (minimal essential medium) comprising 10% FBS, GlutaMAX™ and gentamicin; DMEM comprising 10% FBS, GlutaMAX™ and gentamicin, *etc.* A preferred medium is DMEM-LG/MCDB-201 comprising 2% FBS, ITS, LA+BSA, dextrose, L-ascorbic acid, PDGF, EGF, and penicillin/streptomycin.

Other media in that can be used to culture placental stem cells include DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM), Liebovitz's L-15 medium, MCDB, DMIEM/F12, RPMI 1640, advanced DMEM (Gibco), DMEM/MCDB201 (Sigma), and CELL-GRO FREE.

The culture medium can be supplemented with one or more components including, for example, serum (*e.g*., fetal bovine serum (FBS), preferably about 2-15% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

### 4.4.2 Expansion and Proliferation of Placental Stem Cells

Once a placental stem cell, or isolated population of stem cells (*e.g*., a stem cell or population of stem cells separated from at least 50% of the placental cells with which the stem cell or population of stem cells is normally associated *in vivo*) is isolated, the stem cell or population of stem cells can be proliferated and expanded *in vitro.* For example, a population of placental stem cells can be cultured in tissue culture containers, *e.g*., dishes, flasks, multiwell plates, or the like, for a sufficient time for the stem cells to proliferate to 70-90% confluence, that is, until the stem cells and their progeny occupy 70-90% of the culturing surface area of the tissue culture container.

Placental stem cells can be seeded in culture vessels at a density that allows cell growth. For example, the cells may be seeded at low density (*e.g*., about 1,000 to about 5,000 cells/cm²) to high density (*e.g*., about 50,000 or more cells/cm²). In a preferred embodiment, the cells are cultured at about 0 to about 5 percent by volume CO₂ in air. In some preferred embodiments, the cells are cultured at about 2 to about 25 percent O₂ in air, preferably about 5 to about 20 percent O₂ in air. The cells preferably are cultured at about 25°C to about 40°C, preferably 37°C. The cells are preferably cultured in an incubator. The culture medium can be static or agitated, for example, using a bioreactor. Placental stem cells preferably are grown under low oxidative stress (*e.g*., with addition of glutathione, ascorbic acid, catalase, tocopherol, N-acetylcysteine, or the like).

Once 70%-90% confluence is obtained, the cells may be passaged. For example, the cells can be enzymatically treated, *e.g*., trypsinized, using techniques well-known in the art, to separate them from the tissue culture surface. After removing the cells by pipetting and counting the cells, about 10,000-100,000 stem cells per square centimeter, preferably about 50,000 stem cells per square centimeter, are passaged to a new culture container containing fresh culture medium. Typically, the new medium is the same type of medium from which the stem cells were removed. Provided herein are populations of placental stem cells that have been passaged at least 1, 2, 3, 4, 5, 6, 7, 8, 9, 10, 12, 14, 16, 18, or 20 times, or more, and combinations of the same.

### 4.4.3 Placental Stem Cell Populations

The present invention provides placental stem cells or populations thereof, wherein the cells are CD10+, CD34-, CD200+, and CD105+, for use in the methods of treatment provided herein. Placental stem cell populations can be isolated directly from one or more placentas; that is, the placental stem cell population can be a population of placental cells or umbilical cord cells, comprising placental stem cells or umbilical cord cells, obtained from, or contained within, perfusate, or obtained from, or contained within, digestate (that is, the collection of cells obtained by enzymatic digestion of a placenta or part thereof, or from an umbilical cord). Isolated placental stem cells can also be cultured and expanded to produce placental stem cell populations. Populations of placental cells or umbilical cord cells comprising placental stem cells can also be cultured and expanded to produce placental stem cell populations.

In various embodiments, at least 10%, 20%, 30%, 40%, 50%, 60%, 70%, 80%, 90%, 95%, or 99% of the cells in an isolated placental cell population or umbilical cord cell population are placental stem cells. That is, a placental cell population or umbilical cord cell population comprising placental stem cells can comprise, *e.g*., as much as 1%, 5%, 10%, 20%, 30%, 40%, 50%, 60%, 70%, 80%, 90% non-stem cells.

Provided herein are methods of producing isolated placental stem cell populations by, e.g., selecting placental stem cells or umbilical cord stem cells, whether derived from enzymatic digestion or perfusion, that express particular markers and/or particular culture or morphological characteristics. In one embodiment, for example, a cell population can be produced by a method comprising selecting placental or umbilical cord cells that (a) adhere to a tissue culture plastic substrate, (b) are CD10⁺, CD34⁻, CD200⁺ and CD105⁺; and isolating said cells from other cells to form a cell population. In another example, a cell population can be produced by a method comprising selecting placental or umbilical cord cells that (a) adhere to a substrate, and (b) express CD200 and HLA-G; and isolating said cells from other cells to form a cell population. In another embodiment, the method of producing a cell population comprises selecting placental cells or umbilical cord cells that (a) adhere to a substrate, and (b) express CD73, CD105, and CD200; and isolating said cells from other cells to form a cell population. In another embodiment, the method of producing a cell population comprises selecting placental cells or umbilical cord cells that (a) adhere to a substrate and (b) express CD200 and OCT-4; and isolating said cells from other cells to form a cell population. In another embodiment, the method of producing a cell population comprises selecting placental cells or umbilical cord cells that (a) adhere to a substrate, (b) express CD73 and CD105, and (c) facilitate the formation of one or more embryoid-like bodies in a population of placental cells comprising said stem cell when said population is cultured under conditions that allow for the formation of an embryoid-like body; and isolating said cells from other cells to form a cell population. In another embodiment, the method of producing a cell population comprises selecting placental cells or umbilical cord cells that (a) adhere to a substrate, and (b) express CD73, CD105 and HLA-G; and isolating said cells from other cells to form a cell population. In another embodiment, the method of producing a cell population comprises selecting placental cells that (a) adhere to a substrate, (b) express OCT-4, and (c) facilitate the formation of one or more embryoid-like bodies in a population of placental cells comprising said stem cell when said population is cultured under conditions that allow for the formation of an embryoid-like body; and isolating said cells from other cells to form a cell population. In any of the above embodiments, the method can additionally comprise selecting placental cells or umbilical cord cells that express ABC-p (a placenta-specific ABC transporter protein; *see, e.g.,* Allikmets et al., Cancer Res. 58(23):5337-9 (1998)).

In the above embodiments, the substrate can be any surface on which culture and/or selection of cells, *e.g*., placental stem cells, can be accomplished. Typically, the substrate is plastic, *e.g*., tissue culture dish or multiwell plate plastic. Tissue culture plastic can be coated with a biomolecule, *e.g*., laminin or fibronectin.

Cells, *e.g.,* placental stem cells, can be selected for a placental stem cell population by any means known in the art of cell selection. For example, cells can be selected using an antibody or antibodies to one or more cell surface markers, for example, in flow cytometry or FACS. Selection can be accomplished using antibodies in conjunction with magnetic beads. Antibodies that are specific for certain stem cell-related markers are known in the art. For example, antibodies to OCT-4 (Abcam, Cambridge, MA), CD200 (Abcam), HLA-G (Abcam), CD73 (BD Biosciences Pharmingen, San Diego, CA), CD105 (Abcam; BioDesign International, Saco, ME), *etc.* Antibodies to other markers are also available commercially, *e.g.,* CD34, CD38 and CD45 are available from, *e.g.,* StemCell Technologies or BioDesign International.

Isolated placental stem cell populations can be combined with one or more populations of non-stem cells or non-placental cells. For example, an isolated population of placental stem cells can be combined with blood (*e.g*., placental blood or umbilical cord blood), blood-derived stem cells (*e.g*., stem cells derived from placental blood or umbilical cord blood), populations of blood-derived nucleated cells, bone marrow-derived mesenchymal cells, bone-derived stem cell populations, crude bone marrow, adult (somatic) stem cells, populations of stem cells contained within tissue, cultured stem cells, populations of fully-differentiated cells (*e.g*., chondrocytes, fibroblasts, amniotic cells, osteoblasts, muscle cells, cardiac cells, *etc*.) and the like. Cells in an isolated placental cell population can be combined with cells of another type in ratios of about 100,000,000:1, 50,000,000:1, 20,000,000:1, 10,000,000:1, 5,000,000:1, 2,000,000:1, 1,000,000:1, 500,000:1, 200,000:1, 100,000:1, 50,000:1, 20,000:1, 10,000:1, 5,000:1, 2,000:1, 1,000:1, 500:1, 200:1, 100:1, 50:1, 20:1, 10:1, 5:1, 2:1, 1:1; 1:2; 1:5; 1:10; 1:100; 1:200; 1:500; 1:1,000; 1:2,000; 1:5,000; 1:10,000; 1:20,000; 1:50,000; 1:100,000; 1:500,000; 1:1,000,000; 1:2,000,000; 1:5,000,000; 1:10,000,000; 1:20,000,000; 1:50,000,000; or about 1:100,000,000, comparing numbers of total nucleated cells in each population. Cells in an isolated placental cell population can be combined with cells of a plurality of cell types, as well.

In one embodiment, an isolated population of placental stem cells is combined with hematopoietic stem cells. Such hematopoietic stem cells can be, for example, contained within unprocessed placental, umbilical cord blood or peripheral blood; in total nucleated cells from placental blood, umbilical cord blood or peripheral blood; in an isolated population of CD34⁺ cells from blood, *e.g*., umbilical cord blood or peripheral blood; in unprocessed bone marrow; in total nucleated cells from bone marrow; in an isolated population of CD34⁺ cells from bone marrow, or the like.

### 4.5 PRESERVATION OF PLACENTAL STEM CELLS

Placental cells, *e.g*., placental stem cells, can be preserved, that is, placed under conditions that allow for long-term storage, or conditions that inhibit cell death by, *e.g.,* apoptosis or necrosis.

Cells can be preserved using, *e.g.,* a composition comprising an apoptosis inhibitor, necrosis inhibitor and/or an oxygen-carrying perfluorocarbon, as described in U.S. published patent application US 2007/0190042. In one embodiment, a population of placental stem cells is preserved by contacting the population with a stem cell collection composition comprising an inhibitor of apoptosis and an oxygen-carrying perfluorocarbon, wherein said inhibitor of apoptosis is present in an amount and for a time sufficient to reduce or prevent apoptosis in the population of stem cells, as compared to a population of stem cells not contacted with the inhibitor of apoptosis. In a specific embodiment, said inhibitor of apoptosis is a caspase inhibitor. In another specific embodiment, said inhibitor of apoptosis is a JNK inhibitor. In a more specific embodiment, said JNK inhibitor does not modulate differentiation or proliferation of the placental stem cells. In another embodiment, said stem cell collection composition comprises said inhibitor of apoptosis and said oxygen-carrying perfluorocarbon in separate phases. In another embodiment, said stem cell collection composition comprises said inhibitor of apoptosis and said oxygen-carrying perfluorocarbon in an emulsion. In another embodiment, the stem cell collection composition additionally comprises an emulsifier, *e.g*., lecithin. In another embodiment, said apoptosis inhibitor and said perfluorocarbon are between about 0°C and about 25°C at the time of contacting the stem cells. In another more specific embodiment, said apoptosis inhibitor and said perfluorocarbon are between about 2°C and 10°C, or between about 2°C and about 5°C, at the time of contacting the stem cells. In another more specific embodiment, said contacting is performed during transport of said population of stem cells. In another more specific embodiment, said contacting is performed during freezing and thawing of the population of placental stem cells.

In another embodiment, populations of placental stem cells can be preserved by a method comprising contacting the population with an inhibitor of apoptosis and an organ-preserving compound, wherein said inhibitor of apoptosis is present in an amount and for a time sufficient to reduce or prevent apoptosis in the population of cells, *e.g*., placental stem cells, as compared to a population of cells not contacted with the inhibitor of apoptosis. In a specific embodiment, the organ-preserving compound is UW solution (described in U.S. Patent No. 4,798,824; also known as ViaSpan; *see also* Southard et al., Transplantation 49(2):251-257 (1990)) or a solution described in Stern et al., U.S. Patent No. 5,552,267. In another embodiment, said organ-preserving compound is hydroxyethyl starch, lactobionic acid, raffinose, or a combination thereof. In another embodiment, the stem cell collection composition additionally comprises an oxygen-carrying perfluorocarbon, either in two phases or as an emulsion.

In another embodiment of the method, placental stem cells are contacted with a stem cell collection composition comprising an apoptosis inhibitor and oxygen-carrying perfluorocarbon, organ-preserving compound, or combination thereof, during perfusion. In another embodiment, the cells are contacted during a process of tissue disruption, *e.g*., enzymatic digestion. In another embodiment, placental stem cells are contacted with said stem cell collection compound after collection by perfusion, or after collection by tissue disruption, *e.g*., enzymatic digestion.

Typically, during placental or umbilical cord cell collection, enrichment and isolation, it is preferable to minimize or eliminate cell stress due to hypoxia and mechanical stress. In another embodiment of the method, therefore, placental stem cells are exposed to a hypoxic condition during collection, enrichment or isolation for less than six hours during said preservation, wherein a hypoxic condition is a concentration of oxygen that is less than normal blood oxygen concentration. In a more specific embodiment, the placental stem cells are exposed to said hypoxic condition for less than two hours during said preservation. In another more specific embodiment, the placental stem cells are exposed to said hypoxic condition for less than one hour, or less than thirty minutes, or is not exposed to a hypoxic condition, during collection, enrichment or isolation. In another specific embodiment, the placental stem cells are not exposed to shear stress during collection, enrichment or isolation.

The placental stem cells described herein can be cryopreserved, *e.g*., in cryopreservation medium in small containers, *e.g*., ampoules. Suitable cryopreservation medium includes, but is not limited to, culture medium including, *e.g*., growth medium, or cell freezing medium, for example commercially available cell freezing medium, *e.g*., media designated C2695, C2639 or C6039 (Sigma). Cryopreservation medium preferably comprises DMSO (dimethylsulfoxide), at a concentration of, *e.g.,* about 10% (v/v). Cryopreservation medium may comprise additional agents, for example, Plasmalyte, methylcellulose and/or glycerol. Placental stem cells are preferably cooled at about 1°C/min during cryopreservation. A preferred cryopreservation temperature is about -80°C to about - 180°C, preferably about -125°C to about -140°C. Cryopreserved cells can be transferred to liquid nitrogen prior to thawing for use. In some embodiments, for example, once the ampoules have reached about -90°C, they are transferred to a liquid nitrogen storage area. Cryopreserved cells preferably are thawed at a temperature of about 25°C to about 40°C, preferably to a temperature of about 37°C.

### 4.6 COMPOSITIONS COMPRISING PLACENTAL STEM CELLS

The present invention, for example, use compositions comprising placental stem cells. Non-limiting, representative examples of such compositions are provided herein.

### 4.6.1.1 Cryopreserved Cells

Placental cells, *e.g*., placental stem cells, and populations of placental stem cells, for example, cryopreserved for later use. Methods for cryopreservation of cells, such as stem cells, are well known in the art. Placental stem cell populations can be prepared in a form that is easily administrable to an individual. For example, placental stem cells can be contained within a container that is suitable for medical use. Such a container can be, for example, a sterile plastic bag, flask, jar, or other container from which the placental stem cells population can be easily dispensed. For example, the container can be a blood bag or other plastic, medically-acceptable bag suitable for the intravenous administration of a liquid to a recipient. The container is preferably one that allows for cryopreservation of the combined cell population.

Cryopreserved placental stem cells populations can comprise placental cells or umbilical cord cells derived from a single donor, or from multiple donors. The placental stem cells population can be completely HLA-matched to an intended recipient, or partially or completely HLA-mismatched.

Thus, in one embodiment, provided herein is a composition comprising the population of placental stem cells for use in the invention in a container. In a specific embodiment, the cell population is cryopreserved. In another specific embodiment, the container is a bag, flask, or jar. In more specific embodiment, said bag is a sterile plastic bag. In a more specific embodiment, said bag is suitable for, allows or facilitates intravenous administration of said placental cell population. The bag can comprise multiple lumens or compartments that are interconnected to allow mixing of the placental stem cells and one or more other solutions, *e.g*., a drug, prior to, or during, administration. In another specific embodiment, the composition comprises one or more compounds that facilitate cryopreservation of the cell population. In another specific embodiment, said placental cell population is contained within a physiologically-acceptable aqueous solution. In a more specific embodiment, said physiologically-acceptable aqueous solution is a 0.9% NaCl solution. In another specific embodiment, said placental cell population comprises placental stem cells that are HLA-matched to a recipient of said cell population. In another specific embodiment, said cell population comprises placental stem cells that are at least partially HLA-mismatched to a recipient. In another specific embodiment, said placental stem cells are derived from a plurality of donors.

### 4.6.1.2 Pharmaceutical Compositions

Populations of isolated placental stem cells, or populations of cells comprising the isolated placental stem cells for use in a method of treatment as described herein, can be formulated into pharmaceutical compositions. Such pharmaceutical compositions comprise a population of isolated placental stem cells, or a population of cells comprising isolated placental stem cells, in a pharmaceutically-acceptable carrier, *e.g.,* a saline solution or other accepted physiologically-acceptable solution for *in vivo* administration. Pharmaceutical compositions comprising the isolated placental stem cells described herein can comprise any, or any combination, of the isolated placental stem cells populations, or isolated placental stem cells, described elsewhere herein. The pharmaceutical compositions can comprise fetal, maternal, or both fetal and maternal isolated cells. The pharmaceutical compositions provided herein can further comprise isolated placental stem cells obtained from a single individual, umbilical cord or placenta, or from a plurality of individuals, umbilical cords or placentae. Any of the placental stem cells, described elsewhere herein, can be formulated into pharmaceutical composition, as described below.

The pharmaceutical compositions provided herein can comprise any number of isolated placental stem cells. For example, a single unit dose of isolated placental stem cells can comprise, in various embodiments, about, at least, or no more than 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated cells.

The pharmaceutical compositions provided herein comprise populations of cells that comprise 50% viable cells or more (that is, at least 50% of the cells in the population are functional or living). Preferably, at least 60% of the cells in the population are viable. More preferably, at least 70%, 80%, 90%, 95%, or 99% of the cells in the population in the pharmaceutical composition are viable.

The pharmaceutical compositions provided herein can comprise one or more compounds that, *e.g.,* facilitate engraftment (*e.g.,* anti-T-cell receptor antibodies, an immunosuppressant, or the like); stabilizers such as albumin, dextran 40, gelatin, hydroxyethyl starch, plasmalyte, and the like.

When formulated as an injectable solution, in one embodiment, the pharmaceutical composition comprises about 1% to 1.5% HSA and about 2.5% dextran. In a preferred embodiment, the pharmaceutical composition comprises from about 5 x 10⁶ cells per milliliter to about 2 x 10⁷ cells per milliliter in a solution comprising 5% HSA and 10% dextran, optionally comprising an immunosuppressant, *e.g.,* cyclosporine A at, *e.g.,* 10 mg/kg.

In other embodiments, the pharmaceutical composition, *e.g*., a solution, comprises a plurality of cells, *e.g*., isolated placental stem cells, wherein said pharmaceutical composition comprises between about 1.0 ±0.3 x 10⁶ cells per milliliter to about 5.0 ± 1.5 x 10⁶ cells per milliliter. In other embodiments, the pharmaceutical composition comprises between about 1.5 x 10⁶ cells per milliliter to about 3.75 x 10⁶ cells per milliliter. In other embodiments, the pharmaceutical composition comprises between about 1 x 10⁶ cells/mL to about 50 x 10⁶ cells/mL, about 1 x 10⁶ cells/mL to about 40 x 10⁶ cells/mL, about 1 x 10⁶ cells/mL to about 30 x 10⁶ cells/mL, about 1 x 10⁶ cells/mL to about 20 x 10⁶ cells/mL, about 1 x 10⁶ cells/mL to about 15 x 10⁶ cells/mL, or about 1 x 10⁶ cells/mL to about 10 x 10⁶ cells/mL. In certain embodiments, the pharmaceutical composition comprises no visible cell clumps (*i.e*., no macro cell clumps), or substantially no such visible clumps. As used herein, "macro cell clumps" means an aggregation of cells visible without magnification, *e.g*., visible to the naked eye, and generally refers to a cell aggregation larger than about 150 microns In some embodiments, the pharmaceutical composition comprises about 2.5%, 3.0%, 3.5%, 4.0%, 4.5%, 5.0%, 5.5%, 6.0%, 6.5%, 7.0%, 7.5% 8.0%, 8.5%, 9.0%, 9.5% or 10% dextran, e.g., dextran-40. In a specific embodiment, said composition comprises about 7.5% to about 9% dextran-40. In a specific embodiment, said composition comprises about 5.5 % dextran-40. In certain embodiments, the pharmaceutical composition comprises from about 1% to about 15% human serum albumin (HSA). In specific embodiments, the pharmaceutical composition comprises about 1%, 2%, 3%, 4%, 5%, 65, 75, 8%, 9%, 10%, 11%, 12%, 13%, 14% or 15% HSA. In a specific embodiment, said cells have been cryopreserved and thawed. In another specific embodiment, said cells have been filtered through a 70 µM to 100 µM filter. In another specific embodiment, said composition comprises no visible cell clumps. In another specific embodiment, said composition comprises fewer than about 200 cell clumps per 10⁶ cells, wherein said cell clumps are visible only under a microscope, *e.g*., a light microscope. In another specific embodiment, said composition comprises fewer than about 150 cell clumps per 10⁶ cells, wherein said cell clumps are visible only under a microscope, *e.g.,* a light microscope. In another specific embodiment, said composition comprises fewer than about 100 cell clumps per 10⁶ cells, wherein said cell clumps are visible only under a microscope, *e.g*., a light microscope.

In a specific embodiment, the pharmaceutical composition comprises about 1.0 ±0.3 x 10⁶ cells per milliliter, about 5.5% dextran-40 (w/v), about 10% HSA (w/v), and about 5% DMSO (v/v).

In other embodiments, the pharmaceutical composition comprises a plurality of cells, *e.g.,* a plurality of isolated placental stem cells in a solution comprising 10% dextran-40, wherein the pharmaceutical composition comprises between about 1.0 ± 0.3 x 10⁶ cells per milliliter to about 5.0 ± 1.5 x 10⁶ cells per milliliter, and wherein said composition comprises no cell clumps visible with the unaided eye (*i*.*e*., comprises no macro cell clumps). In some embodiments, the pharmaceutical composition comprises between about 1.5 x 10⁶ cells per milliliter to about 3.75 x 10⁶ cells per milliliter. In a specific embodiment, said cells have been cryopreserved and thawed. In another specific embodiment, said cells have been filtered through a 70 µM to 100 µM filter. In another specific embodiment, said composition comprises fewer than about 200 micro cell clumps (that is, cell clumps visible only with magnification) per 10⁶ cells. In another specific embodiment, the pharmaceutical composition comprises fewer than about 150 micro cell clumps per 10⁶ cells. In another specific embodiment, the pharmaceutical composition comprises fewer than about 100 micro cell clumps per 10⁶ cells. In another specific embodiment, the pharmaceutical composition comprises less than 15%, 14%, 13%, 12%, 11%, 10%, 9%, 8%, 7%, 6%, 5%, 4%, 3%, or 2% DMSO, or less than 1%, 0.9%, 0.8%, 0.7%, 0.6%, 0.5%, 0.4%, 0.3%, 0.2%, or 0.1% DMSO.

Further provided herein are compositions comprising cells, wherein said compositions are produced by one of the methods disclosed herein. For example, in one embodiment, the pharmaceutical composition comprises cells, wherein the pharmaceutical composition is produced by a method comprising filtering a solution comprising placental stem cells to form a filtered cell-containing solution; diluting the filtered cell-containing solution with a first solution to about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter, *e.g*., prior to cryopreservation; and diluting the resulting filtered cell-containing solution with a second solution comprising dextran, but not comprising human serum albumin (HSA) to produce said composition. In certain embodiments, said diluting is to no more than about 15 x 10⁶ cells per milliliter. In certain embodiments, said diluting is to no more than about 10 ± 3 x 10⁶ cells per milliliter. In certain embodiments, said diluting is to no more than about 7.5 x 10⁶ cells per milliliter. In other certain embodiments, if the filtered cell-containing solution, prior to the dilution, comprises less than about 15 x 10⁶ cells per milliliter, filtration is optional. In other certain embodiments, if the filtered cell-containing solution, prior to the dilution, comprises less than about 10 ± 3 x 10⁶ cells per milliliter, filtration is optional. In other certain embodiments, if the filtered cell-containing solution, prior to the dilution, comprises less than about 7.5 x 10⁶ cells per milliliter, filtration is optional.

In a specific embodiment, the cells are cryopreserved between said diluting with a first dilution solution and said diluting with said second dilution solution. In another specific embodiment, the first dilution solution comprises dextran and HSA. The dextran in the first dilution solution or second dilution solution can be dextran of any molecular weight, *e.g*., dextran having a molecular weight of from about 10 kDa to about 150 kDa. In some embodiments, said dextran in said first dilution solution or said second solution is about 2.5%, 3.0%, 3.5%, 4.0%, 4.5%, 5.0%, 5.5%, 6.0%, 6.5%, 7.0%, 7.5% 8.0%, 8.5%, 9.0%, 9.5% or 10% dextran. In another specific embodiment, the dextran in said first dilution solution or said second dilution solution is dextran-40. In another specific embodiment, the dextran in said first dilution solution and said second dilution solution is dextran-40. In another specific embodiment, said dextran-40 in said first dilution solution is 5.0% dextran-40. In another specific embodiment, said dextran-40 in said first dilution solution is 5.5% dextran-40. In another specific embodiment, said dextran-40 in said second dilution solution is 10% dextran-40. In another specific embodiment, said HSA in said solution comprising HSA is 1 to 15 % HSA. In another specific embodiment, said HSA in said solution comprising HSA is about 1%, 2%, 3%, 4%, 5%, 6%, 7%, 8%, 9%, 10%, 11%, 12%, 13%, 14% or 15 % HSA. In another specific embodiment, said HSA in said solution comprising HSA is 10% HSA. In another specific embodiment, said first dilution solution comprises HSA. In a more specific embodiment, said HSA in said first dilution solution is 10% HSA. In another specific embodiment, said first dilution solution comprises a cryoprotectant. In a more specific embodiment, said cryoprotectant is DMSO. In another specific embodiment, said dextran-40 in said second dilution solution is about 10% dextran-40. In another specific embodiment, said composition comprising cells comprises about 7.5% to about 9% dextran. In another specific embodiment, the pharmaceutical composition comprises from about 1.0 ±0.3 x 10⁶ cells per milliliter to about 5.0 ±1.5 x 10⁶ cells per milliliter. In another specific embodiment, the pharmaceutical composition comprises from about 1.5 x 10⁶ cells per milliliter to about 3.75 x 10⁶ cells per milliliter.

In another embodiment, the pharmaceutical composition is made by a method comprising (a) filtering a cell-containing solution comprising placental stem cells prior to cryopreservation to produce a filtered cell-containing solution; (b) cryopreserving the cells in the filtered cell-containing solution at about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter; (c) thawing the cells; and (d) diluting the filtered cell-containing solution about 1:1 to about 1:11 (v/v) with a dextran-40 solution. In certain embodiments, if the number of cells is less than about 10 ± 3 x 10⁶ cells per milliliter prior to step (a), filtration is optional. In a more specific embodiment, the cells in step (b) are cryopreserved at about 10 ± 3 x 10⁶ cells per milliliter. In a more specific embodiment, the cells in step (b) are cryopreserved in a solution comprising about 5% to about 10% dextran-40 and HSA. In certain embodiments, said diluting in step (b) is to no more than about 15 x 10⁶ cells per milliliter.

In another embodiment, the pharmaceutical composition is made by a method comprising: (a) suspending placental stem cells in a 5.5% dextran-40 solution that comprises 10% HSA to form a cell-containing solution; (b) filtering the cell-containing solution through a 70 µM filter; (c) diluting the cell-containing solution with a solution comprising 5.5% dextran-40, 10% HSA, and 5% DMSO to about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter; (d) cryopreserving the cells; (e) thawing the cells; and (f) diluting the cell-containing solution 1:1 to 1:11 (v/v) with 10% dextran-40. In certain embodiments, said diluting in step (c) is to no more than about 15 x 10⁶ cells per milliliter. In certain embodiments, said diluting in step (c) is to no more than about 10 ± 3 x 10⁶ cells/mL. In certain embodiments, said diluting in step (c) is to no more than about 7.5 x 10⁶ cells/mL.

In another embodiment, the composition comprising cells is made by a method comprising: (a) centrifuging a plurality of cells, e.g., placental stem cells, to collect the cells; (b) resuspending the cells in 5.5% dextran-40; (c) centrifuging the cells to collect the cells; (d) resuspending the cells in a 5.5% dextran-40 solution that comprises 10% HSA; (e) filtering the cells through a 70 µM filter; (f) diluting the cells in 5.5% dextran-40, 10% HSA, and 5% DMSO to about 1 to 50 x 10⁶, 1 to 40 x 10⁶, 1 to 30 x 10⁶, 1 to 20 x 10⁶, 1 to 15 x 10⁶, or 1 to 10 x 10⁶ cells per milliliter; (g) cryopreserving the cells; (h) thawing the cells; and (i) diluting the cells 1:1 to 1:11 (v/v) with 10% dextran-40. In certain embodiments, said diluting in step (f) is to no more than about 15 x 10⁶ cells per milliliter. In certain embodiments, said diluting in step (f) is to no more than about 10 ± 3 x 10⁶ cells/mL. In certain embodiments, said diluting in step (f) is to no more than about 7.5 x 10⁶ cells/mL. In other certain embodiments, if the number of cells is less than about 10 ± 3 x 10⁶ cells per milliliter, filtration is optional.

The compositions, *e.g*., pharmaceutical compositions comprising the isolated placental cells, described herein can comprise any of the isolated placental stem cells described herein.

Other injectable formulations, suitable for the administration of cellular products, may be used.

In one embodiment, the pharmaceutical composition comprises isolated placental stem cells that are substantially, or completely, non-maternal in origin, that is, have the fetal genotype; *e.g.,* at least about 90%, 95%, 98%, 99% or about 100% are non-maternal in origin.

In a specific embodiment, the pharmaceutical composition additionally comprises a stem cell that is not obtained from a placenta.

Isolated placental stem cells in the compositions, *e.g*., pharmaceutical compositions, provided herein, can comprise placental stem cells derived from a single donor, or from multiple donors. The isolated placental cells can be completely HLA-matched to an intended recipient, or partially or completely HLA-mismatched.

### 4.6.1.3 Placental Stem Cell Conditioned Media

The placental stem cells for use in the invention can be used to produce conditioned medium. In various embodiments, the conditioned medium comprises medium in which placental stem cells have grown for at least 1, 2, 3, 4, 5, 6, 7, 8, 9, 10, 11, 12, 13, 14 or more days. In other embodiments, the conditioned medium comprises medium in which placental stem cells have grown to at least 30%, 40%, 50%, 60%, 70%, 80%, 90% confluence, or up to 100% confluence. In another embodiment, the conditioned medium comprises medium in which placental stem cells and non-placental, non-umbilical cord stem cells have been cultured.

### 4.6.2 Placental Cell Bank

Cells, *e.g.,* placental stem cells, from postpartum placentas and/or umbilical cords can be cultured in a number of different ways to produce a set of lots, *e.g.,* a set of individually-administrable doses, of placental stem cells. Such lots can, for example, be obtained from stem cells from placental perfusate or umbilical cord perfusate, or from enzyme-digested placental tissue or umbilical cord tissue. Sets of lots of placental stem cells, obtained from one or a plurality of placentas, can be arranged in a bank of cells for, *e.g.,* long-term storage. Generally, adherent stem cells are obtained from an initial culture of placental or umbilical cord material to form a seed culture, *e.g.,* of placental stem cells, which is expanded under controlled conditions to form populations of cells from approximately equivalent numbers of doublings. Lots are preferably derived from the tissue of a single placenta or umbilical cord, but can be derived from the tissue of a plurality of placentas.

In one embodiment, stem cell lots are obtained as follows. Tissue is first disrupted, *e.g*., by mincing, digested with a suitable enzyme, *e.g.,* collagenase (*see* Section 5.2.3, above). The tissue preferably comprises, *e.g*., the entire amnion, entire chorion, both, or an umbilical cord from a single placenta, but can comprise only a part of the amnion, chorion or umbilical cord. The digested tissue is cultured, *e.g.,* for about 1-3 weeks, preferably about 2 weeks. After removal of non-adherent cells, high-density colonies that form are collected, *e.g.,* by trypsinization. These cells are collected and resuspended in a convenient volume of culture medium, and defined as Passage 0 cells.

Passage 0 cells are then used to seed expansion cultures. Expansion cultures can be any arrangement of separate cell culture apparatuses, *e.g*., a Cell Factory by NUNC™. Cells in the Passage 0 culture can be subdivided to any degree so as to seed expansion cultures with, *e.g.,* 1 x 10³, 2 x 10³, 3 x 10³, 4 x 10³, 5 x 10³, 6 x 10³, 7 x 10³, 8 x 10³, 9 x 10³, 1 x 10⁴, 1 x 10⁴, 2 x 10⁴, 3 x 10⁴, 4 x 10⁴, 5 x 10⁴, 6 x 10⁴, 7 x 10⁴, 8 x 10⁴, 9 x 10⁴, or 10 x 10⁴ stem cells. Preferably, from about 1 x 10³ to about 1 x 10⁴ Passage 0 cells per square centimeter are used to seed each expansion culture. The number of expansion cultures can depend upon the number of Passage 0 cells, and may be greater or fewer in number depending upon the particular placenta(s) from which the stem cells are obtained.

Expansion cultures are grown until the density of cells in culture reaches a certain value, *e.g*., about 1 x 10⁵ cells/cm². Cells can either be collected and cryopreserved at this point, or passaged into new expansion cultures as described above. Cells can be passaged, *e.g.,* 2, 3, 4 , 5, 6, 7, 8, 9, 10, 11, 12, 13, 14, 15, 16, 17, 18, 19 or 20 times prior to use. A record of the cumulative number of population doublings is preferably maintained during expansion culture(s). The cells from a Passage 0 culture can be expanded for 2, 3, 4, 5, 6, 7, 8, 9, 10, 12, 14, 16, 18, 20, 22, 24, 26, 28, 30, 32, 34, 36, 38 or 40 doublings, or up to 60 doublings. Preferably, however, the number of population doublings, prior to dividing the population of cells into individual doses, is between about 15 and about 30 doublings. The cells can be culture continuously throughout the expansion process, or can be frozen at one or more points during expansion.

Cells to be used for individual doses can be frozen, *e.g*., cryopreserved for later use. Individual doses can comprise, *e.g*., about 1 million to about 50 million cells per ml, and can comprise between about 10⁶ and about 10¹⁰ cells in total.

In one embodiment, therefore, a placental stem cells cell bank can be made by a method comprising: expanding primary culture placental stem cells from a human postpartum placenta or umbilical cord for a first plurality of population doublings; cryopreserving said placental stem cells to form a Master Cell Bank; expanding a plurality of placental stem cells from the Master Cell Bank for a second plurality of population doublings; cryopreserving the placental stem cells to form a Working Cell Bank; expanding a plurality of placental stem cells from the Working Cell Bank for a third plurality of population doublings; and cryopreserving the placental stem cells in individual doses, wherein said individual doses collectively compose a placental stem cell bank.

In another specific embodiment, primary culture cells comprise placental stem cells from placental perfusate. In another specific embodiment, said primary culture cells comprise placental stem cells from digested placental tissue. In another specific embodiment, said primary culture cells comprise placental stem cells from placental perfusate and from digested placental tissue. In another specific embodiment, all of said placental stem cells in said primary culture are from the same placenta. In another specific embodiment, the method further comprises the step of selecting CD200⁺ placental stem cells from said plurality of cells from said Working Cell Bank to form individual doses. In another specific embodiment, said individual doses comprise from about 10⁴ to about 10⁵ placental stem cells. In another specific embodiment, said individual doses comprise from about 10⁵ to about 10⁶ placental stem cells. In another specific embodiment, said individual doses comprise from about 10⁶ to about 10⁷ placental stem cells. In another specific embodiment, said individual doses comprise from about 10⁷ to about 10⁸ placental stem cells. In another specific embodiment, said individual doses comprise from about 10⁸ to about 10⁹ placental stem cells. In another specific embodiment, said individual doses comprise from about 10⁹ to about 10¹⁰ placental stem cells.

In a preferred embodiment, the donor from which the placenta is obtained (*e.g*., the mother) is tested for at least one pathogen. If the mother tests positive for a tested pathogen, the entire lot from the placenta is discarded. Such testing can be performed at any time during production of placental stem cell lots, including before or after establishment of Passage 0 cells, or during expansion culture. Pathogens for which the presence is tested can include, without limitation, hepatitis A, hepatitis B, hepatitis C, hepatitis D, hepatitis E, human immunodeficiency virus (types I and II), cytomegalovirus, herpesvirus, and the like.

### 5. EXAMPLES

### 5.1 EXAMPLE 1: TREATMENT OF DISEASES, DISORDERS OR CONDITIONS OF THE LUNG USING PLACENTAL STEM CELLS

This Example provides example treatment regimens for diseases, disorders or conditions of the lung.

### 5.1.1 Treatment of Acute Lung Injury

An individual presents with an acute lung injury due to smoke inhalation. The individual is administered 1 x 10⁸ to 5 x 10⁹ CD10⁺CD34⁻CD105⁺CD200⁺ placental stem cells in a 0.9% NaCl solution intravenously. The individual is monitored over the subsequent two weeks to assess reduction in one or more of the symptoms, including an increase in forced expiratory volume (FEV). The individual is monitored over the course of the next year, and placental stem cells in the same dose are administered as needed.

### 5.1.2 Treatment of an Interstitial Lung Disorder

An individual presents with symptoms including shortness of breath, nonproductive cough, and evidence of hemorrhage in one lung. A diagnosis of interstitial lung disease is made. The individual is administered 5 x 10⁸ to 1 x 10⁹ CD10⁺CD34⁻CD105⁺CD200⁺ placental stem cells in a 0.9% NaCl solution intravenously. The individual is monitored over the next 100 days for detectable improvement, as determined by spriometry or other measurement of forced expiratory volume (FEV). The individual is optionally also assessed by one or more of a chest X-ray, CT scan or MRI to determine whether the hemorrhage has improved.

### 5.1.3 Prophylaxis of a Lung Disorder Associated with Graft-Versus-Host Disease

An individual awaiting an allogeneic bone marrow transplant is administered 5 x 10⁸ to 1 x 10⁹ CD10⁺CD34⁻CD105⁺CD200⁺ placental stem cells in 0.9% NaCl solution intravenously within 24 hours prior to bone marrow transplantation. Administration of the cells is repeated within 24 hours after bone marrow transplantation. The individual is monitored over the next 100 days, and is administered a follow-up dose of 5-10 x 10⁸ CD10⁺CD34⁻CD105⁺CD200⁺ placental stem cells if GVHD develops and progresses beyond Grade I.

### 5.1.4 Treatment of a Lung Disorder Caused by Graft-Versus-Host Disease

An individual who has received an allogeneic bone marrow transplant presents with bronchiolitis obliterans and Grade III graft-versus-host disease. The individual is administered 5 x 10⁸ to 1 x 10⁹ CD10⁺CD34⁻CD105⁺CD200⁺ placental stem cells in 0.9% NaCl solution intravenously within 24 hours of presentation, and the individual is monitored daily for one week by spirometry to determine improvement in breathing. If breathing is not substantially improved within 4-7 days after initial administration, as assessed by at least a 10% improvement in either forced expiratory volume (FEV₁) or forced vital capacity (FVC), or an improvement of the FEV₁/FVC ratio to at least 80%, administration of the cells is repeated. The individual is optionally administered one or more of an inhaled steroid, an oral steroid, or azithromycin (*e.g*., 250 mg once a day for approximately 3 months post-presentation) in addition to the placental stem cells. The individual is monitored over the next 100 days, and is administered a follow-up dose of 5 x 10⁸ to 1 x 10⁹ CD10⁺CD34⁻ CD105⁺CD200⁺ placental stem cells at any time if GVHD of Grade III or worse recurs, or if FEV1 or FVC decrease at any time by 10% or more, or if the FEV₁/FVC ratio falls to <70%.

### 5.1.5 Treatment of a Lung Disorder Caused by Graft-Versus-Host Disease

An individual who has received a liver transplant presents with bronchiolitis obliterans and Grade III graft-versus-host disease. The individual is administered 2 x 10⁸ to 8 x 10⁸ CD10⁺CD34⁻CD105⁺CD200⁺ placental stem cells in 0.9% NaCl solution intravenously (day 0); a second administration is given 7 days later. The individual is monitored daily between doses, and for 7 days afterwards, by spirometry to determine improvement in breathing. If breathing is not substantially improved within 4-7 days after the second administration, as assessed by at least a 10% improvement in either forced expiratory volume (FEV₁) or forced vital capacity (FVC), or an improvement of the FEV₁/FVC ratio to at least 75%, or if the GVHD does not abate to Grade II or lower, administration of the cells is repeated, and the individual is administered one or more of an inhaled steroid, an oral steroid, or azithromycin (*e.g*., 250 mg once a day for approximately 3 months post-presentation) in addition to the placental stem cells. The individual is monitored over the next 100 days, and is administered a follow-up dose of 5 x 10⁸ to 1 x 10⁹ CD10⁺CD34⁻CD105⁺CD200⁺ placental stem cells at any time if GVHD of Grade III or worse recurs, or if FEV1 or FVC decrease at any time by 10% or more, or if the FEV₁/FVC ratio falls to <70%.

### 5.1.6 Treatment of Lung Disorders Associated With Rheumatoid Arthritis

An individual presents with rheumatoid arthritis with involvement of the lung. The individual is administered 1-5 x 10⁸ CD10⁺CD34⁻CD105⁺CD200⁺ placental stem cells in 0.9% NaCl solution intravenously. The individual is given methotrexate at a standard dosage and monitored for reduction in lung inflammation.

A second individual presents with rheumatoid arthritis with involvement of the lung. The lung involvement is in part due to administration of methotrexate. Methotrexate therapy is halted, and the individual is administered a combination of unmodified placental stem cells and placental stem cells that have been modified to produce a fusion polypeptide comprising IL-1Ra and DHFR, wherein the two types of stem cells are administered in a 1:1 ratio. The engineered and non-engineered cells are 1-5 x 10⁸ CD10⁺CD34⁻CD105⁺CD200⁺ placental stem cells in 0.9% NaCl solution. The individual is monitored for reduction in lung inflammation.

### 5.1.7 Treatment of a Lung Disorder Associated with SLE

An individual presents with pneumonitis and vasculitis of the lungs, in addition to fever, weakness, arthralgia (pain in the joints), diplopia (double vision), strabismus and hypoaesthesia in both hands and feet, microhematuria (detectable quantity of blood in the urine), hypocomplementemia and high titers of autoimmune antibodies. A diagnosis of systemic lupus erythematosus (SLE) is made. The individual is refractory to pulsotherapy with methylprednisolone, and to cyclophosphamide. The individual is administered 1 x 10⁹ to 5 x 10⁹ CD10⁺CD34⁻CD105⁺CD200⁺ placental stem cells in a 0.9% NaCl solution intravenously, and is administered a second dose 7 days later. The individual is monitored for the next 30-100 days for reduction in lung function, as indicated by reduction in either forced expiratory volume (FEV₁) or forced vital capacity (FVC), or a reduction of the FEV₁/FVC ratio. Given the condition of the individual, administration of placental stem cells is considered effective if any of these indicators do not worsen.

### 5.1.8 Treatment of a Lung Disorder Associated With IBD

An individual, previously diagnosed with inflammatory bowel disease (IBD), presents with dyspnea not associated with sulfasalazine or 5-aminosalicylic acid. Other causes of the dyspnea (*e.g*., allergies, asthma, and the like) were ruled out. The individual is further refractory to oral steroids and immunosuppressive therapy. On suspicion the individual has pulmonary involvement of IBD, the individual is administered 5 x 10⁸ to 1 x 10⁹ CD10⁺CD34⁻CD105⁺CD200⁺ placental stem cells in a 0.9% NaCl solution intravenously. The individual is monitored over the next 7-14 days for improvement in the dyspnea, as assessed by at least a 10% improvement in either forced expiratory volume (FEV₁) or forced vital capacity (FVC), or an improvement of the FEV₁/FVC ratio to at least 75%.

### 5.1.9 Treatment of a Lung Disorder Associated with Scleroderma

An individual, previously diagnosed with scleroderma, presents with lung involvement diagnosed as interstitial lung disease, as evidenced by reduction in the single breath diffusion capacity for carbon monoxide (DLCO) compared to normal, confirmed by the presence of lymphocytes and eosinophils in bronchoalveolar lavage. The individual is administered 5 x 10⁸ to 1 x 10⁹ CD10⁺CD34⁻CD105⁺CD200⁺ placental stem cells in a 0.9% NaCl solution intravenously. The individual is monitored over the next 30-100 days for any detectable improvement in DLCO, dyspnea and/or the presence of lymphocytes and eosinophils in bronchoalveolar lavage; any reduction is an indication the administration of the placental stem cells is efficacious.

### 5.1.10 Treatment of Interstitial Lung Disease

An individual presents with dyspnea, loss of weight, dry, unproductive cough, and a FEV₁/FVC ratio of <80%. A diagnosis of interstitial lung disease (ILD) is made. Because the cause of the lung disease is not apparent, the lung disease is characterized as idiopathic. The individual is administered 5 x 10⁸ to 1 x 10⁹ CD10⁺CD34⁻CD105⁺CD200⁺ placental stem cells in a 0.9% NaCl solution intravenously. The individual is monitored at least once a month for one year for any detectable improvement in, or lessening of worsening of, forced expiratory volume (FEV₁) or forced vital capacity (FVC), or an reduction of the FEV₁/FVC ratio. Administration of placental stem cells is considered effective if any of these indicators do not worsen. If worsening of any of these parameters occurs, the individual is administered a followup dose at the same level as the initial dose.

A second individual presents with dyspnea, loss of weight, dry, unproductive cough, and a FEV₁/FVC ratio of <80%. A diagnosis of interstitial lung disease (ILD) is made. The ILD is apparently due to recent exposure to asbsestos, as confirmed by work history, dry inspratory crackles, and a chest X-ray showing plaques above the diaphragm. The individual is administered 5 x 10⁸ to 1 x 10⁹ CD10⁺CD34⁻CD105⁺CD200⁺ placental stem cells in a 0.9% NaCl solution intravenously, optionally in combination with an oral corticosteroid. The individual is monitored at least once a month for the remainder of the individual's life for any detectable improvement in, or lessening of worsening of, forced expiratory volume (FEV₁) or forced vital capacity (FVC), or a reduction of the FEV₁/FVC ratio. Given the condition of the individual, administration of placental stem cells is considered effective if any of these indicators do not worsen. If worsening of any of these parameters occurs, the individual is administered a followup dose at the same level as the initial dose.

### 5.1.11 Treatment of Interstitial Lung Disease

An individual presents with dyspnea, persistent cough, and an enlargement of the chest (hyperaeration). The individual shows a FEV₁/FVC ratio of <60% even with bronchodilator therapy. Based on the individual's history as a smoker, a diagnosis of chronic obstructive pulmonary disease (emphysema) is made. The individual is administered 5 x 10⁸ to 1 x 10⁹ CD10⁺CD34⁻CD105⁺CD200⁺ placental stem cells in a 0.9% NaCl solution intravenously, optionally in combination with an bronchodilator such as albuterol and an inhaled corticosteroid. The individual is monitored by spirometry at least once a month for the remainder of the individual's life for any detectable improvement in, or lessening of worsening of, forced expiratory volume (FEV₁) or forced vital capacity (FVC), or a reduction of the FEV₁/FVC ratio. Given the condition of the individual, administration of placental stem cells is considered effective if any of these indicators do not worsen.

### 5.2 EXAMPLE 2: LUNG-SPECIFIC LOCALIZATION OF PLACENTAL STEM CELLS

Biodistribution of placental stem cells was evaluated in immunocompromised mice.

In a pilot study, CD10⁺CD34⁻CD105⁺CD200⁺ placental stem cells were administered as a single and/or repeat intravenous tail injection to both male and female NOD-SCID or male C57BL/10SgSnAi-Rag2(tmi)γc(tmi) mice (Taconic Farms, Germantown, New York). Mice were sacrificed at 4, 14, 28 or 47 days posttreatment and samples of lung, liver, heart, kidneys, spleen, adrenal glands, bone marrow, and brain were processed and analyzed by Q-PCR. Following IV administration, human DNA was detected in isolated total DNA from samples of lung, brain, heat and/or liver in mice that were sacrificed 4 days postdose. The highest levels of DNA were detected in the lung. Results indicated that the biodistribution of placental stem cells was primarily limited to the lung at Day 4 posttreatment. Human DNA was not detected in mouse tissue samples sacrificed at 14, 28 or 47 days posttreatment.

A second study was performed in which mice dosed with placental stem cells either as a single dose or repeat IV doses on days 1, 4 and 7 were evaluated for biodistribution and persistence of the placental stem cells by detection of the human telomerase reverse transcriptase (hTERT) gene. Mice were sacrificed at 4 and 24 hours following the first IV dose of placental stem cells as well as 4 hours postdose on day 7 (3d repeat dose) and on days 37 and 92. Human DNA was detected at 4 hours postdose in most tissues tested including most samples of lung, injection site, liver, spleen, and heart, indicating early distribution of cells to organs with higher blood flow. By 24 hours postdose, however, only lung tissue consistently harbored human DNA, whereas other tissues except injection sites and one sample of brain were cleared of human DNA. At 7 days postdose, only lung and a small number of injection sites were still positive for human DNA. By day 37, only a single lung tissue sample was weakly positive for human DNA. No human DNA was detected in any tissues from any animals evaluated at day 92. After repeat IV injections of 1 x 10⁶ cells/mouse, placental stem cells were detected I the same tissues at 4 hours after the third injection as were detected at 4 hours after a single IV injection, and all tissues were completely cleared of human DNA by day 92.

### 5.3 EXAMPLE 3: ANALYSIS OF PLACENTAL STEM CELLS IN A BLEOMYCIN MODEL OF C57BL/6 MICE

This Example provides studies demonstrating the effectiveness of placental stem cells in the treatment of fibrotic lung disease. In this example, the placental stem cells are CD34⁻, CD10⁺, CD105⁺, CD200⁺ culture-expanded, tissue culture plastic-adherent cells from placenta. The cells are karyotypically normal after expansion, and are provided at a concentration of approximately 7.5 x 10⁶ cells/mL.

### Study Using Mice

Bleomycin is a cytostatic drug commonly employed in the treatment of cancer. Administration of bleomycin typically results in chronic pulmonary inflammation that may progress to fibrosis. As such, administration of bleomycin to experimental animals is an accepted model of lung fibrosis in humans.

Eighty-four male C57CL6 mice, 6 weeks old and weighing 20-22 grams, are acclamatized for 72 hours, then assigned by twelves to one of seven different groups: (1) saline administration (negative control); (2) bleomycin; (3) saline + 1.5 x 10⁶ placental stem cells; (4) bleomycin + vehicle; (5) bleomycin + 1.5 x 10⁶ placental stem cells; (6) bleomycin (in 0.9% NaCl solution) + 1.5 x 10⁶ normal human dermal fibroblasts (NHDFs); and (7) bleomycin + pirfenidone. Bleomycin, saline, and cells are administered intravenously into the tail vein in 400 µL solution. Perfenidone is administered orally 400 mg/kg/day in 0.5% carboxymethylcellulose. Within each group, six mice are killed at day 10 post-administration, and the remaining six are killed at day 21 post-administration.

Results of the administrations are evaluated by broncheoalveolar lavage (BAL) and immunohistochemistry. Levels of TNF-α, TGF-β, connective tissue growth factor (CTGF), platelet-derived growth factor (PDGF), keratinocyte growth factor (KGF), hepatocyte growth factor (HGF), interleukin-13 (IL-13), IL-1β, IL-10 and IL-8 levels in BAL fluid and lung homogenate are determined by ELISA or comparable methodology.

For immunohistochemistry, lung lobes are perfused with formalin + a phosphatase inhibitor cocktail. The extent of collagen deposition is analyzed in part by measuring the amount of hydroxyproline in the lung tissue. Briefly, lungs are homogenized in 5 mL saline, and digested in 2 ml of 6 N HCl for 16 hours at 110°C. Following neutralization with NaOH until pH 7, one ml of 0.5 mol/L Chloramine T reagent is then added and allowed to react at room temperature for 20 minutes. 1 ml of 3.15 N perchloric acid and P-dimethylaminobenzaldehyde are then added to each sample and incubated for 20 minutes at 65°C. Samples are cooled for 10 minutes, then read at 557 nm on a spectrophotometer using trans-24-hydroxyL-proline concentrations from 0 to 5 mg/ml as a standard curve. Higher amounts of hydroxyproline indicate higher amounts of collagen deposition. *See* Krishna, G., et al. Am. J. Path. 158:997-1004 (2001).

Histopathology includes examination of lung tissues for evidence of fibrosis or fibrotic loci. placental stem cells are identified by staining with an antibody specific for human vimentin.

Efficacy of placental stem cells is established by a demonstration of a lower level of any inflammation-related cytokine compared to bleomycin controls, or a significantly lower level of hydroxyproline in lung homogenates in the presence of placental stem cells compared to bleomycin controls.

### Study Using Rats

Sixty male SD® rats (Charles River Laboratories), age 7-8 weeks and weighing approximately 176-225 g, are divided into five groups: (1) intratracheal dosing of vehicle (0.9% NaCl solution) followed in 15 minutes by intravenous dosing of vehicle; (2) intratracheal dosing of vehicle followed in 15 minutes by intravenous dosing of 4.0 x 10⁶ cells placental stem cells in 800 µL solution; (3) intratracheal dosing of bleomycin (*e.g*., BLENOXANE®, 3 units per kg) followed in 15 minutes by intravenous dosing of vehicle; (4) intratracheal dosing of bleomycin (3 units per kg) followed in 15 minutes by intravenous dosing of 1.0 x 10⁶ cells placental stem cells in 800 µL solution; and (5) intratracheal dosing of bleomycin (3 units per kg) followed in 15 minutes by intravenous dosing of 4.0 x 10⁶ cells placental stem cells in 800 µL solution.

After 15 days, animals are assessed for various physiological parameters. All animals are assessed for body weight. Blood (0.2 mL) is collected from all animals via the jugular vein and analyzed for peripheral gases, and another ∼3.5 mL is drawn via the periorbital sinus for serum extraction. Blood gas analysis includes analysis of pH, pCO₂, pO₂, aHCO₃ (actual HCO₃), tCO₃, CO-oximetry measurements (*e.g*., hemoglobin, fractional oxyhemoglobin, saturated oxyhemoglobin, carboxyhemoglobin, and methhemoglobin).

All animals are additionally tested antemortem for lung function using a flexiVent (SCIREQ®), and data for Newtonian resistance (measuring resistance of the central airways) and compliance is collected.

Six animals in each group are killed, and the lungs are analyzed for wet weight, ratio of weight to weight of the animal, collagen deposition (by hydroxyproline assay; see above); and histopathology (for fibrosis, and inflammation scoring by H&E staining). For the remaining six animals in each group, broncheoalveolar lavage is performed, and the lavage fluid is analyzed for total leukocyte counts.

Data collected in the study, as described above, is analyzed by analysis of variance, comparing Group 1, above, to Groups 2-5; comparing Group 2 to Groups 4 and 5; comparing Group 3 to Groups 4 and 5; and comparison of Group 4 to Group 5. Overall treatment effect is expected to be significant (p<0.05). Administration of placental stem cells is expected to provide significant benefit to bleomycin-challenged animals receiving the cells (Groups 4 and 5) compared to bleomycin-challenged animals not receiving the cells (Group 3), with respect to at least one of pulmonary function, as determined by flexiVent, fibrosis (as determined by hydroxyproline testing for collagen deposition), or inflammation (as evidenced by reduced inflammation-related cells present in brochoalveolar lavage fluids, or immunohistochemistry of lung tissue.

### 5.4 EXAMPLE 4: PRECLINICAL STUDY OF TREATMENT OF LUNG INFLAMMATION USING AN ANIMAL MODEL

This Example describes the conduct of a prelinical study demonstrating that isolated placental stem cells, administered intravenously, reduce airway inflammation.

Group housed 8-12 week old (age-matched), C57BL/6 male mice are used in the experiment. The mice are maintained on a Light:Dark /12 AM:12 PM cycle in a barrier facility. Food and drinking fluids are available *ad libitum.*

The experiment includes seven treatment groups, consisting of 18 mice each. Mice in groups 1-4 and 6 receive airway instillation of bacterial lipopolysaccharide (LPS), a standard inducer of inflammation once a day over the course of five days to induce airway inflammation that is moderate to severe by histological examination. Mice in groups 1 and 2 receive isolated placental stem cells or placental stem cells at 0.5 or 1.0 million cells per animal by i.v. injection, *e.g*., in the tail vein. Group 3 mice are injected with human dermal fibroblasts at 1.0 million cells per animal by i.v. injection and serve as a cellular negative control. Group 4 mice receive vehicle and serve as a baseline negative control group. Group 5 mice serve as a disease control group, and are instilled with phosphate buffered saline, but do not receive any LPS. The treatment was performed 1 hr after LPS administration. Group 6 mice serve as a positive control, and receive a reference compound, dexamethasone, at 10 mg/kg, to be administered by i.p. injection 1 hr after the LPS challenge. Group 7 mice receive only an i.v. administration of 1.0 million placental stem cells or placental stem cells, to control for the administration of the cells, *e.g.,* on macrophage infiltration into the pulmonary tissues.

Pulmonary inflammation is induced by administering 2 µL of LPS in 30 µl of PBS via intra-tracheal instillation once a day for five days. The disease control group is instilled with an equal volume of phosphate buffered saline. Each treatment group is divided into three subgroups of 6 mice each. The groups of 6 mice undergo broncho-alveolar lavage (BAL) and tissue collection at 6, 24 or 48 hrs after the exposure to LPS.

The following samples are collected from each animal at 6, 24 and 48 hrs: peripheral blood; BAL fluid, collected by instilling 3 x 1 ml 0.1% bovine serum albumin in phosphate buffered saline via tracheal cannula; and the lungs. The cellular composition of broncho-alveolar lavage fluid is determined by FACS analysis with anti-Grl, anti-CD11b (Mac1) and anti-CD45 antibodies using a Becton Dickinson FACScan. The cellular composition of the lavage is expressed as a percentage of Gr1⁺ and CD11b⁺ cells from the gated lymphocyte population. One of the lungs is homogenized and used to determine the levels of TNFα, IL-1β, mKC (mouse homologue of IL-8), IL-10, MIP-1α and MIP-2 are determined in the homogenate. The second lung is fixed in 10% formalin and processed for histopathological analysis by H&E. The levels of TNFα, IL-1β, mKC, IL-10, MIP-1α and MIP-2 are also measured in the sera of the mice by ELISA.

The extent of airway inflammation is determined by analysis of neutrophil infiltration detectable by FACS analysis of lung and broncheoalveolar lavage fluids, with an increase in CD45⁺CD11b⁺ cells represent primarily monocytes/macrophages and polymorphic granulocytes, and CD45⁺Gr1⁺ cells are mostly neutrophils and polymorphic granulocytes. Administration of placental stem cells post-induction of inflammation causes a significant reduction in either CD45⁺CD11b⁺ cells, CD45⁺Gr1⁺ cells, or both present in lung or broncheoalveolar lavage fluids. Inflammation is also determined by the levels in BAL fluids of TNFα, IL-1β, mKC, IL-10, MIP-1α and MIP-2. Administration of placental stem cells reduces the levels of one or more, or all of these cytokines. Dexamethasone (reference compound) treatment at 10 mg/kg decreases neutrophil recruitment to the lungs and lavage after administration of LPS.

## Claims

1. Placental stem cells for use in a method for treatment of a disease, disorder or condition of the lung, wherein the method comprises the administration of the placental stem cell to an individual in a therapeutically effective amount, wherein the therapeutically effective amount is an amount sufficient to cause a detectable improvement in one or more symptoms of said disease, disorder or condition, wherein the placental stem cells are CD10⁺, CD34⁻, CD200⁺, and CD105⁺, as detectable by flow cytometry, and wherein the method comprises detecting said improvement by one or more of peak flow meter, detection of CO₂ levels in the blood, radiography, CT scanning, magnetic resonance imaging, bronchoscopy, or broncheolar lavage.

2. The placental stem cells for use of claim 1, wherein said disease, disorder or condition is:
(a) associated with or caused by an immune response;
(b) an interstitial lung disease;
(c) an obstructive lung disease;
(d) an acute lung injury; or
(e) a lung injury caused by a neoplastic or paraneoplastic disease, pneumonia, or cystic fibrosis.

3. The placental stem cells for use of claim 2, wherein said disease, disorder or condition associated with or caused by an immune response is an autoimmune disease, or a graft-versus-host disease.

4. The placental stem cells for use of claim 3, wherein said autoimmune disease is rheumatoid arthritis, scleroderma, inflammatory bowel disease, or systemic lupus erythematosus.

5. The placental stem cells for use of claim 2, wherein said interstitial lung disease is interstitial pulmonary fibrosis.

6. The placental stem cells for use of claim 2, wherein said obstructive lung disease is asthma, bronchitis, acute respiratory distress syndrome or chronic obstructive pulmonary disease.

7. The placental stem cells for use of claim 2, wherein said acute lung injury is caused by a chemical burn, smoke inhalation, or exposure to a toxic substance.

8. The placental stem cells for use of claim 2, wherein said disease, disorder or condition is a lung injury caused by neopolastic or paraneoplastic disease, pneumonia, or cystic fibrosis, and wherein said administration results in a forced expiratory volume in 1 second (FEV₁) to forced vital capacity (FVC) ratio of above 0.7.

9. The placental stem cells for use of claim 1, wherein said placental stem cells are CD90⁺ and CD45⁻, as detectable by flow cytometry.

10. The placental stem cells for use of claim 9, wherein said placental stem cells are CD44⁺, as detectable by flow cytometry.

11. The placental stem cells for use of claim 9, wherein said placental stem cells are CD80⁻ and CD86⁻, as detectable by flow cytometry.

12. The placental stem cells for use of claim 1, wherein said placental stem cells are one or more of CD29⁺, CD38⁻, CD44⁺, CD54⁺, SH3⁺ or SH4⁺, as detectable by flow cytometry.

13. The placental stem cells for use of claim 1, wherein said placental stem cells are at least one of CD200⁺, CD44⁺, CD45⁻, CD90⁺, CD117⁻, CD133⁻, KDR⁻, CD80⁻, CD86⁻, HLA-ABC⁺, HLA-DR⁻, or PDL⁺.

## Patentansprüche

1. Plazentare Stammzellen zur Verwendung in einem Verfahren zur Behandlung einer Krankheit, einer Störung oder eines Zustandes der Lunge, wobei das Verfahren die Verabreichung der plazentaren Stammzelle an ein Individuum in einer therapeutisch wirksamen Menge umfasst, wobei die therapeutisch wirksame Menge eine Menge ist, die ausreicht, um eine nachweisbare Verbesserung bei einem oder mehreren Symptomen der Krankheit, der Störung oder des Zustandes zu bewirken, wobei die plazentaren Stammsellen CD10⁺, CD34⁻, CD200⁺ und CD105⁺ sind, wie durch Durchflusszytometrie nachweisbar, und wobei das Verfahren das Erfassen der Verbesserung durch einen oder mehrere der Spitzenwert-Durchflußmesser, das Erfassen von CO₂-Spiegeln im Blut, Radiographie, Computertomographie, Magnetresonanztomographie, Bronchoskopie oder Bronchenspülung umfasst.

2. Plazentare Stammzellen zur Verwendung nach Anspruch 1, wobei die Krankheit, Störung oder der Zustand:
(a) mit einer Immunantwort assoziiert ist oder durch diese verursacht wird;
(b) eine interstitielle Lungenerkrankung ist;
(c) eine obstruktive Lungenerkrankung ist;
(d) eine akute Lungenschädigung ist; oder
(e) eine Lungenschädigung ist, die durch eine neoplastische oder paraneoplastische Erkrankung, Pneumonie oder Mukoviszidose verursacht wird.

3. Plazentare Stammzellen zur Verwendung nach Anspruch 2, wobei die Krankheit, die Störung oder der Zustand, die mit einer Immunantwort assoziiert sind oder durch diese verursacht werden, eine Autoimmunkrankheit oder eine Transplantat-Wirt-Erkrankung ist.

4. Plazentare Stammzellen zur Verwendung nach Anspruch 3, wobei die Autoimmunkrankheit rheumatoide Arthritis, Sklerodermie, entzündliche Darmerkrankung oder systemischer Lupus erythematodes ist.

5. Plazentare Stammzellen zur Verwendung nach Anspruch 2, wobei die interstitielle Lungenerkrankung interstitielle Lungenfibrose ist.

6. Plazentare Stammzellen zur Verwendung nach Anspruch 2, wobei die obstruktive Lungenerkrankung Asthma, Bronchitis, akutes Atemnotsyndrom oder chronische obstruktive Lungenerkrankung ist.

7. Plazentare Stammzellen zur Verwendung nach Anspruch 2, wobei die akute Lungenschädigung durch eine Verätzung, Einatmen von Rauch oder Aussetzung gegenüber einer toxischen Substanz verursacht wird.

8. Plazentare Stammzellen zur Verwendung nach Anspruch 2, wobei die Krankheit, Störung oder der Zustand eine Lungenschädigung ist, die durch neopolastische oder paraneoplastische Erkrankung, Pneumonie oder Mukoviszidose verursacht wird, und wobei die Verabreichung zu einem erzwungenen Ausatmungsvolumen in 1 Sekunde (FEV₁) zu einem erzwungenen Vitalkapazitäts (FVC) -Verhältnis von über 0,7 führt.

9. Plazentare Stammzellen zur Verwendung nach Anspruch 1, wobei die plazentaren Stammzellen CD90⁺ und CD45⁻ sind, wie durch Durchflusszytometrie nachweisbar.

10. Plazentare Stammzellen zur Verwendung nach Anspruch 9, wobei die plazentaren Stammzellen CD44⁺ sind, wie durch Durchflusszytometrie nachweisbar.

11. Plazentare Stammzellen zur Verwendung nach Anspruch 9, wobei die plazentaren Stammzellen CD80⁻ und CD86⁻ sind, wie durch Flusszytometrie nachweisbar.

12. Plazentare Stammzellen zur Verwendung nach Anspruch 1, wobei die plazentaren Stammzellen eine oder mehrere von CD29⁺, CD38⁻, CD44⁺ CD54⁺, SH3⁺ oder SH4⁺ sind, wie durch Flusszytometrie nachweisbar.

13. Plazentare Stammzellen zur Verwendung nach Anspruch 1, wobei die plazentaren Stammzellen mindestens eine von CD200⁺, CD44⁺ CD45⁻, CD90⁺, CD117⁻, CD133⁻, KDR⁻, CD80⁻, CD86⁻, HLA-ABC⁺, HLA-DR⁻ oder PDL⁺ sind.

## Revendications

1. Cellules-souches placentaires pour l'utilisation dans un procédé pour le traitement d'une maladie, d'un trouble ou d'un état du poumon, dans lequel le procédé comprend l'administration de la cellule-souche placentaire à un individu en une quantité efficace d'un point de vue thérapeutique, dans laquelle la quantité efficace d'un point de vue thérapeutique est une quantité suffisante pour provoquer une amélioration détectable d'un ou plusieurs symptômes de ladite maladie, dudit trouble ou dudit état, dans lesquelles les cellules-souches placentaires sont des CD10⁺, CD34⁻, CD200⁺ et CD105+, telles que détectables par cytométrie en flux, et dans lesquelles le procédé comprend la détection de ladite amélioration par un ou plusieurs d'une mesure de débit maximal, d'une détection de niveaux de CO₂ dans le sang, d'une radiographie, d'un balayage par TDM, d'une imagerie par résonance magnétique, d'une bronchoscopie, ou d'un lavage bronchéolaire.

2. Cellules-souches placentaires pour l'utilisation selon la revendication 1, dans lesquelles ladite maladie, ledit trouble ou ledit état est:
(a) associé(e) à ou provoqué par une réponse immunitaire;
(b) une maladie de poumon interstitielle;
(c) une maladie de poumon obstructive;
(d) une lésion pulmonaire aiguë; ou
(e) une lésion pulmonaire provoquée par une maladie néoplasique ou paranéoplasique, une pneumonie, ou une fibrose kystique.

3. Cellules-souches placentaires pour l'utilisation selon la revendication 2, dans lesquelles ladite maladie, ledit trouble ou ledit état, associé(e) à ou provoqué(e) par une réponse immunitaire, est une maladie auto-immune, ou une réaction de greffe contre hôte.

4. Cellules-souches placentaires pour l'utilisation selon la revendication 3, dans lesquelles ladite maladie auto-immune est l'arthrite rhumatoïde, la sclérodermie, une maladie intestinale inflammatoire, ou le lupus érythémateux systémique.

5. Cellules-souches placentaires pour l'utilisation selon la revendication 2, dans lesquelles ladite maladie de poumon interstitielle est la fibrose pulmonaire interstitielle.

6. Cellules-souches placentaires pour l'utilisation selon la revendication 2, dans lesquelles ladite maladie de poumon obstructive est l'asthme, la bronchite, le syndrome de détresse respiratoire aiguë ou la maladie pulmonaire obstructive chronique.

7. Cellules-souches placentaires pour l'utilisation selon la revendication 2, dans lesquelles ladite lésion pulmonaire aiguë est provoquée par une brûlure chimique, une inhalation de fumées, ou une exposition à une substance toxique.

8. Cellules-souches placentaires pour l'utilisation selon la revendication 2, dans lesquelles ladite maladie, ledit trouble ou ledit état est une lésion pulmonaire provoquée par une maladie néoplasique ou paranéoplasique, une pneumonie, ou une fibrose kystique, et dans lesquelles ladite administration aboutit à un rapport de volume expiratoire forcé en 1 seconde (FEV1) sur une capacité vitale forcée (FVC) supérieur à 0,7.

9. Cellules-souches placentaires pour l'utilisation selon la revendication 1, dans lesquelles lesdites cellules-souches placentaires sont des CD90⁺ et CD45⁻, telles que détectables par cytométrie en flux.

10. Cellules-souches placentaires pour l'utilisation selon la revendication 9, dans lesquelles lesdites cellules-souches placentaires sont des CD44⁺, telles que détectable par cytométrie en flux.

11. Cellules-souches placentaires pour l'utilisation selon la revendication 9, dans lesquelles lesdites cellules-souches placentaires sont des CD80⁻ et CD86⁻, telles que détectables par cytométrie en flux.

12. Cellules-souches placentaires pour l'utilisation selon la revendication 1, dans lesquelles lesdites cellules-souches placentaires sont une ou plusieurs des CD29⁺, CD38⁻, CD44⁺, CD54⁺, SH3⁺ ou SH4⁺, telles que détectables par cytométrie en flux.

13. Cellules-souches placentaires pour l'utilisation selon la revendication 1, dans lesquelles lesdites cellules-souches placentaires sont au moins une parmi CD200⁺, CD44⁺, CD45⁻, CD90⁺, CD117⁻, CD133⁻, KDR⁻, CD80⁻, CD86⁻, HLA-ABC⁺, HLA-DR⁻, ou PDL⁺.
